# EUROPÄISCHE PATENTSCHRIFT

(11) **EP 0 420 806 B1**
(45) Veröffentlichungstag und Bekanntmachung des Hinweises auf die Patenterteilung: **05.07.1995**
(21) Anmeldenummer: 90810712.1
(22) Anmeldetag: 18.09.1990
(51) Int. Cl.: C07F 9/38, A61K 31/675, C07F 9/59

(54) **Phosphonsäure, Verfahren zur Herstellung und Verwendung als Arzneimittelwirkstoff**
Phosphonic acids, process for preparation and use as an active pharmaceutical composition
Acides phosphoniques, procédé de préparation et utilisation comme composition pharmaceutique

(30) Priorität: 26.09.1989 CH 3479/89
(43) Veröffentlichungstag der Anmeldung: 03.04.1991
(73) Patentinhaber: CIBA-GEIGY AG, 4002 Basel (CH)
(72) Erfinder: Angst, Christof, Dr., CH-4310 Rheinfelden (CH); Allgeier, Hans, Dr., D-7850 Lörrach-Haagen (DE); Heckendorn, Roland, Dr., CH-4144 Arlesheim (CH); Wallach, Daniel, Dr., CH-4054 Basel (CH)

(56) Entgegenhaltungen:
- EP-A- 0 233 154
- EP-A- 0 302 826

## Beschreibung

Die Erfindung betrifft substituierte 2-Aminoalk-3-ensäurederivate der Formel I 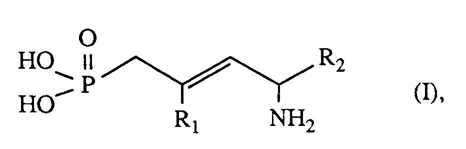
worin R₁ einen durch gegebenenfalls acyliertes oder aliphatisch oder araliphatisch veräthertes Hydroxy, durch Halogen, durch gegebenenfalls acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls aliphatisch N-substituierten oder N-acylierten azacycloaliphatischen Kohlenwasserstoffrest bedeutet und R₂ freies oder verestertes Carboxy darstellt, und ihre Salze, Verfahren zur Herstellung der erfindungsgemässen Verbindungen, diese enthaltende pharmazeutische Präparate und ihre Verwendung als Arzneimittelwirkstoffe.

Aliphatische Kohlenwasserstoffreste sind beispielsweise Alkylreste mit bis und mit 10, insbesondere bis und mit 8 C-Atomen, vorzugsweise Niederalkylreste.

Durch Hydroxy substituierte aliphatische Kohlenwasserstoffreste sind beispielsweise Mono- oder Dihydroxyniederalkyl.

Acyliertes Hydroxy ist beispielsweise Niederalkanoyloxy oder im Phenylteil gegebenenfalls substituiertes Benzoyloxy. Dementsprechend sind unter einem durch acyliertes Hydroxy substituierten aliphatischen Kohlenwasserstoffrest beispielsweise Niederalkanoyloxyniederalkyl oder im Phenylteil gegebenenfalls substituiertes Benzoyloxyniederalkyl zu verstehen.

Aliphatisch veräthertes Hydroxy ist beispielsweise Niederalkoxy; araliphatisch veräthertes Hydroxy gegebenenfalls substituiertes Phenylniederalkoxy. Dementsprechend ist unter einem durch aliphatisch veräthertes Hydroxy substituierten aliphatischen Kohlenwasserstoffrest beispielsweise Niederalkoxyniederalkyl und unter einem durch araliphatisch veräthertes Hydroxy substituierten aliphatischen Kohlenwasserstoffrest beispielsweise gegebenenfalls substituiertes Phenylniederalkoxyniederalkyl zu verstehen.

Ein durch Halogen substituierter aliphatischer Kohlenwasserstoffrest ist beispielsweise Halogenniederalkyl.

Gegebenenfalls acyliertes und/oder aliphatisch substituiertes Amino ist beispielsweise Amino, N-Mono- oder N,N-Diniederalkylamino, N-Niederalkanoylamino, im Phenylteil gegebenenfalls substituiertes N-Benzoylamino oder N-Niederalkanoyl-N-niederalkylamino. Durch gegebenenfalls acyliertes und/oder aliphatisch substituiertes Amino substituierte aliphatische Kohlenwasserstoffreste sind dementsprechend beispielsweise Aminoniederalkyl, Niederalkylaminoniederalkyl, Niederalkanoylaminoniederalkyl, im Phenylteil gegebenenfalls substituiertes Benzoylaminoniederalkyl, Diniederalkylaminoniederalkyl oder N-Niederalkanoyl-N-niederalkyl-aminoniederalkyl.

Durch einen azacycloaliphatischen Rest substituierte aliphatische Kohlenwasserstoffreste sind beispielsweise 4- bis 7-gliedrige Azacycloalkylniederalkylreste, deren Azacyloalkylteil über das N- oder ein C-Atom gebunden und im zweitgenannten Fall gegebenenfalls N-niederalkyliert, N-niederalkanoyliert oder durch eine im Phenylteil gegebenenfalls substituierte Benzoylgruppe N-substituiert sein kann. 4- bis 7-Gliedriges Azacycloalkylniederalkyl, dessen Azacyloalkylteil über das N-Atom gebunden ist, bedeutet beispielsweise N,N-Niederalkylenamino-C₁-C₇-alkyl, d.h. Azacycloalk-1-yl-C₁-C₇-alkyl. 4- bis 7-Gliedriges Azacycloalkylniederalkyl, dessen Azacyloalkylteil über ein C-Atom gebunden und gegebenenfalls N-niederalkyliert, N-niederalkanoyliert oder durch eine im Phenylteil gegebenenfalls substituierte Benzoylgruppe N-substituiert ist, bedeutet beispielsweise 5- bis 7-gliedriges, über ein C-Atom gebundenes Azacycloalkyl-C₁-C₇-alkyl oder N-C₂-C₇-Alkanoylazacycloalkyl-C₁-C₇-alkyl, ferner N-C₁-C₄-Alkylazacycloalkyl-C₁-C₇-alkyl oder im Phenylteil gegebenenfalls substituiertes N-Benzoylazacycloalkyl-C₁-C₇-alkyl.

Durch einen diazacycloaliphatischen Rest substituierte aliphatische Kohlenwasserstoffreste sind beispielsweise 4- bis 7-gliedriges Diazacycloalkylniederalkylreste, deren Diazacycloalkylteil über ein N-Atom gebunden ist und am anderen N-Atom gegebenenfalls niederalkyliert, niederalkanoyliert oder durch eine im Phenylteil gegebenenfalls substituierte Benzoylgruppe substituiert sein kann, wie 4- bis 7-gliedriges, über ein N-Atom gebundene, gegebenenfalls N'-niederalkylierte, N'-niederalkanoylierte oder durch eine im Phenylteil gegebenenfalls substituierte Benzoylgruppe N'-substituierte Diazacycloalkylniederalkylreste, insbesondere 5- bis 7-gliedriges Diazacycloalk-1-yl-C₁-C₇-alkyl.

Durch einen azoxacycloaliphatischen Rest substituierte aliphatische Kohlenwasserstoffreste sind beispielsweise 4- bis 7-gliedrige Azoxacycloalkylniederalkylreste, deren Azoxacycloalkylteil über das N-Atom gebunden ist, wie 4- bis 7-gliedriges, über das N-Atom gebundene Azoxacycloalkylniederalkylreste, insbesondere 5- bis 7-gliedriges N,N-(Oxaniederalkylen)amino-C₁-C₇-alkyl, d.h. Azoxacycloalk-1-yl-C₁-C₇-alkyl.

Durch einen oxacycloaliphatischen Rest substituierte aliphatische Kohlenwasserstoffreste sind beispielsweise 4- bis 7-gliedriges Oxacycloalkylniederalkylreste, deren Oxacycloalkylteil über ein C-Atom gebunden ist, wie 5- bis 7-gliedriges, über ein C-Atom gebundene Oxacycloalkyl-C₁-C₇-alkyl.

Über ein C-Atom gebundene oxacycloaliphatische Kohlenwasserstoffreste sind beispielsweise 5- bis 7-gliedriges, über ein C-Atom gebundene Oxacycloalkylgruppen.

Über ein C-Atome gebundene, gegebenenfalls aliphatisch N-substituierte oder N-acylierte azacycloaliphatische Kohlenwasserstoffreste sind über ein C-Atom gebundene gegebenenfalls N-niederalkylierte, N-niederalkanoylierte oder durch im Phenylteil gegebenenfalls substituiertes Benzoyl N-substituierte Azacycloalkylreste, wie 5- bis 7-gliedriges, über ein C-Atom gebundenes Azacycloalkyl oder N-C₂-C₇-Alkanoylazacycloalkyl, ferner N-C₁-C₄-Alkylazacycloalkyl oder gegebenenfalls substituiertes N-Benzoylazacycloalkyl.

Verestertes Carboxy ist beispielsweise mit einem aliphatischen, cycloaliphatischen oder araliphatischen Alkohol verestertes Carboxy, wie Niederalkoxycarbonyl, 4- bis und mit 7-gliedriges, insbesondere 5- oder 6-gliedriges Cycloalkoxycarbonyl, wie Cyclopentyloxy- oder Cyclohexyloxycarbonyl, oder gegebenenfalls substituiertes Phenylniederalkoxycarbonyl.

In den vorstehend genannten Gruppen können Phenylreste unsubstituiert oder in üblicher Weise, beispielsweise durch Niederalkyl, Niederalkoxy, Halogen, Cyano und/oder Trifluormethyl, mono-, di- oder trisubstituiert, insbesondere mono- oder disubstituiert, sein.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist beispielsweise C₁-C₇-Alkyl, vorzugsweise C₁-C₄-Alkyl, wie Methyl, Äthyl, Propyl, Isopropyl oder Butyl, kann aber auch Isobutyl, Sekundärbutyl, Tertiärbutyl oder eine Pentyl-, Hexyl- oder Heptylgruppe sein.

Mono- oder Dihydroxyniederalkyl ist beispielsweise Hydroxy-C₁-C₇-alkyl, wie Hydroxymethyl, 2-Hydroxyäthyl, 3-Hydroxypropyl, 4-Hydroxybutyl, 5-Hydroxypentyl oder 6-Hydroxyhexyl, bzw. Dihydroxy-C₂-C₇-alkyl, in welchem die Hydroxygruppen an verschiedene C-Atome gebunden sind, wie 1,2-Dihydroxyäthyl oder insbesondere 1,3-Dihydroxyprop-2-yl.

Niederalkanoyl ist beispielsweise C₂-C₇-Alkanoyl, insbesondere C₂-C₄-Alkanoyl, wie Acetyl, Propionyl oder Butyryl, kann aber auch eine C₅-C₆-Alkanoylgruppe, wie Pivaloyl, sein. Dementsprechend ist Niederalkanoyloxyniederalkyl insbesondere C₂-C₇-Alkanoyloxy-C₁-C₇-alkyl, wie Acetoxymethyl, Propionyloxymethyl, Butyryloxymethyl, 2-Acetyloxyäthyl, 3-Acetyloxypropyl, 4-Acetyloxybutyl, 5-Acetyloxypentyl oder 6-Acetyloxyhexyl. Analog ist unter Benzoyloxyniederalkyl beispielsweise im Phenylteil gegebenenfalls substituiertes Benzoyloxy-C₁-C₇-alkyl, wie Benzoyloxymethyl, 2-Benzoyloxyäthyl, 3-Benzoyloxypropyl, 4-Benzoyloxybutyl, 5-Benzoyloxypentyl oder 6-Benzoyloxyhexyl, zu verstehen.

Niederalkoxy ist beispielsweise C₁-C₇-Alkoxy, vorzugsweise C₁-C₄-Alkoxy, wie Methoxy, Äthoxy, Propyloxy, Isopropyloxy oder Butyloxy , kann aber auch Isobutyloxy, Sekundärbutyloxy, Tertiärbutyloxy oder eine Pentyloxy-, Hexyloxy- oder Heptyloxygruppe sein. Entsprechend bedeutet Niederalkoxyniederalkyl beispielsweise C₁-C₄-Alkoxy-C₁-C₇-alkyl-, wie Methoxymethyl, Äthoxymethyl, 2-Methoxyäthyl, 2-Äthoxyäthyl, 3-Methoxypropyl, 4-Methoxybutyl, 5-Methoxypentyl oder 6-Methoxyhexyl.

Phenylniederalkoxy bedeutet beispielsweise gegebenenfalls wie angegeben substituiertes Phenyl-C₁-C₄-alkoxy, wie Benzyloxy, 2-Phenyläthoxy oder 3-Phenylpropyloxy. Entsprechend bedeutet Phenylniederalkoxyniederalkyl beispielsweise einen gegebenenfalls wie angegeben substituierten Phenyl-C₁-C₄-alkoxy-C₁-C₇-alkyl-, wie Benzyloxymethyl-, 2-Phenyläthoxymethyl-, 2-Benzyloxyäthyl-, 3-Benzyloxypropyl-, 4-Benzyloxybutyl-, 5-Benzyloxypentyl- oder 6-Benzyloxyhexylrest.

Halogenniederalkyl ist beispielsweise Halogen-C₁-C₇-alkyl, wie Halogenmethyl, 2-Halogenäthyl, 3-Halogenpropyl, 4-Halogenbutyl, 5-Halogenpentyl oder 6-Halogenhexyl, wobei Halogen Chlor oder insbesondere Fluor bedeutet

Aminoniederalkyl ist beispielsweise Amino-C₁-C₇-alkyl, wie Aminomethyl, 2-Aminoäthyl, 3-Aminopropyl, 4-Aminobutyl, 5-Aminopentyl oder 6-Aminohexyl.

Niederalkylamino ist beispielsweise C₁-C₇-Alkylamino, insbesondere C₁-C₄-Alkylamino, wie Methylamino, Äthylamino, Propylamino oder Butylamino, kann aber auch eine C₅-C₆-Alkylamino-, wie Pentylamino- oder Hexylaminogruppe, sein. Dementsprechend ist Niederalkylaminoniederalkyl insbesondere C₁-C₄-Alkylamino-C₁-C₇-alkyl, wie Methylaminomethyl, Äthylaminomethyl, Propylaminomethyl, Butylaminomethyl, 2-Methylaminoäthyl, 3-Methylaminopropyl, 4-Methylaminobutyl, 5-Methylaminopentyl oder 6-Methylaminohexyl.

Niederalkanoylamino ist beispielsweise C₂-C₇-Alkanoylamino, insbesondere C₂-C₄-Alkanoylamino, wie Acetylamino, Propionylamino oder Butyrylamino, kann aber auch C₅-C₆-Alkanoylamino, wie Pivaloylamino, sein. Dementsprechend ist Niederalkanoylaminoniederalkyl insbesondere C₂-C₇-Alkanoylamino-C₁-C₇-alkyl, wie Acetylaminomethyl, Propionylaminomethyl, Butyrylaminomethyl, 2-Acetylaminoäthyl, 3-Acetylaminopropyl, 4-Acetylaminobutyl, 5-Acetylaminopentyl oder 6-Acetylaminohexyl. Analog ist unter Benzoylaminoniederalkyl beispielsweise im Phenylteil gegebenenfalls substituiertes Benzoylamino-C₁-C₇-alkyl, wie Benzoylaminomethyl, 2-Benzoylaminoäthyl, 3-Benzoylaminopropyl, 4-Benzoylaminobutyl, 5-Benzoylaminopentyl oder 6-Benzoylaminohexyl, zu verstehen.

Diniederalkylamino ist beispielsweise Di-C₁-C₇-Alkylamino, insbesondere Di-C₁-C₄-Alkylamino, wie Dimethylamino, Diäthylamino, N-Äthyl-N-methyl-amino, N-Methyl-N-propyl-amino, Dipropylamino oder Dibutylamino. Dementsprechend ist Diniederalkylaminoniederalkyl insbesondere Di-C₁-C₄-alkylamino-C₁-C₇-alkyl, wie Dimethylaminomethyl, Diäthylaminomethyl, N-Äthyl-N-methyl-aminomethyl, N-Methyl-N-propyl-aminomethyl, Dipropylaminomethyl, Dibutylaminomethyl, 2-Dimethylaminoäthyl, 3-Dimethylaminopropyl, 4-Dimethylaminobutyl, 5-Dimethylaminopentyl oder 6-Dimethylaminohexyl.

N-Niederalkanoyl-N-niederalkyl-aminoniederalkyl ist beispielsweise N-C₂-C₇-Alkanoyl-N-C₁-C₄-alkyl-amino-C₁-C₇-alkyl, wie N-Acetyl-N-methyl-aminomethyl, N-Acetyl-N-äthyl-aminomethyl, N-Propionyl-N-methyl-aminomethyl, N-Butyryl-N-methyl-aminomethyl, 2-(N-Acetyl-N-methyl-amino)äthyl, 2-(N-Propionyl-N-methylamino)äthyl, 2-(N-Acetyl-N-äthyl-amino)äthyl, 3-(N-Acetyl-N-methyl-amino)propyl, 4-(N-Acetyl-N-methyl-amino)butyl, 5-(N-Acetyl-N-methyl-amino)pentyl oder 6-(N-Acetyl-N-methyl-amino)hexyl.

4- bis 7-gliedriges, über ein N-Atom gebundenes Azacycloalkyl-C₁-C₇-alkyl ist vorzugsweise N,N-Niederalkylenamino-C₁-C₇-alkyl, d.h. Azacycloalk-1-yl-C₁-C₇-alkyl, beispielsweise Pyrrolidinomethyl, Piperidinomethyl, 2-Pyrrolidinoäthyl, 2-Piperidinoäthyl, 3-Pyrrolidinopropyl, 3-Piperidinopropyl, 4-Pyrrolidinobutyl, 4-Piperidinobutyl, 5-Pyrrolidinopentyl, 5-Piperidinopentyl, 6-Pyrrolidinohexyl oder 6-Piperidinohexyl.

4- bis 7-gliedriges, über ein C-Atom gebundenes Azacycloalkyl-C₁-C₇-alkyl ist vorzugsweise Azacycloalk-3-yl-C₁-C₇-alkyl bzw. 4-yl-C₁-C₇-alkyl, z.B. Piperidin-4-ylmethyl, 2-(Piperidin-4-yl)äthyl, 3-(Piperidin-4-yl)propyl oder 4-(Piperidin-4-yl)butyl.

5- bis 7-gliedriges, über ein C-Atom gebundenes N-C₂-C₇-Alkanoylazacycloalkyl-C₁-C₇-alkyl ist vorzugsweise 1-C₂-C₇-Alkanoylazacycloalk-3-yl-C₁-C₇-alkyl bzw. 4-yl-C₁-C₇-alkyl, z.B. 1-Acetylpiperidin-4-ylmethyl, 2-(1-Acetylpiperidin-4-yl)äthyl, 3-(1-Acetylpiperidin-4-yl)propyl oder 4-(1-Acetylpiperidin-4-yl)butyl.

5- bis 7-gliedriges, über ein C-Atom gebundenes N-C₁-C₄-Alkylazacycloalkyl-C₁-C₇-alkyl ist vorzugsweise N-C₁-C₄-Alkylazacycloalk-3-yl-C₁-C₇-alkyl bzw. 4-yl-C₁-C₇-alkyl, z.B. 1-Methylpiperidin-4-ylmethyl, 1-Äthylpiperidin-4-ylmethyl, 2-(1-Methylpiperidin-4-yl)äthyl, 2-(1-Äthylpiperidin-4yl)äthyl, 3-(1-Methylpiperidin-4-yl)propyl, 3-(1-Äthylpiperidin-4-yl)propyl, 4-(1-Methylpiperidin-4-yl)butyl oder 4-(1-Äthylpiperidin-4-yl)butyl.

5- bis 7-gliedriges, über ein C-Atom gebundenes und im Phenylteil gegebenenfalls substituiertes N-Benzoylazacycloalkyl-C₁-C₇-alkyl ist vorzugsweise N-C₁-C₄-Benzoylazacycloalk-3-yl-C₁-C₇-alkyl bzw. 4-yl-C₁-C₇-alkyl, z.B. 1-Benzoylpiperidin-4-ylmethyl, 2-(1-Benzoylpiperidin-4-yl)äthyl, 3-(1-Benzoylpiperidin-4-yl)propyl oder 4-(1-Benzoylpiperidin-4-yl)butyl.

5- bis 7-gliedriges, über ein N-Atom gebundenes und gegebenenfalls N'-niederalkylierte, N'-niederalkanoyliertes oder durch eine im Phenylteil gegebenenfalls substituierte Benzoylgruppe N'-substituiertes Diazacycloalk-1-yl-C₁-C₇-alkyl ist beispielsweise wie N,N-(Azaniederalkylen)amino-C₁-C₇-alkyl, d.h. Diazacycloalk-1-yl-C₁-C₇-alkyl, N'-C₁-C₄-Alkyldiazacycloalk-1-yl-C₁-C₇-alkyl oder N'-C₂-C₇-Alkanoylazacycloalk-1-yl-C₁-C₇-alkyl, beispielsweise Piperazino- bzw. N'-Methyl- bzw. N'-Acetylpiperazinomethyl, 2-(Piperazino- bzw. N'-Methyl- bzw. N'-Acetylpiperazino)äthyl, 3-(Piperazino- bzw. N'-Methyl- bzw. N'-Acetylpiperazino)propyl oder 4-(Piperazin- bzw. N'-Methyl- bzw. N'-Acetylpiperazino)butyl.

5- bis 7-gliedriges N,N-(Oxaniederalkylen)amino-C₁-C₇-alkyl, d.h. Azoxacycloalk-1-yl-C₁-C₇-alkyl ist beispielsweise Morpholinomethyl, 2-Morpholinoäthyl, 3-Morpholinopropyl oder 4- Morpholinobutyl.

5- bis 7-gliedriges, über ein C-Atom gebundenes Oxacycloalkyl-C₁-C₇-alkyl ist insbesondere 5- bis 7-gliedriges Oxacycloalk-3-yl-C₁-C₇-alkyl oder Oxacycloalk-4-yl-C₁-C₇-alkyl, wie Tetrahydropyran-4-ylmethyl, 2-(Tetrahydropyran-4-yl)äthyl, 3-(Tetrahydropyran-4-yl)propyl oder 4-(Tetrahydropyran-4-yl)butyl.

5- bis 7-gliedriges, über ein C-Atom gebundenes Oxacycloalkyl ist insbesondere entsprechendes Oxacycloalk-3-yl bzw. 4-yl, z.B. Tetrahydropyran-4-yl.

5- bis 7-gliedriges, über ein C-Atom gebundenes Azacycloalkyl, N-C₁-C₄-Alkylazacycloalkyl oder N-C₂-C₇-Alkanoylazacycloalkyl ist vorzugsweise Azacycloalk-3-yl bzw. 4-yl oder 1-C₂-C₇-Alkanoyl-azacycloalk-3-yl bzw. 4-yl, z.B. Piperidin-4-yl oder 1-Acetylpiperidin-4-yl, ferner N-C₁-C₄-Alkylazacycloalk-3-yl bzw. -4-yl oder im Phenylteil gegebenenfalls substituiertes N-Benzoylazacycloalk-3-yl oder -4-yl, z.B. 1-Methylpiperidin-4-yl oder 1-Benzoylpiperidin-4-yl.

Niederalkoxycarbonyl ist beispielsweise C₁-C₇-Alkoxycarbonyl, insbesondere C₁-C₄-Alkoxycarbonyl, wie Methoxycarbonyl, Äthoxycarbonyl, Propoxycarbonyl, Isopropoxycarbonyl oder Butyloxycarbonyl, kann aber auch eine C₅-C₇-Alkoxycarbonyl-, wie Pentyloxycarbonyl-, Hexyloxycarbonyl- oder Heptyloxycarbonylgruppe sein.

Phenylniederalkoxycarbonyl ist beispielsweise Phenyl-C₁-C₄-alkoxycarbonyl, wie Benzyloxycarbonyl, 2-Phenyläthoxycarbonyl, 3-Phenylpropoxycarbonyl oder 4-Phenylbutyloxycarbonyl.

Die Verbindungen der Formel I liegen auf Grund ihres amphoteren Charakters in Form innerer Salze vor und können sowohl Säureadditionssalze als auch Salze mit Basen bilden.

Säureadditionssalze von Verbindungen der Formel I sind beispielsweise deren pharmazeutisch verwendbare Salze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Äthyl-, Diäthyl- oder Triäthylamin, Mono-, Di- oder Tri-(hydroxyniederalkyl)aminen, wie Äthanol-, Diäthanol- oder Triäthanolamin, Tris-(hydroxymethyl)methylamin oder 2-Hydroxytertiärbutylamin, oder N-(Hydroxyniederalkyl)-N,N-diniederalkyl- aminen bzw. N-(Polyhydroxyniederalkyl)-N-niederalkylaminen, wie 2-(Dimethylamino)äthanol oder D-Glucamin, oder quaternären aliphatischen Ammoniumhydroxiden, z.B. Tetrabutylammoniumhydroxid.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Erfindung betrifft beispielsweise Verbindungen der Formel I, worin R₁ einen durch gegebenenfalls aliphatisch oder araliphatisch veräthertes Hydroxy, gegebenenfalls aliphatisch substituiertes Amino oder Halogen substituierten aliphatischen Kohlenwasserstoffrest bedeutet und R₂ freies oder verestertes Carboxy darstellt, und ihre Salze.

Die Verbindungen der Formel I weisen wertvolle pharmakologische Eigenschaften auf. Insbesondere besitzen sie eine ausgeprägte und selektive antagonistische Wirkung gegenüber N-Methyl-D-asparaginsäure-sensitiven (NMDA-sensitiven) excitatorischen Aminosäurerezeptoren von Warmblütern. Diese kann in vitro beispielsweise in der Versuchsanordnung nach G. Fagg und A. Matus, Proc. Nat. Acad. Sci., USA, 81, 6876-80 (1984) ermittelt werden. Dabei wird bestimmt, in welchem Ausmass die Bindung von L-³H-Glutaminsäure an NMDA-sensitiven Rezeptoren gehemmt wird. Die NMDA-antagonistischen Eigenschaften der neuen Verbindungen können aber auch in vivo z.B. an der Maus, anhand der Hemmwirkung auf NMDA-induzierte Konvulsionen demonstriert werden.

Auf Grund dieser Eigenschaften sind die Verbindungen der Formel I und ihre pharmazeutisch verwendbaren Salze vorzüglich geeignet zur Behandlung von pathologischen Zuständen, die auf eine Blockierung von NMDA-sensitiven Rezeptoren ansprechen, beispielsweise von ischämischen Erkrankungen, wie der cerebralen Ischämie und von ischämischen Erkrankungen des Auges, von Gefäss- und Muskelspasmen, wie von Migräne oder von lokaler oder generaler Spastizität, und insbesondere von Konvulsionen, wie der Epilepsie.

Die antikonvulsiven Eigenschaften der erfindungsgemässen Verbindungen können beispielsweise an der Maus anhand ihrer ausgeprägten Schutzwirkung gegenüber durch Elektroschock ausgelösten oder audiogen hervorgerufenen Konvulsionen bestimmt werden, wobei man z.B. das etablierte Elektroschock-Mausmodell bzw. die Versuchsanordnung gemäss Chapman et al., Arzneimittel-Forsch. 34, 1261 (1984) heranziehen kann.

Von EP-A 302 826 und EP-A 233 154 sind NMDA-antagonistisch wirksame Verbindungen bekannt, die der Formel I strukturähnlich sind, und in denen R₁ Halogenniederalkyl, Hydroxyniederalkyl oder Niederalkoxyniederalkyl bedeuten kann. Aus EP-A 233 154 sind insbesondere auch Verbindungen der Formel I namentlich vorbekannt, in denen R₁ Propyl bzw. Butyl bedeutet.

Die erfindungsgemässen Verbindungen zeichnen sich, insbesondere im Elektroschock-Mausmodell, durch eine gegenüber strukturverwandten Verbindungen verbesserte Wirkung aus.

Die die Eignung der erfindungsgemäss bereitgestellten Verbindungen für die Behandlung von Migräne begründenden antispastischen Eigenschaften können beispielsweise an der Ratte anhand ihrer depressionshemmenden Wirkung in der frontalen Cortex gemäss der Versuchsanordnung von R.Marannes et al., Brain Res. 457, 226 (1988) gezeigt werden. In diesem Modell senken die erfindungsgemäss bereitgestellten Verbindungen im Dosisbereich von etwa 3 bis 30 mg/kg ip. den Schwellenwert der "spreading depression" und verkürzen deren Dauer.

Die Erfindung betrifft in erster Linie Verbindungen der Formel I, worin R₁ Mono- oder Dihydroxyniederalkyl, Niederalkanoyloxyniederalkyl, Benzoyloxyniederalkyl, Niederalkoxyniederalkyl, Phenylniederalkoxyniederalkyl, Halogenniederalkyl, Aminoniederalkyl, Niederalkylaminoniederalkyl, Niederalkanoylaminoniederalkyl, Diniederalkylaminoniederalkyl, N-Niederalkyl-N-niederalkanoyl-aminoniederalkyl, 5- bis 7-gliedriges Azacycloalkylniederalkyl, dessen Azacyloalkylteil über das N- oder ein C-Atom gebunden und im zweitgenannten Fall gegebenenfalls N-niederalkyliert, N-niederalkanoyliert oder durch eine im Phenylteil gegebenenfalls substituierte Benzoylgruppe N-substituiert sein kann, 5- bis 7-gliedriges Diazacycloalkylniederalkyl, dessen Diazacycloalkylteil über ein N-Atom gebunden und gegebenenfalls N'-niederalkyliert, N'-niederalkanoyliert oder durch eine im Phenylteil gegebenenfalls substituierte Benzoylgruppe N'-substituiert ist, 5- bis 7-gliedriges, über das N-Atom gebundenes Azoxacycloalkylniederalkyl, 5- bis 7-gliedriges, über ein C-Atom gebundenes Oxacycloalkylniederalkyl, 5- bis 7-gliedriges, über ein C-Atom gebundenes, gegebenenfalls N-niederalkyliertes, N-niederalkanoyliertes oder durch eine im Phenylteil gegebenenfalls substituierte Benzoylgruppe N-substituiertes Azacycloalkyl oder 5- bis 7-gliedriges, über ein C-Atom gebundenes Oxacycloalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihre Salze.

Die Erfindung betrifft in erster Linie beispielsweise Verbindungen der Formel I, worin R₁ Hydroxyniederalkyl, Niederalkoxyniederalkyl, Phenylniederalkoxyniederalkyl, Aminoniederalkyl, Niederalkylaminoniederalkyl, Diniederalkylaminoniederalkyl, N,N-Niederalkylenamino bzw. N,N-(Aza- oder Oxaniederalkylen)aminoniederalkyl oder Halogenniederalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihre Salze.

Die Erfindung betrifft vor allem Verbindungen der Formel I, worin R₁ Hydroxy-C₁-C₇-alkyl, wie Hydroxymethyl, 2-Hydroxyäthyl, 3-Hydroxypropyl oder 4-Hydroxybutyl, Dihydroxy-C₂-C₇-alkyl, wie 1,3-Dihydroxyprop-2-yl, C₂-C₇-Alkanoyloxy-C₁-C₇-alkyl, wie Acetoxymethyl, Propionyloxymethyl, Butyryloxymethyl, 2-Acetyloxyäthyl, 3-Acetyloxypropyl, 4-Acetyloxybutyl, 5-Acetyloxypentyl oder 6-Acetyloxyhexyl, im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Benzoyloxy-C₁-C₇-alkyl, wie Benzoyloxymethyl, 2-Benzoyloxyäthyl, 3-Benzoyloxypropyl, 4-Benzoyloxybutyl, 5-Benzoyloxypentyl oder 6-Benzoyloxyhexyl, C₁-C₄-Alkoxy-C₁-C₇-alkyl, wie Methoxymethyl, Äthoxymethyl, 2-Methoxyäthyl, 3-Methoxypropyl oder 4-Methoxybutyl, eine im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituierte Phenyl-C₁-C₄-alkoxy-C₁-C₇-alkylgruppe, wie Benzyloxymethyl, 2-Benzyloxyäthyl, 3-Benzyloxypropyl oder 4-Benzyloxybutyl, Halogen-C₁-C₇-alkyl, wie Halogenmethyl, 2-Halogenäthyl, 3-Halogenpropyl, 4-Halogenbutyl, 5-Halogenpentyl oder 6-Halogenhexyl, wobei Halogen Chlor oder insbesondere Fluor bedeutet, Amino-C₁-C₇-alkyl, wie Aminomethyl, 2-Aminoäthyl, 3-Aminopropyl, 4-Aminobutyl, 5-Aminopentyl oder 6-Aminohexyl, C₁-C₄-Alkylamin-C₁-C₇-alkyl, wie Methylaminomethyl, Äthylaminomethyl, Propylaminomethyl, Butylaminomethyl, 2-Methylaminoäthyl, 3-Methylaminopropyl, 4-Methylaiminobutyl, 5-Methylaminopentyl oder 6-Methylaminohexyl, C₂-C₇-Alkanoylamino-C₁-C₇-alkyl, wie Acetylaminomethyl, Propionylaminomethyl, Butyrylaminomethyl, 2-Acetylaminoäthyl, 3-Acetylaminopropyl, 4-Acetylaminobutyl, 5-Acetylaminopentyl oder 6-Acetylaminohexyl, N-C₂-C₇-Alkanoyl-N-C₁-C₄-alkylamino-C₁-C₇-alkyl, wie N-Acetyl-N-methyl-aminomethyl, N-Acetyl-N-äthylaminomethyl, N-Propionyl-N-methyl-aminomethyl, N-Butyryl-N-methylamino-methyl, 2-(N-Acetyl-N-methyl-amino)äthyl, 2-(N-Propionyl-N-methyl-amino)äthyl, 2-(N-Acetyl-N-äthyl-amino)äthyl, 3-(N-Acetyl-N-methyl-amino)propyl, 4-(N-Acetyl-N-methylamino)butyl, 5-(N-Acetyl-N-methyl-amino)pentyl oder 6-(N-Acetyl-N-methyl-amino)hexyl, Di-C₁-C₇-Alkylamino-C₁-C₇-alkyl, wie Dimethylaminomethyl, Diäthylaminomethyl, N-Äthyl-N-methyl- aminomethyl, N-Methyl-N-propyl-aminomethyl, Dipropylaminomethyl, Dibutylaminomethyl, 2-Dimethylaminoäthyl, 3-Dimethylaminopropyl, 4-Dimethylaminobutyl, 5-Dimethylaminopentyl oder 6-Dimethylaminohexyl, Azacycloalk-1-yl-C₁-C₇-alkyl, wie Pyrrolidinomethyl, Piperidinomethyl, 2-Pyrrolidinoäthyl, 2-Piperidinoäthyl, 3-Pyrrolidinopropyl, 3-Piperidinopropyl, 4-Pyrrolidinobutyl, 4-Piperidinobutyl, 5-Pyrrolidinopentyl, 5-Piperidinopentyl, 6-Pyrrolidinohexyl oder 6-Piperidinohexyl, Azacycloalk-3-yl-C₁-C₇-alkyl bzw. 4-yl-C₁-C₇-alkyl, z.B. Piperidin-4-ylmethyl, 2-(Piperidin-4-yl)äthyl, 3-(Piperidin-4-yl)propyl oder 4-(Piperidin-4-yl)butyl, 1-C₂-C₇-Alkanoylazacycloalk-3-yl-C₁-C₇-alkyl bzw. 4-yl-C₁-C₇-alkyl, z.B. 1-Acetylpiperidin-4-ylmethyl, 2-(1-Acetylpiperidin-4-yl)äthyl, 3-(1-Acetylpiperidin-4-yl)propyl oder 4-(1-Acetylpiperidin-4-yl)butyl, N-C₁-C₄-Alkylazacycloalk-3-yl-C₁-C₇-alkyl bzw. 4-yl-C₁-C₇-alkyl, z.B. 1-Methylpiperidin-4-ylmethyl, 1-Äthylpiperidin-4-ylmethyl, 2-(1-Methylpiperidin-4-yl)äthyl, 2-( 1-Äthylpiperidin-4-yl)äthyl, 3-(1-Methylpiperidin-4-yl)propyl, 3-(1-Äthylpiperidin-4-yl)propyl, 4-(1-Methylpiperidin-4-yl)butyl oder 4-(1-Äthylpiperidin-4-yl)butyl, N-C₁-C₄-Benzoylazacycloalk-3-yl-C₁-C₇-alkyl bzw. 4-yl-C₁-C₇-alkyl, z.B. 1-Benzoylpiperidin-4-ylmethyl, 2-(1-Benzoylpiperidin-4-yl)äthyl, 3-(1-Benzoylpiperidin-4-yl)propyl oder 4-(1-Benzoylpiperidin-4-yl)butyl, Diazacycloalk-1-yl-C₁-C₇-alkyl, N'-C₁-C₄-Alkyldiazacycloalk-1-yl-C₁-C₇-alkyl oder N'-C₂-C₇-Alkanoylazacycloalk-1-yl-C₁-C₇alkyl, beispielsweise Piperazino- bzw. N'-Methyl- bzw. N'-Acetylpiperazinomethyl, 2-(Piperazino- bzw. N'-Methyl- bzw. N'-Acetylpiperazino)äthyl, 3-(Piperazino- bzw. N'-Methyl- bzw. N'-Acetylpiperazino)propyl oder 4-(Piperazino- bzw. N'-Methyl- bzw. N'-Acetylpiperazino)butyl, Azoxacycloalk-1-yl-C₁-C₇-alkyl, beispielsweise Morpholinomethyl, 2-Morpholinoäthyl, 3-Morpholinopropyl oder 4- Morpholinobutyl, 5- bis 7-gliedriges Oxacycloalk-3-yl-C₁-C₇-alkyl oder -4-yl-C₁-C₇-alkyl, wie Tetrahydropyran-4-yl-methyl, 2-(Tetrahydropyran-4-yl)äthyl, 3-(Tetrahydropyran-4-yl)propyl oder 4-(Tetrahydropyran-4-yl)butyl, 5- bis 7-gliedriges Azacycloalk-3-yl bzw. 4-yl oder 1-C₂-C₇-Alkanoyl-azacycloalk-3-yl bzw. 4-yl, z.B. Piperidin-4-yl oder 1-Acetylpiperidin-4-yl, N-C₁-C₄-Alkylazacycloalk-3-yl bzw. 4-yl oder im Phenylteil gegebenenfalls substituiertes N-Benzoylazacycloalk-3-yl oder -4-yl, z.B. 1-Methylpiperidin-4-yl oder 1-Benzoylpiperidin-4-yl, oder 5- bis 7-gliedriges Oxacycloalk-3-yl bzw. 4-yl, z.B. Tetrahydropyran-4-yl, darstellt und R₂ Carboxy, C₁-C₄-Alkoxycarbonyl, wie Methoxycarbonyl oder Äthoxycarbonyl, 5- bis 7-gliedriges Cycloalkoxycarbonyl, wie Cyclopentyloxy- oder Cyclohexyloxycarbonyl, oder gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxycarbonyl, wie Benzyloxycarbonyl oder 2-Phenyläthoxycarbonyl, ist, und ihre Salze, insbesondere ihre pharmazeutisch verwendbaren Salze.

Die Erfindung betrifft vor allem beispielsweise Verbindungen der Formel I, worin R₁ Hydroxy-C₁-C₇-alkyl, wie Hydroxymethyl, 2-Hydroxyäthyl, 3-Hydroxypropyl oder 4-Hydroxybutyl, C₁-C₄-Alkoxy-C₁-C₇-alkyl, wie Methoxymethyl, Äthoxymethyl, 2-Methoxyäthyl, 2-Äthoxyäthyl, 3-Methoxypropyl oder 4-Methoxybutyl, eine im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono-, di- oder trisubstituierte Phenyl-C₁-C₄-alkoxy-C₁-C₇-alkyl-, wie Benzyloxymethyl-, 2-Benzyloxyäthyl-, 3-Benzyloxypropyl- oder 4-Benzyloxybutylgruppe, Amino-C₁-C₇-alkyl, wie Aminomethyl, 2-Aminoäthyl, 3-Aminopropyl, 4-Aminobutyl, 5-Aminopentyl oder 6-Aminohexyl, C₁-C₄-Alkylamino-C₁-C₇-alkyl, wie Methylaminomethyl, Äthylaminomethyl, Propylaminomethyl, Butylaminomethyl, 2-Methylaminoäthyl, 3-Methylaminopropyl, 4-Methylaminobutyl, 5-Methylaminopentyl oder 6-Methylaminohexyl, C₂-C₇-Alkanoylamino-C₁-C₇-alkyl, wie Acetylaminomethyl, Propionylaminomethyl, Butyrylaminomethyl, 2-Acetylaminoäthyl, 3-Acetylaminopropyl, 4-Acetylaminobutyl, 5-Acetylaminopentyl oder 6-Acetylaminohexyl, Di-C₁-C₇-Alkylamino-C₁-C₇-alkyl, wie Dimethylaminomethyl, Diäthylaminomethyl, N-Äthyl-N-methyl- aminomethyl, N-Methyl-N-propyl-aminomethyl, Dipropylaminomethyl, Dibutylaminomethyl, 2-Dimethylaminoäthyl, 3-Dimethylaminopropyl, 4-Dimethylaminobutyl, 5-Dimethylaminopentyl oder 6-Dimethylaminohexyl, 5- bis 7-gliedriges N,N-Alkylenamino-C₁-C₇-alkyl bzw. N,N-(Aza- oder Oxaalkylen)amino-C₁-C₇-alkyl, wie Pyrrolidinomethyl, Piperidinomethyl, Morpholinomethyl, Piperazino- bzw. N'-Methyl- bzw. N'-Acetylpiperazinomethyl, 2-Pyrrolidinoäthyl, 2-Piperidinoäthyl, 2-Morpholinoäthyl, 3-Pyrrolidinopropyl, 3-Piperidinopropyl, 3-Morpholinopropyl, 4-Pyrrolidinobutyl, 4-Piperidinobutyl, 5-Pyrrolidinopentyl, 5-Piperidinopentyl oder 6-Piperidinohexyl, oder Halogen-C₁-C₇-alkyl, wie Halogenmethyl, 2-Halogenäthyl, 3-Halogenpropyl, 4-Halogenbutyl, 5-Halogenpentyl oder 6-Halogenhexyl, darstellt, wobei Halogen Chlor oder insbesondere Fluor bedeutet und R₂ Carboxy, C₁-C₄-Alkoxycarbonyl, wie Methoxycarbonyl oder Äthoxycarbonyl, 5- bis 7-gliedriges Cycloalkoxycarbonyl, wie Cyclopentyloxy- oder Cyclohexyloxycarbonyl, oder gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxycarbonyl, wie Benzyloxycarbonyl oder 2-Phenyläthoxycarbonyl, ist, und ihre Salze, insbesondere ihre pharmazeutisch verwendbaren Salze

Die Erfindung betrifft vorzugsweise Verbindungen der Formel I, worin R₁ Hydroxy-C₁-C₇-alkyl, wie Hydroxymethyl, 2-Hydroxyäthyl oder 3-Hydroxypropyl, im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Benzoyloxy-C₁-C₇-alkyl, wie 2-Benzoyloxyäthyl, C₁-C₄-Alkoxy-C₁-C₇-alkyl, wie Äthoxymethyl oder 2-Methoxyäthyl, im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxy-C₁-C₇-alkyl, wie Benzyloxymethyl oder 2-Benzyloxyäthyl, Halogen-C₁-C₇-alkyl, wie Halogenmethyl, 2-Halogenäthyl, wobei Halogen Chlor oder insbesondere Fluor bedeutet, Amino-C₄-C₇-alkyl, wie 4-Aminobutyl oder 6-Aminohexyl, N-C₂-C₇-Alkanoyl-N-C₁-C₄-alkyl-amino-C₁-C₇-alkyl, wie 2-(N-Acetyl-N-methyl-amino)äthyl, 5- bis 7-gliedriges Azacycloalk-3-yl bzw. 4-yl oder 1-C₂-C₇-Alkanoyl-azacycloalk-3-yl bzw. 4-yl, z.B. Piperidin-4-yl oder 1-Acetylpiperidin-4yl, darstellt und R₂ Carboxy, C₁-C₄-Alkoxycarbonyl, wie Methoxycarbonyl oder Äthoxycarbonyl, oder gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxycarbonyl, wie Benzyloxycarbonyl oder 2-Phenyläthoxycarbonyl, ist, und ihre Salze, insbesondere ihre pharmazeutisch verwendbaren Salze.

Die Erfindung betrifft insbesondere einerseits Verbindungen der Formel I, worin R₁ Hydroxy-C₁-C₄-alkyl, wie Hydroxymethyl, 2-Hydroxyäthyl, 3-Hydroxypropyl oder 4-Hydroxybutyl, C₁-C₄-Alkoxy-C₁-C₄-alkyl, wie Methoxymethyl, Äthoxymethyl, 2-Methoxyäthyl, 3-Methoxypropyl oder 4-Methoxybutyl, eine im Phenylteil gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono-, di- oder trisubstituierte Phenyl-C₁-C₄-alkoxy-C₁-C₄-alkyl-, wie Benzyloxymethyl, 2-Benzyloxyäthyl-, 3-Benzyloxypropyl- oder 4-Benzyloxybutylgruppe, oder Halogen-C₁-C₄-alkyl, wie Halogenmethyl, 2-Halogenäthyl, 3-Halogenpropyl, 4-Halogenbutyl, 5-Halogenpentyl oder 6-Halogenhexyl, darstellt, wobei Halogen Chlor oder insbesondere Fluor bedeutet und R₂ Carboxy, C₁-C₄-Alkoxycarbonyl, wie Methoxycarbonyl oder Äthoxycarbonyl, 5- bis 7-gliedriges Cycloalkoxycarbonyl, wie Cyclopentyloxy- oder Cyclohexyloxycarbonyl, oder gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxycarbonyl, wie Benzyloxycarbonyl oder 2-Phenyläthoxycarbonyl, ist, und ihre Salze, insbesondere ihre pharmazeutisch verwendbaren Salze.

Die Erfindung betrifft vor allem andrerseits Verbindungen der Formel I, worin R₁ Amino-C₁-C₇-alkyl, wie Aminomethyl, 2-Aminoäthyl, 3-Aminopropyl, 4-Aminobutyl, 5-Aminopentyl, 6-Aminohexyl oder 7-Aminoheptyl, C₁-C₄-Alkylamino-C₁-C₇-alkyl, wie Methylaminomethyl, Äthylaminomethyl, Propylaminomethyl, Butylaminomethyl, 2-Methylaminoäthyl, 3-Methylaminopropyl, 4-Methylaminobutyl, 5-Methylaminopentyl oder 6-Methylaminohexyl, C₂-C₇-Alkanoylamino-C₁-C₇-alkyl, wie Acetylaminomethyl, Propionylaminomethyl, Butyrylaminomethyl, 2-Acetylaminoäthyl, 3-Acetylaminopropyl, 4-Acetylaminobutyl, 5-Acetylaminopentyl oder 6-Acetylaminohexyl, Di-C₁-C₄-Alkylamino-C₁-C₇-alkyl, wie Dimethylaminomethyl, Diäthylaminomethyl, N-Äthyl-N-methyl-aminomethyl, N-Methyl-N-propyl-aminomethyl, Dipropylaminomethyl, Dibutylaminomethyl, 2-Dimethylaminoäthyl, 3-Dimethylaminopropyl, 4-Dimethylaminobutyl, 5-Dimethylaminopentyl oder 6-Dimethylaminohexyl, 5- bis 7-gliedriges N,N-Alkylenamino-C₁-C₇-alkyl bzw. N,N-(Aza- oder Oxaalkylen)amino-C₁-C₇-alkyl, wie Pyrrolidinomethyl, Piperidinomethyl, Morpholinomethyl, Piperazino- bzw. N'-Methyl- bzw. N'-Acetylpiperazinomethyl, Pyrrolidinoäthyl, Piperidinoäthyl, Morpholinoäthyl, Pyrrolidinopropyl, Piperidinopropyl, Morpholinopropyl, Pyrrolidinobutyl, Piperidinobutyl, Pyrrolidinopentyl, Piperidinopentyl oder Piperidinohexyl, bedeutet und R₂ Carboxy, C₁-C₄-Alkoxycarbonyl, wie Methoxycarbonyl oder Äthoxycarbonyl, 5- bis 7-gliedriges Cycloalkoxycarbonyl, wie Cyclopentyloxy oder Cyclohexyloxycarbonyl, oder gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituiertes Phenyl-C₁-C₄-alkoxycarbonyl, wie Benzyloxycarbonyl oder 2-Phenyläthoxycarbonyl, ist, und ihre Salze, insbesondere ihre pharmazeutisch verwendbaren Salze.

Die Erfindung betrifft ganz besonders Verbindungen der Formel I, worin R₁ Hydroxy-C₁-C₇-alkyl, wie Hydroxyäthyl, C₁-C₄-Alkoxy-C₁-C₇-alkyl, wie Methoxymethyl, Äthoxymethyl oder 2-Methoxyäthyl, Benzoyloxy-C₁-C₄-alkyl, wie 2-Benzoyloxyäthyl, Phenyl-C₁-C₄-alkoxy-C₁-C₇-alkyl, wie Benzyloxymethyl oder 2-Benzyloxyäthyl, Amino-C₄-C₇-alkyl, wie 4-Aminobutyl, oder 6-Aminohexyl, N-C₂-C₇-Alkanoyl-N-C₁-C₄-alkyl-amino-C₂-C₇-alkyl, wie 2-(N-Acetyl-N-methylamino)äthyl, 5- bis 7-gliedriges Azacycloalk-3-yl bzw. 4-yl oder 1-C₂-C₇-Alkanoyl-azacycloalk-3-yl bzw. 4-yl, wie Piperidin-4-yl oder 1-Acetylpiperidin-4-yl, oder Halogen-C₁-C₄-alkyl, wobei Halogen Chlor oder insbesondere Fluor bedeutet, wie 2-Halogenäthyl, darstellt und R₂ Carboxy oder C₁-C₄-Alkoxycarbonyl, wie Methoxycarbonyl oder Äthoxycarbonyl, bedeutet, und ihre Salze, insbesondere ihre pharmazeutisch verwendbaren Salze.

Bevorzugter Gegenstand der Erfindung sind einerseits Verbindungen der Formel I, worin R₁ Amino-C₄-C₇-alkyl, wie 4-Aminobutyl oder 6-Aminohexyl, N-C₁-C₇-Alkanoyl-N-C₁-C₄-alkyl-amino-C₁-C₇-alkyl, wie 2-(N-Acetyl-N-methyl-amino)äthyl, 5-bis 7-gliedriges, über ein C-Atom gebundenes Azacycloalkyl, wie Piperidin-4-yl oder 5-bis 7-gliedriges, über ein C-Atom gebundenes (N-C₂-C₇-Alkanoylaza)cycloalkyl, wie 1-C₂-C₇-Alkanoylpiperidin-4-yl, z.B. 1-Acetylpiperidin-4-yl, bedeutet und R₂ Carboxy oder C₁-C₄-Alkoxycarbonyl, wie Methoxycarbonyl oder Äthoxycarbonyl, ist, und ihre Salze.

Bevorzugter Gegenstand der Erfindung sind andrerseits Verbindungen der Formel I, worin R₁ C₁-C₄-Alkoxy-C₁-C₄-alkyl, wie Methoxymethyl, Äthoxymethyl oder 2-Methoxyäthyl, Phenyl-C₁-C₄-alkoxy-C₁-C₄-alkyl, wie Benzyloxymethyl oder 2-Benzyloxyäthyl, Benzoyloxy-C₁-C₄-alkyl, wie 2-Benzoyloxyäthyl, Hydroxy-C₁-C₄-alkyl, wie Hydroxymethyl oder 2-Hydroxyäthyl, oder Halogen-C₂-C₄-alkyl, wie 2-Fluoräthyl, bedeutet und R₂ Carboxy oder C₁-C₄-Alkoxycarbonyl, wie Methoxycarbonyl oder Äthoxycarbonyl, darstellt, und ihre Salze.

Die Erfindung betrifft vornehmlich einerseits Verbindungen der Formel I, worin R₁ Amino-C₄-C₇-alkyl, wie 4-Aminobutyl, 5-Aminopentyl, 6-Aminohexyl oder 7-Aminoheptyl, bedeutet und R₂ Carboxy, C₁-C₄-Alkoxycarbonyl, wie Methoxycarbonyl oder Äthoxycarbonyl, oder eine gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituierte Phenyl-C₁-C₄-alkoxycarbonyl-, wie Benzyloxycarbonyl- oder 2-Phenyläthoxycarbonylgruppe, darstellt, und ihre Salze, insbesondere ihre pharmazeutisch verwendbaren Salze.

Die Erfindung betrifft vornehmlich andrerseits Verbindungen der Formel I, worin R₁ C₁-C₄-Alkoxy-C₂-C₄-alkyl, wie 2-Methoxyäthyl, 2-Äthoxyäthyl, 3-Methoxypropyl oder 4-Methoxybutyl, Hydroxy-C₂-C₄-alkyl, wie 2-Hydroxyäthyl, 3-Hydoxypropyl oder 4-Hydroxybutyl, oder Halogen-C₂-C₄-alkyl, wie 2-Fluoräthyl, 2-Chloräthyl, 3-Fluorpropyl oder 4-Fluorbutyl, bedeutet und R₂ Carboxy, C₁-C₄-Alkoxycarbonyl, wie Methoxycarbonyl oder Äthoxycarbonyl, oder eine gegebenenfalls durch C₁-C₄-Alkyl, wie Methyl, C₁-C₄-Alkoxy, wie Methoxy, Halogen der Atomnummer bis und mit 35, wie Fluor oder Chlor, Cyano und/oder Trifluormethyl mono- oder disubstituierte Phenyl-C₁-C₄-alkoxycarbonyl-, wie Benzyloxycarbonyl- oder 2-Phenyläthoxycarbonylgruppe darstellt, und ihre Salze, insbesondere ihre pharmazeutisch verwendbaren Salze.

Die Erfindung betrifft in allererster Linie Verbindungen der Formel I, worin R₁ 2-Hydroxyäthyl, 3-Hydroxypropyl, Hydroxymethyl, Methoxymethyl, Äthoxymethyl, 2-Methoxyäthyl, 2-Benzoyloxyäthyl, Benzyloxymethyl, 2-Benzyloxyäthyl, 4-Aminobutyl, 6-Aminohexyl, 2-(N-Acetyl-N-methyl-amino)äthyl, Piperidin-4-yl, 1-Acetylpiperidin-4-yl oder 2-Fluoräthyl darstellt und R₂ Carboxy oder C₁-C₄-Alkoxycarbonyl bedeutet, und ihre Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung der erfindungsgemässen Verbindungen ist dadurch gekennzeichnet, dass man
in einer Verbindung der Formel II 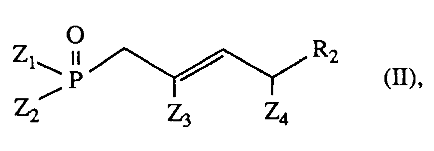
worin Z₁, Z₂ fur gegebenenfalls geschütztes Hydroxy stehen, Z₃ einen einen durch gegebenenfalls geschütztes oder acyliertes oder aliphatisch oder araliphatisch veräthertes Hydroxy, durch Halogen, durch gegebenenfalls geschütztes oder acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls geschützten oder aliphatisch N-substituierten oder N-acylierten azacycloaliphatischen Kohlenwasserstoffrest darstellt und Z₄ geschütztes Amino bedeutet, geschütztes Amino Z₄ und, sofern vorhanden, als Bestandteil von Z₃ in Amino und, sofern vorhanden, geschütztes Hydroxy Z₁, Z₂ und/oder als Bestandteil von Z₃ in Hydroxy überführt und, sofern vorhanden einen geschützten azacycloaliphatischen Kohlenwasserstoffrest Z₃ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

In Ausgangsstoffen der Formel II bedeutet geschütztes Hydroxy Z₁ und/oder Z₂ beispielsweise veräthertes, insbesondere aliphatisch oder aromatisch veräthertes Hydroxy, geschütztes Hydroxy als Bestandteil von Z₃ beispielsweise acyliertes oder silyliertes Hydroxy, und geschütztes Amino Z₄ und, sofern vorhanden, als Bestandteil von Z₃ beispielsweise acyliertes Amino.

Aliphatisch veräthertes Hydroxy ist beispielsweise Niederalkoxy, wie Methoxy, Äthoxy oder insbesondere Isopropyloxy. Aromatisch veräthertes Hydroxy ist beispielsweise gegebenenfalls durch Niederalkyl, Niederalkoxy, Halogen, Cyano und/oder Nitro substituiertes Phenoxy.

Acyliertes Hydroxy weist als Acylgruppe beispielsweise den Acylrest einer araliphatischen Carbonsäure oder eines Halbesters der Kohlensäure auf und bedeutet beispielsweise Niederalkanoyloxy, oder eine im Phenylteil gegebenenfalls durch Niederalkyl, Niederalkoxy, Halogen, Cyano und/oder Trifluoromethyl substituierte Phenylniederalkanoyloxy- oder Phenylniederalkoxycarbonyloxygruppe, z.B. Benzyloxycarbonyloxy.

Silyliertes Hydroxy ist beispielsweise Triniederalkylsilyloxy, z.B. Trimethyl- oder Tributylsilyloxy.

Acyliertes Amino weist als Acylgruppe beispielsweise von einer geeigneten organischen Säure, wie von Ameisensäure oder einem araliphatischen oder aromatischen Halbester der Kohlensäure abgeleitetes Acyl auf. Acyliertes Amino bedeutet somit beispielsweise Formylamino, Niederalkoxycarbonylamino, wie Methoxy-, Äthoxy- oder Tertiärbutyloxycarbonylamino, gegebenenfalls im Phenylteil durch Niederalkyl, Niederalkoxy, Halogen, Cyano und/oder Nitro substituiertes Phenylniederalkoxycarbonylamino, wie Benzyloxycarbonylamino, oder durch Niederalkyl, Niederalkoxy, Halogen, Cyano und/oder Nitro substituiertes Phenoxycarbonylamino.

Die Freisetzung der geschützten Gruppen aus Verbindungen der Formel II, d.h. von Hydroxy aus geschützten Hydroxygruppen Z₁, Z₂ und/oder aus geschützten Hydroxygruppen als Bestandteil von Z₃ bzw. von Amino aus geschützten Aminogruppen Z₄ und, sofern vorhanden, aus geschützten Aminogruppen als Bestandteil von Z₃, erfolgt beispielsweise durch Behandeln mit einem sauren Mittel, beispielsweise mit einem Triniederalkylhalogensilan, wie Trimethylbromsilan, Tributylbromsilan oder Trimethyljodsllan. Dabei arbeitet man vorzugsweise in einem inerten Lösungsmittel, wie einem halogenierten aliphatischen Kohlenwasserstoff, z.B. in Dichlormethan oder in zweiter Linie Tri- oder Tetrachlormethan, Trichloräthan oder Tetrachloräthan, beispielsweise im Temperaturbereich von etwa -25° bis etwa +50°C, vorzugsweise von etwa 0° bis 30°C, z.B. bei Raumtemperatur, d.h. bei etwa 15° bis 25°C, vorteilhaft unter weitgehend wasserfreien Bedingungen und unter Inertgas, wie Argon oder Stickstoff. Die Aufarbeitung erfolgt vorteilhaft unter Hinzufügen eines Halogenwasserstoffabfängers, insbesondere einer aliphatischen Epoxyverbindung, wie eines Epoxyniederalkans, z.B. von Propylenoxid in einem Niederalkanol, wie Äthanol.

In einer bevorzugten Ausführungsform geht man beispielsweise von Verbindungen der Formel II aus, worin Z₁ und Z₂ Niederalkoxy, z.B. Isopropyloxy, bedeuten und Z₄ Niederalkanoylamino, wie Formylamino, darstellt und behandelt diese in einem aliphatischen Halogenkohlenwasserstoff, wie Dichlormethan, bei etwa 15° bis etwa 25°C mit einem Triniederalkylbromsilan, wie Trimethylbromsilan oder Tributylbromsilan, lässt einige Zeit, z.B. etwa 2 bis 30 Stunden, ausreagieren, fügt dann eine äthanolische Lösung von Propylenoxid hinzu und filtriert das Prodükt ab.

Ausgangsstoffe der Formel II werden beispielsweise hergestellt, indem man einen α,β-ungesättigten Aldehyd der Formel IIa 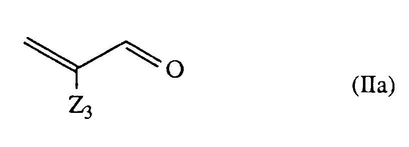
mit einem α-Isocyanessigsäureester der Formel IIb 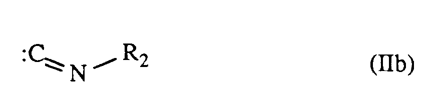
in an sich bekannter Weise, beispielsweise in Gegenwart eines Kupfer- oder Goldkatalysators, z.B. von Kupfer-I-oxid oder Bis(cyclohexylisocyanid)gold-I-tetrafluoroborat, zu dem entsprechenden 5-substituierten 2-Oxazolin-4-carbonsäureester der Formel IIc 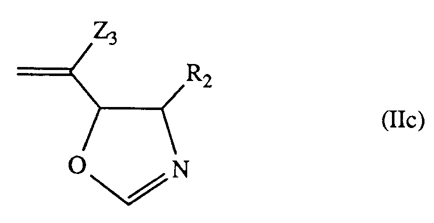
umsetzt, diesen durch Hydrolyse, z.B. in wässrigem Tetrahydrofuran, in den entsprechenden offenkettigen Ester der Formel IId 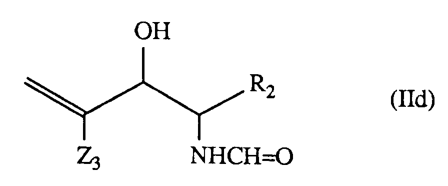
überführt, diesen durch Behandeln mit Thionylbromid in an sich bekannter Weise in den entsprechenden ω-Bromester der Formel IIe 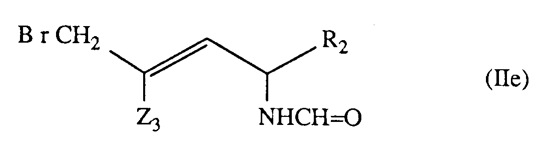
umwandelt und diesen in an sich bekannter Weise mit einem Phosphorigsäuretriester der Formel P(Zₐ)(Z_{b})(Z_{c}), worin Zₐ, Z_{b} und Z_{c} für gleiche oder verschiedene in einer Ätherform geschützte Hydroxygruppen stehen, wie einem Triniederalkylphosphit, z.B. mit Triisopropylphosphit, zur entsprechenden Verbindung der Formel II' 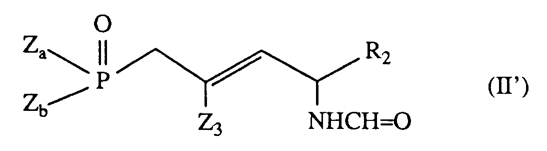
weiterumsetzt.

Verfahrensgemäss erhaltliche Verbindungen können in üblicher Weise in andere Verbindungen der Formel I überführt werden.

So kann man man freie und veresterte Carboxygruppen R₂ in üblicher Weise ineinander überführen. Insbesondere kann man verestertes Carboxy R₂ durch Hydrolyse in Carboxy bzw. freies Carboxy R₂ durch Umsetzung mit einem Alkohol in verestertes Carboxy überführen. Ferner kann man verestertes Carboxy R₂ zu einer anderen veresterten Carboxygruppe umestern. Dabei arbeitet man in üblicher Weise unter hydrolytischen, alkoholytischen oder umesternden Bedingungen.

Die Hydrolyse von Carbonsäureestern (I; R₂= verestertes Carboxy) erfolgt in üblicher Weise, erforderlichenfalls in Gegenwart eines sauren oder basischen Mittels, wie einer Mineralsäure, z.B. von Chlorwasserstoff oder Schwefelsäure, oder einer Base, wie eines Alkalimetallhydroxides, z.B. von Natriumhydroxid.

Bei der Umesterung von Estern (I; R₂= verestertes Carboxy) mit Alkoholen arbeitet man üblicherweise unter Säure- oder basekatalytischen Bedingungen, beispielsweise in Gegenwart einer katalytischen Menge einer Mineralsäure, wie von Chlorwasserstoff oder Schwefelsäure, oder einer Metallbase, wie von Natriumhydroxid, oder indem man die Alkoholkomponente in Form eines Metallalkoholates, z.B. eines Alkalimetallalkoholates, einsetzt.

Ferner kann man in durch araliphatisch veräthertes Hydroxy substituierte aliphatische Kohlenwasserstoffresten, wie α-Phenylniederalkoxyniederalkylresten R₁ die α-Phenylniederalkoxygruppe reduktiv, z.B. durch Behandeln mit Wasserstoff in Gegenwart eines Hydrierungskatalysators, wie von Palladium auf Kohle oder Raney-Nickel, in Hydroxy überführen.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise Aminosäuren, insbesondere _{D}- oder _{L}-Lysin, optisch aktive Alkaloidbasen, wie Strychnin, Cinchonin oder Brucin, oder _{D}- oder _{L}-(1-Phenyl)äthylamin, 3-Pipecolin, Ephedrin. Amphetamin und ähnliche synthetisch zugängliche Basen, optisch aktive Carbon- oder Sulfonsäuren, wie Chinasäure oder _{D}- oder _{L}-Weinsäure, _{D}- oder _{L}-Di-o-toluylweinsäure, _{D}- oder _{L}-Äpfelsäure, _{D}- oder _{L}-Mandelsäure, oder _{D}- oder _{L}-Camphersulfonsäure, bzw. optisch aktive Alkohole, wie Borneol oder _{D}- oder _{L}--(1-Phenyl)äthanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiumsillkat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgen Beispiele dienen zur Illustration der Erfindung; Temperaturen sind in Celsiusgraden, Drucke in mbar angegeben.

Beispiel 1: 3,57g (8,5mMol) 6-Acetoxy-4-diisopropylphosphonomethyl-2-formylaminohex-3-ensäureäthylester werden in 22 ml Dichlormethan gelöst und bei Raumtemperatur tropfenweise mit 4,4 mi (34 mMol) Trimethylbromsilan versetzt. Man lässt 22 Stunden bei Raumtemperatur stehen, tropft 22 ml Äthanol hinzu, lässt nochmals 22 Stunden stehen, dampft am Rotationsverdampfer ein, löst den Rückstand in 22 ml Äthanol und tropft ein Gemisch von 22 ml Propylenoxid und 22 ml Äthanol hinzu. Es bildet sich eine Suspension, die noch 90 Minuten gerührt und dann abgesaugt wird. Man erhält 2-Amino-6-hydroxy-4-phosphonomethyl-hex-3-ensäureäthylester vom Smp. 195° (Zers.)

Das Ausgangsmaterial kann z.B. folgendermassen hergestellt werden:
13,0 g (100mMol) Essigsäure-(4-oxo)butylester, 92,0 g (112,6 mMol) Dimethylammoniumchlorid und 10,8 ml (117 mMol) 37%-ige Formaldehydlösung werden unter Rühren 1 Stunde auf 100° erhitzt. Man lässt abkühlen und extrahiert 3-mal mit je 30 ml Diäthyläther. Die organischen Phasen werden vereinigt, mit gesättigter Kochsalzlösung gewaschen, über Magnesiumsulfat getrocknet und zur Trockne eingedampft. Man erhält Essigsäure-(3-formyl)but-3-enylester als farbloses Öl, das ohne weitere Reinigung weiterumgesetzt werden kann.

13,5g (95 mMol) Essigsäure-(3-formyl)but-3-enylester und 10,4 g (95 mMol) Isocyanessigsäureäthylester werden zu einer Suspension von 0,38g Kupfer-I-oxid in 50 ml Benzol zugetropft. Nach Abklingen der exothermen Reaktion lässt man noch 45 Minuten bei Raumtemperatur nachrühren, filtriert über Hyflo® und dampft zur Trockne ein. Der Rückstand wird in 75 ml Tetrahydrofuran aufgenommen, mit 25 ml Wasser versetzt und unter Rühren 4 Stunden zum Rückfluss erhitzt. Man dampft zur Trockne ein und chromatographiert an Silicagel mit Toluol/ Isopropanol (9:1) als Elutionsmittel. Man erhält 6-Acetoxy-2-formylamino-3-hydroxy-4-methylen-hexansäureäthylester als bräunliches Öl.

9,19 g (35,9 mMol) 6-Acetoxy-2-formylamino-3-hydroxy-4-methylen-hexansäureäthylester werden in 100 ml Dichlormethan gelöst und bei Raumtemperatur tropfenweise mit 3,34 ml (43,1 mMol) Thionylbromid versetzt. Nach 1 Stunde gibt man 10 ml Wasser hinzu und lässt 10 Minuten intensiv rühren. Die organische Phase wird abgetrennt, nacheinander mit Wasser, gesättigter Kaliumhydrogencarbonatlösung und nochmals mit Wasser gewaschen, über Magnesiumsulfat getrocknet, filtriert und eingedampft. Man erhält 6-Acetoxy-4-brommethyl-2-formylamino-hex-3-ensäureäthylester als bräunliches Öl.

8,7 g (25 mMol) 6-Acetoxy-4-brommethyl-2-formylamino-hex-3-ensäureäthylester und 21 mi (75 mMol) Triisopropylphosphit (90%-ig) werden auf 80° bis 90°C erwärmt und unter einem Druck von etwa 100 mbar 19 Stunden gerührt. Das überschüssige Triisopropylphosphit wird abdestilliert und der Eindampfrückstand an 150 g Silicagel mit zunächst Essigsäureäthylester und dann Essigsäureäthylester/Äthanol (9:1) als Elutionsmittel chromatographiert. Man erhält 6-Acetoxy-4-diisopropylphosphonomethyl-2-formylamino-hex-3-ensäureäthylester als gelbliches Öl.

Beispiel 2: 0,415 g (1,55mMol) 2-Amino-6-hydroxy-4-phosphonomethyl-hex-3-en säureäthylester werden in 3 ml Wasser 24 Stunden zum Rückfluss erhitzt. Das Reaktionsgemisch wird eingedampft, an 10 g Silicagel mit Äthanol/Wasser (1:1) als Elutionsmittel chromatographisch gereinigt und aus Äthanol kristallisiert. Man erhält 2-Amino-6-hydroxy-4-phosphonomethyl-hex-3-ensäure vom Smp. >300°.

Beispiel 3: 0,5g (1,0 mMol) 8-(N-Benzyloxycarbonylamino)-4-diäthylphosphonomethyl-2-formylamino-oct-3-ensäuremethylester werden in 5,0 ml 6n-Salzsäure 6 Stunden zum Rückfluss erhitzt. Eindampfen zur Trockne ergibt 2,8-Diamino-4-phosphonomethyloct-3-ensäure-dihydrochlorid in Form eines gummiartigen Festkörpers, das aus Acetonitril umkristallisiert wird; Smp. 128° (Zers.)

Das Ausgangsmaterial kann folgendermassen hergestellt werden:
Zu einer Lösung von 5,52 g (47 mMol) 6-Aminohexan-1-ol und 3,95 g (47 mMol) Natriumhydrogencarbonat in 100 ml Aceton und 50 ml Wasser werden tropfenweise 7,06 mi (47 mMol) Chlorameisensäurebenzylester zugegeben. Man lässt 18 Stunden bei Raumtemperatur rühren, engt auf etwa 70 ml ein, filtriert den weissen Niederschlag ab, wäscht diesen mit etwa 20 ml Wasser, nimmt in 250 ml Methylenchlorid auf, trocknet über Magnesiumsulfat, filtriert dieses ab und dampft den Rückstand zur Trockne ein. Man erhält 6-(N-Benzyloxycarbonylamino)hexan-1-ol in Form weisser Kristalle vom Smp. 58-60°.

Zu einer bei -50° gerührten Lösung von 0,19 ml (2,20 mMol) Oxalylchlorid in 10 ml Methylenchlorid werden unter Stickstoff 0,32 ml (4,40 mMol) Dimethylsulfoxid zugetropft. Man lässt 15 Minuten rühren und gibt dann 0,5 g (2 mMol) 6-(N-Benzyloxycarbonylamino)hexan-1-ol hinzu. Man lässt 25 Minuten bei -50° nachrühren, gibt tropfenweise 1,78 ml (10 mMol) N-Äthyl-N,N-diisopropylamin hinzu und giesst in 10 ml Eiswasser. Die organische Phase wird abgetrennt und die Wasserphase mit 10 ml Methylenchlorid extrahiert. Die organischen Phasen werden vereinigt, 2-mal mitje 5 ml n-Salzsäure und 1-mal mit 10 ml gesättigter Kochsalzlösung gewaschen, über Magnesiumsulfat getrocknet und zur Trockne eingedampft. Das erhaltene Öl wird an Silicagel mit Hexan/Essigsäureäthylester (1:1) als Laufmittel chromatographisch gereinigt. Man erhält 6-(N-Benzyloxycarbonylamino)hexanal.

Zu einer Lösung von 1,5 g (17 mMol) wasserfreiem Piperazin und 2,03g (34 mMol) Essigsäure in 18,7 ml Wasser werden 2,44 g (30 mMol) 37%-ige wässrige Formaldehydlösung zugegeben. Man lässt 15 Minuten bei 25° rühren und fügt dann 7,48g (30 mMol) 6-(N-Benzyloxycarbonylamino)hexanal hinzu. Die Reaktionsmischung wird 2 Stunden zum Rückfluss erhitzt, dann mit Eiswasser gekühlt und 2-mal mit je 50 ml Methylenchlorid extrahiert. Die Auszüge werden vereinigt, 2-mal mit je 25 ml gesättigter Natriumhydrogencarbonatlösung und mit 25 ml gesättigter Kochsalzlösung gewaschen, getrocknet und zur Trockne eingedampft. Man erhält 6-(N-Benzyloxycarbonylamino)-2-methylen-hexanal als gelbliche Flüssigkeit.

4,0 g (15,3 mMol) 6-(N-Benzyloxycarbonylamino)-2-methylen-hexanal und 1,62 ml (16,8 mMol) Isocyanessigsäuremethylester werden in 50 ml Toluol gelöst und bei 40° zu einer Suspension von 0,12 g 96,4%-igem Kupfer-I-oxid in 50 ml Toluol zugetropft. Man lässt 2,5 Stunden bei Raumtemperatur nachrühren, filtriert, gibt auf eine mit 60 g Silicagel gefüllte Säule und extrahiert zunächst mit Hexan/Essigsäureäthylester (1:1) und dann mit Essigsäureäthylester. Man erhält 5-[6-(N-Benzyloxycarbonylamino)hex-1-en-2-yl]-oxazolin-4-carbonsäuremethylester; Öl.

9,4 g (26,1 mMol) 5-[6-(N-Benzyloxycarbonylamino)hex-1-en-2-yl]-oxazolin-4-carbonsäuremethylester werden in 40 ml Tetrahydrofuran und 20 ml Wasser gelöst, mit einigen Tropfen Triäthylamin versetzt und 18 Stunden zum Rückfluss erhitzt. Das Lösungsmittel wird unter vermindertem Druck abgezogen und das zurückbleibende Öl in insgesamt 125 ml Methylenchlorid aufgenommen, über Magnesiumsulfat getrocknet und zur Trockne eingedampft. Man erhält 8-(N-Benzyloxycarbonylamino)-2-formylamino-3-hydroxy-4-methylen-octansäuremethylester.

2,46 g (6,5 mMol) 8-(N-Benzyloxycarbonylamino)-2-formylamino-3-hydroxy-4-methylen-octansäuremethylester in 25 ml Tetrahydrofuran werden mit 5,5 ml (46 mMol) Hexa-1,5-dien und dann bei -50° tropfenweise mit 2,6 ml (32,5 mMol) Thionylbromid versetzt. Man lässt 2 Stunden bei 0° bis 5° nachrühren, giesst in 25 ml eiskalte gesättigte Natriumhydrogencarbonatlösung und extrahiert 2-mal mit je 20 ml Methylenchlorid. Die organische Phase wird mit 10 ml gesättigter Kochsalzlösung gewaschen, über Magnesiumsulfat getrocknet und zur Trockne eingedampft. Das erhaltene Öl wird an Silicagel mit Hexad/Essigsäureäthylester (3:1) chromatographisch gereinigt. Man erhält 8-(N-Benzyloxycarbonylamino)-4-brommethyl-2-formylamino-oct-3-ensäuremethylester.

1,45 g (3,3 mMol) 8-(N-Benzyloxycarbonylamino)-4-brommethyl-2-formylamino-oct-3-ensäuremethylester werden mit 5 ml Triäthylphosphit versetzt und unter Rühren 8 Stunden auf 75°erhitzt. Das überschüssige Triäthylphosphit wird unter vermindertem Druck abdestilliert und ergibt einen öligen Rückstand. Dieser wird an einer Silicagelsäule mit zunächst Essigsäureäthylester und dann Essigsäureäthylester/Methanol (9:1) chromatographisch gereinigt. Man erhält 8-(N-Benzyloxycarbonylamino)-4-diäthylphosphonomethyl-2-formylamino-oct-3-ensäuremethylester.

Beispiel 4: 1,77 g (4,5 mMol) 4-Diisopropylphosphonomethyl-2-formylamino-6-methoxy-hex-3-ensäureäthylester werden in 12 ml Dichlormethan gelöst und bei Raumtemperatur tropfenweise mit 2,32 ml (18,0 mMol) Trimethylbromsilan versetzt. Man lässt 22 Stunden bei Raumtemperatur stehen, tropft 12 ml Äthanol hinzu, lässt nochmals 24 Stunden stehen, dampft am Rotationsverdampfer ein, löst den Rückstand in 10 ml Äthanol und fügt ein Gemisch von 2 ml Propylenoxid und 2 ml Äthanol hinzu. Es bildet sich eine Suspension, die weitere 2 Stunden bei Raumtemperatur und 2 Stunden unter Eiskühlung gerührt und dann abgesaugt wird. Man erhält 2-Amino-6-methoxy-4-phosphonomethyl-hex-3-ensäureäthylester vom Smp. 242° (Zers.).

Das Ausgangsmaterial kann z.B. wie folgt hergestellt werden:
19,7 g (193 mMol) 4-Methoxybutanal, 17,7 g (217 mMol) Dimethylammoniumchlorid und 17,0 ml (226 mMol) 37%-ige Formaldehydlösung werden unter Rühren 3 Stunden auf 100° erhitzt Man lässt abkühlen und extrahiert 3-mal mit Diäthyläther. Die organischen Phasen werden mit gesättigter Kochsalzlösung gewaschen, vereinigt, über Natriumsulfat getrocknet, filtriert und zur Trockne eingedampft. Man erhält 4-Methoxy-2-methylenbutanal als gelbliches Öl, das ohne weitere Reinigung weiterumgesetzt werden kann.

16,5 g (144,5 mMol) 4-Methoxy-2-methylen-butanal und 15,8 ml (144,5 mMol) Isocyanessigsäureäthylester werden in 145 ml Toluol gelöst und mit 400 mg Kupfer-I-oxid versetzt. Nach Abklingen der exothermen Reaktion lässt man noch 2 Stunden nachrühren, filtriert über Hyflo® und dampft zur Trockne ein. Der Rückstand wird in 145 ml Tetrahydrofuran aufgenommen, mit 33 ml Wasser versetzt und unter Rühren 2 Stunden zum Rückfluss erhitzt. Man dampft zur Trockne ein, versetzt mit Toluol und dampft erneut ein. Chromatographie an Silicagel mit Toluol/Äthanol (95:5) als Elutionsmittel ergibt 2-Formylamino-3-hydroxy-6-methoxy-4-methylen-hexansäureäthylester als rotbraunes Öl.

19,0 g (77,5 mMol) 2-Formylamino-3-hydroxy-6-methoxy-4-methylen-hexansäureäthylester werden in 190 ml 1,2-Dichloräthan gelöst und bei Raumtemperatur tropfenweise mit 7,20 ml (93,0 mMol) Thionylbromid versetzt. Nach 45 Minuten gibt man 100 ml Wasser hinzu und lässt 10 Minuten intensiv rühren. Die organische Phase wird abgetrennt, nacheinander mit Wasser, 1N Kaliumhydrogencarbonatlösung und nochmals mit Wasser gewaschen, über Natriumsulfat getrocknet, filtriert und eingedampft. Man erhält 4-Brommethyl-2-formylamino-6-methoxy-hex-3-ensäureäthylester als rotbraunes Öl.

3,38 g (11,0 mMol) 4-Brommethyl-2-formylamino-6-methoxy-hex-3-ensäureäthylester und 12,0 ml (44 mMol) Triisopropylphophit (96%-ig) werden auf 80° erwärmt und unter einem Druck von etwa 130 mbar 18 Stunden gerührt. Das überschüssige Triisopropylphosphit wird unter vermindertem Druck abdestilliert und der Eindampfrückstand an Silicagel mit Essigsäureäthylester chromatographisch gereinigt. Man erhält 4-Diisopropylphosphonomethyl-2-formylamino-6-methoxy-hex-3-ensäureäthylester als gelbliches Öl.

Beispiel 5: 0,98 g (3,5 mMol) 2-Amino-6-methoxy-4-phosphonomethyl-hex-3-en-säureäthylester werden in 7 ml Wasser 17 Stunden zum Rückfluss erhitzt. Das Reaktionsgemisch wird eingedampft und aus einem Gemisch von Wasser und Äthanol kristallisiert. Man erhält 2-Amino-6-methoxy-4-phosphonomethyl-hex-3-ensäure vom Smp. 214°C (Zers.).

Beispiel 6: 0,52 g (1,36 mmol) 4-Diisopropylphosphonomethyl-6-fluor-2-formylamino-hex-3-ensäureäthylester werden in 3,5 ml Dichlormethan gelöst und bei Raumtemperatur tropfenweise mit 0,7 ml (5,45 mMol) Trimethylbromsilan versetzt. Man lässt 24 Stunden bei Raumtemperatur stehen, tropft 3,5 ml Äthanol hinzu, lässt nochmals 24 Stunden stehen, dampft am Rotationsverdampfer ein, löst den Rückstand in 2,4 ml Äthanol und fügt ein Gemisch von 0,6 ml Propylenoxid und 0,6 ml Äthanol hinzu. Es bildet sich eine Suspension, die weitere 2 Stunden bei Raumtemperatur und 2 Stunden unter Eiskühlung gerührt und dann abgesaugt wird. Man erhält 2-Amino-6-fluor-4-phosphonomethyl-hex-3-ensäureäthylester vom Smp. 222° (Zers.).

Das Ausgangsmaterial kann z.B. wie folgt hergestellt werden:
2,4 g (26,6 mMol) 4-Fluorbutanal, 2,44 g (30,0 mMol) Dimethylammoniumchlorid und 2,34 ml (31,1 mMol) 37%-ige Formaldehydlösung werden unter Rühren 2 Stunden auf 100° erhitzt. Man lässt abkühlen und extrahiert 3-mal mit Diäthyläther. Die organischen Phasen werden mit gesättigter Kochsalzlösung gewaschen, vereinigt, über Natriumsulfat getrocknet, filtriert und zur Trockne eingedampft. Man erhält 4-Fluor-2-methylen-butanal als gelbliches Öl, das ohne weitere Reinigung weiterumgesetzt werden kann.

1,43 g (14,0 mMol) 4-Fluor-2-methylen-butanal und 1,53 ml (14,0 mMol) Isocyanessigsäureäthylester werden in 14 ml Toluol gelöst und mit 40 mg Kupfer-I-oxid versetzt. Nach Abklingen der exothermen Reaktion lässt man noch 2 Stunden nachrühren, filtriert über Hyflo® und dampft zur Trockne ein. Der Rückstand wird in 14 ml Tetrahydrofuran aufgenommen, mit 3,1 ml Wasser versetzt und unter Rühren 2 Stunden zum Rückfluss erhitzt. Man dampft zur Trockne ein, versetzt mit Toluol und dampft erneut ein. Chromatographie an Silicagel mit Toluol/Essigsäureäthylester (1:1) als Elutionsmittel ergibt 6-Fluor-2-formylamino-3-hydroxy-4-methylen-hexansäureäthylester als dunkelgelbes Öl.

1,40 g (6,0 mMol) 6-Fluor-2-formylamino-3-hydroxy-4-methylen- hexansäureäthylester werden in 14 ml 1,2-Dichloräthan gelöst und bei Raumtemperatur tropfenweise mit 0,56 mi (7,2 mMol) Thionylbromid versetzt. Nach 45 Minuten gibt man 12 ml Wasser hinzu und lässt 15 Minuten intensiv rühren. Die organische Phase wird abgetrennt, nacheinander mit Wasser, 1N Kaliumhydrogencarbonatlösung und nochmals mit Wasser gewaschen, über Natriumsulfat getrocknet, filtriert und eingedampft. Man erhält 4-Brommethyl-6-fluor-2-formylamino-hex-3-ensäureäthylester als braungelbes Öl.

1,43 g (4,82 mMol) 4-Brommethyl-6-fluor-2-formylamino-hex-3-ensäureäthylester und 5,3 ml (19 mMol) Triisopropylphophit (96%-ig) werden auf 80° erwärmt und unter einem Druck von etwa 130 mbar 18 Stunden gerührt. Das überschüssige Triisopropylphosphit wird unter vermindertem Druck abdestilllert und der Eindampfrückstand an Silicagel mit Essigsäureäthylester chromatographisch gereinigt. Man erhält 4-Diisopropylphosphonomethyl-6-fluor-2-formylamino-hex-3-ensäureäthylester als gelbliches Öl.

Beispiel 7: 0,5 g (1,86 mMol) 2-Amino-6-fluoro-4-phosphonomethyl-hex-3-ensäureäthylester werden in 4 ml Wasser 17 Stunden zum Rückfluss erhitzt. Das Reaktionsgemisch wird eingedampft und am stark sauren Ionenaustauscher (Dowex 50Wx8; H^{⊕}-Form) mit Wasser aufgetrennt. Man erhält 2-Amino-6-fluoro-4-phosphonomethylhex-3-ensäure vom Smp. 160-162°C (Zers.).

Beispiel 8: In analoger Weise wie in Beispiel 3 beschrieben erhält man ausgehend von 8-Aminooctan-1-ol 2,10-Diamino-4-phosphonomethyl-dec-3-ensäure-dihydrochlorid, Smp. 126°.

Beispiel 9: 8,63 g (15,2 mMol) 10-(N-Benzyloxycarbonylamino)-4-diisopropylphosphonomethyl-2-formylamino-dec-3-ensäureäthylester werden in 22 ml Dichlormethan gelöst und bei Raumtemperatur tropfenweise mit 9,82 ml (75,9 mMol) Trimethylbromsilan versetzt. Man rührt 22 Stunden bei Raumtemperatur, tropft dann 22 ml abolutes Äthanol zu, rührt nochmals 22 Stunden und dampft am Rotationsverdampfer ein. Der Rückstand wird mit 20 ml Toluol übergossen und im Vakuum eingedampft. Diesen Vorgang wiederholt man noch dreimal. Der erhaltene hellgelbe Schaum wird in 150 ml absolutem Äthanol gelöst und innert 90 Minuten tropfenweise mit einer Lösung von 7,5 ml Propylenoxid in 7,5 ml Äthanol versetzt. Es bildet sich eine Kristall-Suspension, die über Nacht bei Raumtemperatur gerührt wird. Das Produkt wird abfiltriert und mit Äthanol und Äther gewaschen. Nach Trocknen im Hochvakuum bei Raumtemperatur erhält man 4,70g Rohprodukt als hellgelbe Kristalle. Zur weiteren Reinigung wird es mit 46 ml Wasser verrührt. Nach Abfiltrieren von wenig ungelöstem Material (0,33 g) wird das Klare hellgelbe Filtrat im Vakuum total eingedampft. Den Rückstand versetzt man mit 20 ml Äthanol und 20 ml Toluol und dampft erneut zur Trockne ein. Diesen Vorgang wiederholt man noch zweimal mit Toluol. Der Rückstand wird nach Trocknen im Hochvakuum in 150 ml absolutem Äthanol suspendiert und unter Rühren tropfenweise mit einer 5-normalen Lösung von Chlorwasserstoffgas in Äthanol versetzt, bis das Gemisch kongosauer reagiert. Die entstandene klare Lösung wird innert 1 Stunde tropfenweise mit einem Gemisch von 7,4 ml Propylenoxid in 7,4 ml Äthanol versetzt. Es bildet sich eine Kristallsuspension, die weitere 15 Stunden gerührt und dann abgesaugt wird. Nach Waschen mit Äthanol und Äther wird das Produkt 48 Stunden im Hochvakuum bei 50° getrocknet. Man erhält so 2,86 g 2,10-Diamin-4-phosphonomethy-dec-3-ensäureäthylester, der bei 157° zu sintern beginnt und bei 194° unter Zersetzung schmilzt.

Dieses Produkt enthält als Verunreinigungen etwa 5-10 Gew.-% der entsprechenden, in 10-Stellung N-benzylierten sowie der N-(2hydroxy)propylierten Verbindung.

Das Ausgangsmaterial wird wie folgt hergestellt:
In analoger Weise wie in Beispiel 3 beschrieben erhält man ausgehend von 8-Aminooctan-1-ol über 8-(N-Benzyloxycarbonylamino)octan-1-ol und 8-(N-Benzyloxycarbonylamino)octanal das 8-(N-Benzyloxycarbonylamino)-2-methylen-octanal.

15,30 g (52,9 mMol) 8-(N-Benzyloxycarbonylamino)-2-methylen-octanal und 7,37 ml (67,4 mMol) Isocyanessigsäureäthylester werden in 78 ml Toluol gelöst und zu einer Suspension von 0,30 g Kupfer-I-oxid in 76 ml Toluol unter Argon innert 75 Minuten zugetropft. Man lässt 90 Minuten bei 30° nachrühren, kühlt auf Raumtemperatur ab, filtriert und gibt das Klare hellrote Filtrat auf eine mit 250 g Silicagel (Korngrösse 0,04-0,063 mm) gefüllte Säule und eluiert mit Hexa/Essigsäureäthylester (2:1). Man erhält nach Eindampfen der geeigneten Fraktionen 8,15 g 5-[8-(N-Benzyloxycarbonylamino)oct-1-en-2-yl]-oxazolin-4-carbonsäureäthylester als farblosen Honig.

8,15 g (20,25 mMol) 5-[8-(N-Benzyloxycarbonylamino)oct-1-en-2-yl]-oxazolin-4-carbonsäureäthylester werden in 40 ml Tetrahydrofuran und 20 ml Wasser unter Rühren 4 Stunden zum Rückfluss erhitzt. Das Reaktionsgemisch wird im Vakuum bei 45° zur Trockne eingedampft und der honigartige Rückstand noch zweimal nach Toluolzugabe eingedampft. Das Rohprodukt wird in Dichlormethan gelöst, mit Natriumsulfat getrocknet, filtriert und eingedampft. Nach Trocknen im Hochvakuum bei Raumtemperatur erhält man 9,03 g 10-(N-Benzyloxycarbonylamino)-2-formylamino-3-hydroxy-4-methylen-decansäureäthylester als gelblichen Honig.

13,70 g (32,60 mMol) roher 10-(N-Benzyloxycarbonylamino)-2-formylamino-3-hydroxy-4-methylen-decansäureäthylester in 137 ml Tetrahydrofuran werden unter Argon mit 18,5 ml (156,4 mMol) Hexa-1,5-dien und dann bei 10° tropfenweise innert 15 Minuten mit 6,1 ml (78,2 mMol) Thionylbromid versetzt. Man lässt 1 Stunde bei 10° und 2 Stunden bei Raumtemperatur nachrühren, giesst in 200 ml eiskalte gesättigte Natriumhydrogencarbonatlösung, trennt die organische Phase ab und extrahiert 1-mal mit Dichlormethan nach. Die organischen Phasen werden mit eiskalter 0,5-normaler Natriumhydrogencarbonatlösung und hernach gesättigter Kochsalzlösung gewaschen, über Natriumsulfat getrocknet und bei 40° im Vakuum zur Trockne eingedampft. Der erhaltene rohe 10-(N-Benzyloxycarbonylamino)-4-brommethyl-2-formylamino-dec-3-ensäureäthylester (27 g, gelber Honig) wird sofort mit 64 ml (260 mMol) Triisopropylphosphit (96%-ig) versetzt und unter einem Druck von etwa 100 mbar 17 Stunden bei 80° gerührt, wobei das entstehende Isopropylbromid in einer Kühlfalle (CO₂) abgefangen wird. Anschliessend wird das überschüssige Triisopropylphosphit unter vermindertem Druck abdestilliert und der Eindampfrückstand (23 g) an einer Säule mit 650 g Silicagel (0,04-0,063 mm) mit Essigsäureäthylester/Methanol (95:5) chromatographisch gereinigt. Man erhält 8,73 g 10-(N-Benzyloxycarbonylamino)-4-diisopropylphosphonomethyl-2-formylamino-dec-3-ensäureäthylester als gelblichen Honig.

Beispiel 10: 2,25 g (6,98 mMol) 2,10-Diamino-4-phosphonomethyl-dec-3-ensäureäthylester werden unter Argon in 45 ml 2-normaler Salzsäure gelöst und 17 Stunden bei einer Bad-Temperatur von 120° gerührt. Die Klare, hellbraune Lösung wird am Rotationsverdampfer eingedampft. Der Rückstand wild in 20 ml Äthanol gelöst und nach Zugabe von 30 ml Toluol im Vakuum eingedampft. Diesen Vorgang wiederholt man noch dreimal. Der erhaltene, beige Schaum wird in 75 ml absolutem Äthanol gelöst und innert 35 Minuten tropfenweise mit einer Lösung von 15 ml Propylenoxid in 15 ml Äthanol versetzt. Die gebildete Kristall-Suspension, die einen pH-Wert von 3 aufweist, wird nach 1½-stündigem Rühren abfiltriert und gut mit Äthanol und Äther gewaschen. Nach dem Trocknen im Vakuum erhält man 1,65 g beiges Rohprodukt, das in der minimalen Menge Wasser (ca. 2 ml) gelöst und an einer Säule, gefüllt mit 67 g reversed-phase Kieselgel (Opti-Up C₁₂, Korngrösse 40 »m), mit reinem Wasser als Elutionsmittel bei schwachem Ueberdruck (0,2 bar) chromatographiert wird. Man erhält eine reine Fraktion, Rf-Wert auf Kieselgel = 0,37 mit n-Propanol/Wasser/Pyridin/Essigsäure (15:12:10:3) als Fliessmittel, und mehrere Mischfraktionen mit einem Nebenprodukt vom Rf-Wert = 0,48. Erneute Chromatographie dieser Mischfraktionen und Vereinigung der reinen Fraktionen gefolgt von Lyophilisation aus Wasser ergibt 2,10-Diamino-4-phosphonomethyl-dec-3-ensäure-hemihydrochloridhydrat als amorphes Glas, das ab 134° langsam sintert und sich bei 149° unter Aufschäumen zersetzt.

Beispiel 11: 4,3 g (9,9 mMol) 7-Acetoxy-4-diisopropylphosphonomethyl-2-formylaminohept-3-ensäureäthylester werden in 25 ml Dichlormethan gelöst und bei Raumtemperatur tropfenweise mit 5,1 ml (39,5 mMol) Trimethylbromsilan versetzt. Man lässt 24 h bei Raumtemperatur stehen, tropft 25 ml Äthanol zu und lässt nochmals 24 h stehen, dampft ein, löst den Rückstand in 25 ml Äthanol und tropft ein Gemisch aus 25 ml Propylenoxid und 25 ml Äthanol zu. Es bildet sich eine Suspension, die 1 Stunde bei Raumtemperatur und 1 Stunde im Eisbad gerührt und dann abgesaugt wird. Nach der Trocknung erhält man 2-Amino-7-hydroxy-4-phosphonomethyl-hept-3-ensäureäthylester vom Smp. 210°C (Zers.).

Das Ausgangsmaterial kann z.B. wie folgt hergestellt werden:
10 g (69,4 mMol) 5-Acetoxypentanal, 6,37 g (78,2 mMol) Dimethylammoniumchlorid und 6,1 ml (81,2 mMol) 37 %-iger Formaldehydlösung werden unter Rühren 1½ Stunden am Rückfluss gehalten (Badtemperatur ∼110°C). Man lässt abkühlen, extrahiert dreimal mit Äther, vereinigt die organischen Phasen, trocknet über MgSO₄, filtriert und dampft ein. Das 5-Acetoxy-2-methylen-pentanal wird als gelbliches Öl erhalten, welches ohne weitere Reinigung umgesetzt werden kann.

9,6 g (61,5 mMol) 5-Acetoxy-2-methylen-pentanal und 7,38 ml (67,6 mMol) Isocyanessigsäureäthylester werden in 70 ml Toluol bei Raumtemperatur vorgelegt und mit 250 mg Kupfer-I-oxid versetzt. Nach Abklingen der exothermen Reaktion lässt man noch 1 Stunde nachrübren, filtriert über Hyflo® und dampft ein. Der Rückstand wird in 50 ml Tetrahydrofuran aufgenommen, mit 10 ml Wasser versetzt und 3 Stunden am Rückfluss gehalten. Man dampft zur Trockene ein, versetzt mit Toluol und dampft nochmals ein. Chromatographie an Silicagel mit Toluol/Essigsäureäthylester (4:1) ergibt 7-Acetoxy-2-formylamino-3-hydroxy-4-methylen-heptansäureäthylester als oranges Öl.

5,9 g (20,5 mMol) 7-Acetoxy-2-formylamino-3-hydroxy-4-methylen-heptansäureäthylester werden in 60 ml Dichlormethan gelöst und bei Raumtemperatur tropfenweise mit 1,9 ml (24,6 mMol) Thionylbromid versetzt. Nach 1 Stunde gibt man 40 ml Wasser hinzu und lässt 10 Minuten intensiv rühren. Die organische Phase wird abgetrennt, nacheinander mit Wasser, 1N KHCO₃-Lösung und nochmals mit Wasser gewaschen, über MgSO₄ getrocknet, filtriert und eingedampft. Man erhält 7-Acetoxy-4-brommethyl-2-formylamino-hept-3-ensäureäthylester als braunes Öl, welches roh weiter umgesetzt wird.

6,1 g (17,4 mMol) 7-Acetoxy-4-brommethyl-2-formylamino-hept-3-ensäuräthylester und 19,1 ml (69,6 mMol) Triisopropylphosphit (90%-ig) werden auf 80°C erwärmt und unter einem Druck von etwa 130 mbar 18 Stunden gerührt. Das überschüssige Triisopropylphosphit wird abdestilliert und der Rückstand an Silicagel mit Essigsäureäthyl-nigt. Man erhält 7-Acetoxy-4-diisopropylphosphonomethyl-2-formylamino-hept-3-ensäureäthylester als gelbes Öl.

Beispiel 12: 1,1 g (3,9 mMol) 2-Amino-7-hydroxy-4-phosphonomethyl-hept-3-ensäureäthylester in 8 ml Wasser 18 Stunden bei 130°C im Bombenrohr gerührt. Die dunkle Reaktionslösung wird mit Aktivkohle behandelt und über Hyflo® filtriert. Das farblose Filtrat wird auf ∼3 ml eingeengt und mit ∼25 ml Äthanol versetzt. Die entstandene Suspension wird abgenutscht und im Hochvakuum bei 50°C getrocknet. Man erhält 2-Amino-7-hydroxy-4-phosphonomethyl-hept-3-ensäure mit dem Smp. ab 190°C (Zers.).

Beispiel 13: 8,2 g (18,9 mMol) 6-(N-Acetyl-N-methyl-amino)-4-diisopropylphosphonomethyl-2-formylamino-hex-3-ensäureäthylester werden in 40 ml Dichlormethan gelöst und bei Raumtemperatur tropfenweise mit 9,8 ml (75,6 mMol) Trimethylbromsilan versetzt. Man lässt 24 Stunden bei Raumtemperatur stehen, tropft 40 ml Äthanol hinzu, lässt nochmals 24 Stunden stehen, dampft ein, löst den Rückstand in 40 ml Äthanol und tropft ein Gemisch aus 40 ml Propylenoxid und 40 ml Äthanol hinzu. Es bildet sich eine Suspension, die noch 1 Stunde bei Raumtemperatur und 1 Stunde bei 0° gerührt und dann abgesaugt wird. Nach der Trocknung erhält man 6-(N-Acetyl-N-methyl-amino)-2-amino-4-phosphonomethyl-hex-3-ensäureäthylester vom Smp. 222-23°C (Zers.).

Das Ausgangsmaterial kann z.B. wie folgt hergestellt werden;
35,8 g (0,2 Mol) 4-Aminobutyraldehyd-diäthylacetal (90 %-ig) werden in 600 ml Dichlormethan gelöst, mit 300 ml gesättigter Natriumhydrogencarbonatlösung versetzt und auf 0°C gekühlt. Man tropft bei 0-5°C 17 ml (0,24 Mol) Acetylchlorid hinzu und lässt 6 Stunden bei 0-5°C nachrühren. Die organische Phase wird abgetrennt und die wässrige Phase noch zweimal mit Dichlormethan nachextrahiert. Die organischen Phasen werden vereinigt, über MgSO₄ getrocknet, filtriert und am Rotationsverdampfer eingedampft. Der Rückstand wird an Silicagel mit Essigsäureäthylester chromatographisch gereinigt. Man erhält 4-(N-Acetyl-amino)-butyraldehyd-diäthylacetal als gelbliches Öl.

35 g (172,2 mMol) 4-(N-Acetyl-amino)-butyraldehyd-diäthylacetal werden in 180 ml Dimethylformamid gelöst, portionsweise mit 8,3 g (206,6 Mmol) Natriumhydrid-Dispersion (60 %ig in ineralöl) versetzt und 45 Minuten bei Raumtemperatur gerührt. Dann gibt man 12,9 ml (206,6 mMol) Methyljodid in 20 ml Dimethylformamid zu und rührt 4 Stunden bei Raumtemperatur nach. Das Reaktionsgemisch wird mit Wasser/Eis versetzt und dreimal mit Essigsäureäthylester extrahiert. Die organischen Phasen werden mit mit Wasser und gesättigter Kochsalzlösung gewaschen, vereinigt, über MgSO₄ getrocknet, filtriert und eingedampft. Der Rückstand wird im Hochvakuum destilliert. Man erhält 4-(N-Acetyl-N-methyl-amino)-butyraldehyd-diäthylacetal als farbloses Öl vom Kp_{0,1} = 92-94°.

30 g (138,2 mMol) 4-(N-Acetyl-N-methyl-amino)-butyraldehyd-diäthylacetal, 12,6 g (154,4 mMol) Dimethylammoniumchlorid und 12,1 ml (161,7 mMol) Formaldehydlösung 37 % werden unter Rühren 45 Minuten am Rückfluss gehalten. Man lässt abkühlen und extrahiert dreimal mit Dichlormethan. Die organischen Phasen werden vereinigt, über MgSO₄ getrocknet, filtriert und eingedampft. Man erhält 4-(N-Acetyl-N-methylamino)-2-methylen-butyraldehyd als gelbliches Öl, welches ohne weitere Reinigung umgesetzt werden kann.

19,9 g (128,3 mMol) 4-(N-Acetyl-N-methyl-amino)-2-methylen-butanal und 15,4 ml (141,1 mMol) Isocyanessigsäureäthylester werden in 80 ml Toluol bei Raumtemperatur vorgelegt und mit 500 mg Kupfer-I-oxid versetzt. Nach AbKlingen der exothermen Reaktion lässt man noch 1 Stunde bei Raumtemperatur nachrühren, filtriert über Hyflo® und dampft ein. Der Rückstand wird in 60 ml Tetrahyrofuran aufgenommen, mit 20 ml Wasser versetzt und 4 Stunden am Rückfluss gehalten. Man dampft ein, versetzt mit Toluol und dampft nochmals ein. Chromatographie an Silicagel mit Essigsäureäthylester/Isopropanol (7:1) ergibt 6-(N-Acetyl-N-methyl-amino)-2-formylamino-3-hydroxy-4-methylen-hexansäureäthylester als gelbes Öl.

15,8 g (55,2 mMol) 6-(N-Acetyl-N-methyl-amino)-2-formylamino-3-hydroxy-4-methylen-hexansäureäthylester werden in 150 ml Dichlormethan gelöst und bei Raumtemperatur tropfenweise mit 5,1 ml (66,2 mMol) Thionylbromid versetzt. Nach 1 h gibt man 100 ml Wasser zu und lässt 10 Minuten intensiv rühren. Die organische Phase wird abgetrennt, nacheinander mit Wasser, 1N KHCO₃-Lösung und nochmals mit Wasser gewaschen, über MgSO₄ getrocknet, filtriert und eingedampft. Man erhält 6-(N-Acetyl-N-methyl-amino)-4-brommethyl-2-formylamino-hex-3-ensäureäthylester als gelb-oranges Öl, welches roh weiter umgesetzt wird.

16,0 g (45,8 mMol) 6-(N-Acetyl-N-methyl-amino)-4-brommethyl-2-formylamino-hex-3-ensäureäthylester und 50,3 ml (183,3 mMol) Triisopropylphosphit (90 %-ig) werden auf 80° erwärmt und unter einem Druck von etwa 130 mbar 18 Stunden gerührt. Das überschüssige Triisopropylphosphit wird abdestilliert und der Rückstand an Silicagel mit Essigsäureäthylester/Isopropanol (7:2) chromatographisch gereinigt. Man erhält 6-(N-Acetyl-N-methyl-amino)-4-diisopropylphosphonomethyl-2-formylamino-hex-3-ensäureäthylester als gelbes Öl.

Beispiel 14: 3,3 g (6,82 mMol) 6-Benzoyloxy-4-diisopropylphosphonomethyl-2-formylamino-hex-3-ensäureäthylester werden in 20 ml Dichlormethan gelöst und bei Raumtemperatur tropfenweise mit 3,52 ml (27,3 mMol) Trimethylbromsilan versetzt. Man lässt 22 Stunden bei Raumtemperatur stehen, tropft 20 ml Äthanol zu, lässt nochmals 22 Stunden stehen, dampft ein, löst den Rückstand in 20 ml Äthanol und tropft ein Gemisch aus 20 ml Propylenoxid und 20 ml Äthanol hinzu. Es bildet sich eine Suspension die 1 Stunde bei Raumtemperatur und 1 Stunde bei 0°C gerührt und dann abgesaugt wird. Nach der Trocknung erhält man 2-Amino-6-benzoyloxy-2-amino-4-phosphonomethyl-hex-3-ensäureäthylester mit dem Smp. 236-7°C (Zers.).

Das Ausgangsmaterial kann z.B. wie folgt hergestellt werden:
10 g (52 mMol) 4-Benzoyloxy-butanal, 4,78 g (58,6 mMol) Dimethylammoniumchlorid und 4,6 ml (60,8 mMol) Formaldehydlösung (37 %-ig) werden unter Rühren 1 Stunde am Rückfluss gehalten (Badtemperatur ∼110°C). Man lässt abkühlen und extrahiert dreimal mit Äther, vereinigt die organischen Phasen, wäscht mit gesättigter Kochsalzlösung, trocknet über MgSO₄, filtriert und dampft ein. Man erhält 4-Benzoyloxy-2-methylenbutanal als gelbliches Öl, welches ohne weitere Reinigung umgesetzt werden kann.

10 g (49 mMol) 4-Benzoyloxy-2-methylen-butanal und 5,3 ml (49 mMol) Isocyanessigsäureäthylester werden in 70 ml Toluol bei Raumtemperatur vorgelegt und mit 200 mg Kupfer-I-oxid versetzt. Nach Abklingen der exothermen Reaktion lässt man noch 1 Stunde nachrühren, filtriert über Hyflo® und dampft ein. Der Rückstand wird in 50 ml Tetrahydrofuran aufgenommen, mit 10 ml Wasser versetzt und 3 Stunden am Rückfluss gehalten. Man dampft ein, versetzt nochmals mit Toluol und dampft erneut ein. Chromatographie an Silicagel mit Toluol/Essigsäureäthylester (3:2) ergibt 6-Benzoyloxy-2-formylamin-3-hydroxy-4-methylen-hexansäureäth ylester als braunes Öl.

8 g (23,9 mMol) 6-Benzoyl-2-formylamin-3-hydroxy-4-methylen-hexansäureäthylester werden in 80 ml Dichlormethan gelöst und bei Raumtemperatur tropfenweise mit 2,22 ml (28,6 mMol) Thionylbromid versetzt. Nach 2 Stunden gibt man 60 ml Wasser zu und lässt 10 Minuten intensiv rühren. Die organische Phase wird abgetrennt, nacheinander mit Wasser, 1N KHCO₃-Lösung und nochmals mit Wasser gewaschen, über MgSO₄ getrocknet, filtriert und eingedampft. Man erhält 6-Benzoyloxy-4-brommethyl-2-formylamino-hex-3-ensäureäthylest er als braunes Öl, welches roh weiter umgesetzt wird.

8,4 g (21 mMol) 6-Benzoyloxy-4-brommethyl-2-formylamino-hex-3-ensäureäthylester und 23 ml (84 mMol) Triisopropylphosphit (90%ig) werden auf 80°C erwärmt und unter einem Druck von ∼130 mbar 18 Stunden gerührt. Das überschüssige Triisopropylphosphit wird abdestilliert und der Rückstand an Silicagel mit Essigsäureäthylester chromatographisch gereinigt. Man erhält 6-Benzoyloxy-4-diisopropylphosphonomethyl-2-formylamino-hex-3-ensäureäthylester als braunes Öl.

Beispiel 15: 4,0 g (8,51 mMol)
6-Benzyloxy-4-Diisopropylphosphonomethyl-2-formylamino-hex-3-ensäureäthylester werden in 24 ml Dichlormethan gelöst und bei Raumtemperatur tropfenweise mit 4,4 ml (34 mMol) Trimethylbromsilan versetzt. Man lässt 22 Stunden bei Raumtemperatur stehen, tropft 24 ml Äthanol zu, lässt nochmals 24 Stunden stehen, dampft am Rotationsverdampfer ein, löst den Rückstand in 24 ml Äthanol und tropft ein Gemisch aus 24 ml Propylenoxid und 24 ml Äthanol hinzu. Es bildet sich eine Suspension, die 1 Stunde bei Raumtemperatur und 1 Stunde bei 0° nachgerührt und dann abgesaugt wird. Nach der Trocknung erhält man 2,2 g eines weissen, kristallinen Produktes, welches ein Gemisch aus 2-Amino-6-benzyloxy-4-phosphonomethyl-hex-3-ensäureäthylester und 2-Amino-6-benzyloxy-4-phosphonomethyl-hex-3-ensäure darstellt. Um ein einheitliches Produkt zu erhalten wird diese Gemisch mit 20 ml n-Natronlauge in 30 ml Äthanol über Nacht bei Raumtemperatur verseift, mit n-Salzsäure sauer gestellt und mit Propylenoxid neutralisiert. Da das Produkt schlecht kristallisiert, dampft man am Rotationsverdampfer ein, filtriert den Rückstand in Wasser über 20 g Kieselgel und dampft die das gewünschte Produkt enthaltenden Fraktionen am Rotationsverdampfer ein. Der Rückstand wird mit 10 ml Tertiärbutanol/Wasser (1:1) gelöst und gefriergetrocknet. Man erhält 2-Amino-6-benzyloxy-4-phosphonomethyl-hex-3-ensäure als Lyophilisat.

Das Ausgangsmaterial kann z.B. wie folgt hergestellt werden:
6 g (0,2 Mol) einer Natriumhydrid-Dispersion (80 %-ig in Weissoel) werden in 120 ml absolutem Dimethylformamid bei Raumtemperatur vorgelegt und tropfenweise mit 22,2 ml (0,25 Mol) 1,4-Butandiol versetzt. Nach beendigter Zugabe wird noch 30 Minuten bei Raumtemperatur nachgerührt. Nun werden 23,1 ml (0,2 Mol) Benzylchlorid langsam zugetropft, wobei eine leichte Exothermie erkennbar wird. Das Reaktionsgemisch wird über Nach bei Raumtemperatur gerührt, mit Wasser/Eis versetzt und zweimal mit Äther extrahiert. Die organischen Phasen werden mit Wasser und mit gesättigter Kochsalzlösung gewaschen, vereinigt, über MgSO₄ getrocknet, filtriert und eingedampft. Der Rückstand wird im Wasserstrahlvakuum über eine 10 cm Vigreux-Kolonne fraktioniert destilliert. Man erhält 4-Benzyloxybutanol vom Kp₂₂ = 161-2°.

36,6 g (170 mMol) Pyridiniumchlorochromat werden in 120 ml Dichlormethan bei Raumtemperatur unter N₂ vorgelegt und mit einer Lösung von 20,4 g (113 mMol) 4-Benzyloxybutanol in 20 ml Dichlormethan versetzt. Das Reaktionsgemisch wird rasch dunkel und die Reaktion ist leicht exotherm. Das Reaktionsgemisch wird 3½ Stunden bei Raumtemperatur gerührt. Die überstehende Dichlormethanphase wird abdekantiert und am Rotationsverdampfer eingedampft. Der Rückstand wird über 100 g Silicagel filtriert. Die Produktefraktionen werden am Rotationsverdampfer eingedampft und im Hochvakuum über eine 10 cm Vigreux-Kolonne destilliert. Man erhält 4-Benzyloxybutanal vom Kp_{0,1} = 72-3°.

8,0 g (44,9 mMol) 4-Benzyloxybutanal, 4,12 g (50,6 mMol) Dimethylammoniumchlorid und 3,95 ml (52,6 mMol) 37%-ige Formaldehydlösung werden unter Rühren 1 Stunden auf 110° Badtemperatur gehalten. Man lässt abkühlen und extrahiert dreimal mit Äther. Die organischen Phasen werden mit gesättigter Kochsalzlösung gewaschen, vereinigt, über MgSO₄ getrocknet, filtriert und eingedampft. Man erhält 4-Benzyloxy-2-methylenbutanal als gelbliches Öl, das ohne weitere Reinigung weiterumgesetzt werden kann.

8,0 g (42 mMol) 4-Benzyloxy-2-methylen-butanal und 4,57 ml (42 mMol) Isocyanessigsäureäthylester werden in 60 ml Toluol vorgelegt und mit 200 mg Kupfer-I-oxid versetzt. Nach Abklingen der exothermen Reaktion lässt man 2 Stunden nachrühren, filtriert über Hyflo® und dampft ein. Der Rückstand wird in 50 ml Tetrahydrofuran aufgenommen, mit 10 ml Wasser versetzt und 3 Stunden am Rückfluss gehalten. Man dampft zur Trockene ein, versetzt mit Toluol und dampft erneut ein. Chromatographie an Silicagel mit Toluol/Essigsäureäthylester (3:2) als Elutionsmittel ergibt 6-Benzyloxy-2-formylamino-3-hydroxy-4-methylen-hexansäureäthylester als rotbraunes Öl.

7,0 g (21,8 mMol) 6-Benzyloxy-2-formylamino-3-hydroxy-4-methylen-hexansäureäthylester werden in 70 ml Dichlormethan gelöst und bei Raumtemperatur tropfenweise mit 2,0 ml (26,1 mMol) Thionylbromid versetzt. Nach 2 Stunden gibt man 40 ml Wasser hinzu und lässt 10 Minuten intensiv rühren. Die organische Phase wird abgetrennt, nacheinander mit Wasser, n-KHCO₃-Lösung und nochmals mit Wasser gewaschen, über MgSO₄ getrocknet, filtriert und eingedampft. Man erhält 6-Benzyloxy-4-brommethyl-2-formylamino-hex-3-ensäureäthylester als rotbraunes Öl, welches ohne Reinigung weiter umgesetzt wird.

7,9 g (20,5 mMol) 6-Benzyloxy-4-brommethyl-2-formylamino-hex-3-ensäureäthylester und 22,5 ml (82,2 mMol) Triisopropylphosphit (90%-ig) werden auf 80° erwärmt und unter einem Druck von etwa 130 mbar 18 Stunden gerührt. Das überschüssige Triisopropylphosphit wird am Rotationsverdampfer abdestilliert und der Rückstand an Silicagel mit Essigsäureäthylester chromatographisch gereinigt. Man erhält 6-Benzoyl-4-diisopropylphosphonomethyl-2-formylamino-hex-3-ensäureäthylester als gelbliches Öl.

Beispiel 16: 3,15 g (6,84 mMol) 4-(1-Acetylpiperidin-4-yl)-5-diisopropylphosphono-2-formylamino-pent-3-ensäureäthylester werden in 17 ml Dichlormethan gelöst und bei Raumtemperatur tropfenweise mit 3,54 ml (27,3 mMol) Trimethylbromsilan versetzt. Man lässt 16 Stunden bei Raumtemperatur stehen, tropft 17 ml Äthanol hinzu, lässt weitere 18 Stunden stehen, dampft am Raotationsverdampfer ein, löst den Rückstand in 12 ml Äthanol und fügt ein Gemisch von 3 ml Propylenoxid und 3 ml Äthanol hinzu. Es bildet sich eine Suspension, die weitere 2 Stunden bei Raumtemperatur und 2 Stunden unter Eiskühlung gerührt und dann abgesaugt wird. Man erhält 2-Amino-4-(1-acetylpiperidin-4-yl)-5-phosphono-pent-3-ensäureäthylester vom Smp. 225° (Zers.).

Das Ausgangsmaterial kann z.B. wie folgt hergestellt werden:
3,95 g (23,3 mMol) 2-(1-Acetylpiperidin-4-yl)-äthanol, 2,12 g (26,3 mMmol) Dimethylammoniumchlorid und 3 ml (40 mMol) 37%-ige Formaldehydlösung werden unter Rühren 2 Stunden auf 110°C erhitzt. Man lässt abkühlen und extrahiert mehrmals mit Diäthyläther. Die organischen Phasen werden vereinigt, mit gesättigter Kochsalzlösung gewaschen, über Natriumsulfat getrocknet, filtriert und zur Trockne eingedampft. Man erhält 2-(1-Acetylpiperidin-4-yl)-propenal als gelbliches Öl, das ohne weitere Reinigung weiterumgesetzt werden kann.

2,9 g (16,0 mMol) 2-(1-Acetylpiperidin-4-yl)-propenal und 1,75 ml (16 mMol) Isocyanessigsäureäthylester werden in 13 ml Toluol gelöst und mit 46 mg Kupfer-(I)-oxid versetzt. Nach Abklingen der exothermen Reaktion lässt man noch 2 STunden nachrühren, filtriert über Hyflo® und dampft zur Trockne ein. Der Rückstand wird in 13 ml Tetrahydrofuran aufgenommen, mit 6 ml Wasser versetzt und unter Rühren 2 Stunden zum Rückfluss erhitzt. Man dampft zur Trockne ein, versetzt mit Toluol und dampft erneut ein. Chromatographie an Silicagel mit Essigsäureäthylester/Methanol (9:1) als Elutionsmittel ergibt 4-(1-Acetylpiperidin-4-yl)-2-fomylamino-3-hydroxy-pent-4-ensäureäthylester als gelbbraunes Öl.

3,3 g (10,5 mMol) 4-(1-Acetylpiperidin-4-yl)-2-fomylamino-3-hydroxy-pent-4-ensäureäthylester werden in 25 ml 1,2-Dichloräthan gelöst und bei Raumtemperatur tropfenweise mit 0,98 ml (12,6 mMol) Thionylbromid versetzt. Nach 1½ Stunden gibt man 20 ml Wasser hinzu und lässt 15 Minuten intensiv rühren. Die organische Phase wird abgetrennt, nacheinander mit Wasser, n-Kaliumhydrogencarbonatlösung und nochmals mit Wassr gewaschen, über Natriumsulfat getrocknet, filtriert und eingedampft. Man erhält 4-(1-Acetylpiperidin-4-yl)-5-brom-2-formylamino-pent-3-ensäureäthylester als robraunes Öl.

2,68 g (7,14 mMol) 4-(1-Acetylpiperidin-4-yl)-5-brom-2-formylamino-pent-3-en-säureäthylester und 7,5 ml (28,5 mMol) Triisopropylphosphit (90%-ig) werden auf 80° erwärmt und unter einem Druck von etwa 130 mbar 18 Stunden gerührt. Das überschüssige Triisopropylphosphit wird unter vermindertem Druck abdestilliert und der Eindampfrückstand an Silicagel mit Essigsäureäthylester/Methanol (9:1) chromatographisch gereinigt. Man erhält 4-(1-Acetylpiperidin-4-yl)-5-diisopropylphosphon-2-formylamino-pent-3-ensäureäthylesterals gelbliches Öl.

Beispiel 17: 1 g (2,17 mMol) 4-(1-Acetylpiperidin-4-yl)-5-diisopropylphosphon-2-formylamino-pent-3-ensäureäthylester werden in 20 ml 6n-Salzsäure 8 Stunden zum Rückfluss erhitzt. Nach dem Eindampfen wird der Rückstand in 25 ml Äthanol gelöst. Nun fügt man 3 ml Propylenoxid hinzu, lässt 2 Stunden bei Raumtemperatur und 1 Stunde unter Eiskühlung rühren und saugt dann die gebildete Suspension ab. Man erhält 2-Amino-4-(piperidin-4-yl)-5-phosphono-pent-3-ensäure vom Smp. 212° (Zers.).

Beispiel 18: 0,68 g (1,49 mMol) 5-Benzyloxy-4-diisopropylphosphonomethyl-2-formylamino-pent-3-ensäureäthylester werden in 10 ml Dichlormethan gelöst und bei Raumtemperatur tropfenweise mit 0,8 ml (6 mMol) Trimethylbromsilan versetzt. Man lässt 6 Stunden bei Raumtemperatur stehen, tropft 10 ml Äthanol hinzu, lässt nochmals 18 Stunden stehen, dampft ein, löst den Rückstand in 5 ml Äthanol und tropft ein Gemisch aus 5 ml Propylenoxid und 5 ml Äthanol hinzu. Es bildet sich eine Suspension, die 2 Stunden bei Raumtemperatur gerührt und dann abgesaugt wird. Nach der Trocknung erhält man 2-Amino-5-benzyloxy-4-phosphonomethyl-pent-3-ensäureäthyler vom Smp. 218-220° (Zers.).

Das Ausgangsmaterial kann z.B. wie folgt hergestellt werden:
3,0 g (62,4 mMol) 50%-ige Natriumhydrid-Dispersion in Mineralöl werden in 50 ml Tetrahydrofuran und 40 ml Dimethylformamid vorgelegt, bei 0° langsam mit einer Lösung von 10,0 g (62,4 mMol) 3-Hydroxy-2-methylen-propionaldehyd-diäthylacetal in 10 ml Tetrahydrofuran versetzt und 2 Stunden bei bei 0° gerührt. Man verdünnt mit 15 ml Tetrahydrofuran und 10 ml Dimethylformamid und lässt weitere 2 Stunden bei Raumtemperatur rühren. Nun gibt man 7,2 ml (62,4 mMol) Benzylchlorid in 10 ml Dimethylformamid bei 0° zu und rührt 18 Stunden bei Raumtemperatur. Das Reaktionsgemisch wird mit Wasser versetzt, dreimal mit Essigsäureäthylester extrahiert und die organischen Phasen mit gesättigter Kochsalzlösung gewaschen. Die organischen Phasen werden vereinigt, über Na₂SO₄ getrocknet, filtriert und eingedampft. Der Rückstand wird an Silicagel mit Methylenchlorid chromatographiert und ergibt 3-Benzyloxy-2-methylenpropionaldehyd-diäthylacetal als leicht gelbliche Flüssigkeit.

4,8 g (19,1 mMol) 3-Benzyloxy-2-methylen-propionaldehyd-diäthylacetal und 0,36 g (1,9 mMol) p-Toluolsulfonsäure-monohydrat werden 3 Stunden in 60 ml Aceton gerührt. Man verdünnt mit 400 ml Methylenchlorid, extrahiert mit n-KHCO₃-Lösung und gesättigter Kochsalzlösung, trocknet über Na₂SO₄, filtriert und dampft ein. Das zurückbleibende 3-Benzyloxy-2-methylen-propanal (gelbe Flüssigkeit) wird ohne weitere Reinigung zusammen mit 2,1 ml (19 mMol) Isocyanessigsäureäthylester in 25 ml Toluol bei Raumtemperatur vorgelegt und mit 50 mg Kupfer-I-oxid versetzt. Nach Abklingen der exothermen Reaktion lässt man noch 1 Stunde bei Raumtemperatur nachrühren, filtriert über Hyflo® und dampft ein. Der Rückstand wird in 25 ml Tetrahydrofuran aufgenommen, mit 5 ml Wasser versetzt und 4 Stunden am Rückfluss gehalten. Man dampft ein, versetzt mit Toluol und dampft erneut ein. Chromatographie an Silicagel mit Toluol/Essigsäureäthylester (1:1) und anschliessende Kristallisation aus Diäthyläther ergibt 5-Benzyloxy-2-formylamino-3-hydroxy-4-methylenpentansäureäthylester vom Smp. 112-114°C.

1,0 g (3,25 mMol) 5-Benzyloxy-2-formylamino-3-hydroxy-4-methylen-pentansäureäthylester werden in 30 ml 1,2-Dichloräthan suspendiert und bei Raumtemperatur tropfenweise mit 0,38 ml (4,9 mMol) Thionylbromid versetzt. Nach 45 Minuten gibt man zur gelben Lösung 20 ml Wasser zu und lässt 15 Minuten intensiv rühren. Die organische Phase wird abgetrennt, mit gesättigter Kochsalzlösung gewaschen, über Na₂SO₄ getrocknet, filtriert und eingedampft. Man erhält 5-Benzyloxy-4-brommethyl-2-formylamino-pent-3-ensäureäthylester als gelbliches Öl, welches roh weiterumgesetzt wird.

1,14 g (3,1 mMol) 5-Benzyloxy-4-brommethyl-2-formylamino-pent-3-ensäureäthyester und 10 ml (38,5 mMol) Triisopropylphosphit (95 %) werden auf 80°C erwärmt und unter einem Druck von etwa 130 mbar 3 ½ Stunden gerührt. Das überschüssige Triisopropylphosphit wird abdestilliert und der Rückstand an Silicagel mit Essigsäureäthylester chromatographisch gereinigt Man erhält 5-Benzyloxy-4-diisopropylphosphonomethyl-2-formylamino-pent-3-ensäureäthylester als gelbliches Öl.

Beispiel 19: 1,10 g (2,8 mMol) 5-Äthoxy-4-diisopropylphosphonomethyl-2-formylamino-pent-3-ensäureäthylester weden in 20 ml Dichlormethan gelöst und bei Raumtemperatur tropfenweise mit 1,6 ml (12,3 mMol) Trimethylbromsilan versetzt. Man lässt 7 Stunden bei Raumtemperatur stehen, tropft 20 ml Äthanol zu und lässt nochmals 15 Stunden stehen, dampft ein, löst den Rückstand in 10 ml Äthanol und tropft ein Gemisch aus 10 ml Propylenoxid und 10 ml Äthanol zu. Es bildet sich eine Suspension, die 2 Stunden bei Raumtemperatur gerührt und dann abgesaugt wird. Nach der Trocknung erhält man 5-Äthoxy-2-amino-4-phosphonomthyl-pent-3-ensäureäthylester vom Smp. 217-218°C (Zers.).

Das Ausgangsmaterial kann z.B. wie folgt hergestellt werden:
50 g (283 mMol) 3-Äthoxy-propionaldehyd-diäthylacetal, 27,3 g (335 mMol) Dimethylammoniumchlorid und 30 ml (392 mMol) 36%-ige Formaldehydlösung werden unter Rühren 2 Stunden auf 110°C erhitzt. Man lässt abkühlen und extrahiert dreimal mit Diäthyläther. Die organischen Phasen werden mit gesättigter Kochsalzlösung gewaschen, vereinigt, über Natriumsulfat getrocknet, filtriert und zur Trockne eingedampft. Man erhält 2-Äthoxy-propanal als gelbe Flüssigkeit, die ohne weitere Reinigung weiterumgesetzt werden kann.

4,2 g (36,1 mMol) 2-Äthoxy-propanal und 4,4 ml (40 mMol) Isocyanessigsäureäthylester werden in 50 ml Toluol gelöst und mit 200 mg Kupfer-I-oxid versetzt. Nach Abklingen der exothermen Reaktion lässt man noch 1 Stunde nachrühren, filtriert über Hyflo® und dampft zur Trockne ein. Der Rückstand wird in 50 ml Tetrahydrofuran aufgenommen, mit 12 ml Wasser versetzt und unter Rühren 1 Stunde zum Rückfluss erhitzt. Man dampft zur Trockne ein, versetzt mit Toluol und dampft erneut ein. Chromatographie an Silicagel mit Toluol/Isopropanol (9:1) als Elutionsmittel ergibt 5-Äthoxy-2-formylamino-3-hydroxy-4-methylen-pentansäureäthylester als gelbes Oel.

3,70 g (15,1 mMol) 5-Äthoxy-2-formylamino-3-hydroxy-4-methylen-pentansäureäthylester werden in 100 ml 1,2-Dichloräthan gelöst und bei Raumtemperatur tropfenweise mit 1,8 ml (22,8 mMol) Thionylbromid versetzt. Nach 1 Stunde gibt man 100 ml Wasser hinzu und lässt 15 Minuten intensiv rühren. Die organische Phase wird abgetrennt, nacheinander mit 1N Kaliumhydrogencarbonatlösung und Sole gewaschen, über Natriumsulfat getrocknet, filtriert und eingedampft. Man erhält 5-Äthoxy-4-brommethyl-2-formylamino-pent-3-ensäureäthylester als gelbbraunes Oel, welches roh weiter umgesetzt wird.

3,12 g (10,1 mMol) 5-Äthoxy-4-brommethyl-2-formylamino-pent-3-ensäureäthylester und 30 ml (118 mMol) Triisopropylphosphit (90 %) werden auf 80°C erwärmt und unter einem Druck von etwa 130 mbar 7 Stunden gerührt. Das überschüssige Triisopropylphosphit wird abdestilliert und der Rückstand an Silicagel mit Methylenoxid/Methanol (97:3 bis 95:5) chromatographisch gereinigt. Man erhält 5-Äthoxy-4-diisopropylphosphonomethyl-2-förmylamino-pent-3-ensäureäthylester als gelbes Oel.

Beispiel 20: Tabletten, enthaltend je 50 mg 2-Amino-6-hydroxy-4-phosphonomethyl-hex-3-ensäure oder ein Salz, z.B. das Natriumsalz, davon können wie folgt hergestellt werden:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert.

Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 21: Lacktabletten, enthaltend je 100 mg 2-Amino-6-hydroxy-4-phosphonomethyl-hex-3-ensäure oder ein Salz, z.B. das Natriumsalz, davon können wie folgt hergestellt werden:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Malsstärke werden gemischt und mit einem Kleister, hergestellt aus 15 g Maisstärke und Wasser (unter Erwärmen), befeuchtet und granuliert. Das Granulat wird getrocknet, der Rest der Malsstärke, der Talk und das Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lakkiert; Endgewicht der Lacktablette: 283 mg.

Beispiel 22: Gelatinesteckkapseln, enthaltend 100 mg Wirkstoff, z.B. 2-Amino-6-hydroxy-4-phosphonomethyl-hex-3-ensäure oder ein Salz, z.B. das Natriumsalz, davon, können z.B. folgendermassen hergestellt werden:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 23: Eine 0,2%-ige Injektions- oder Infusionslösung von 2-Amino-6-hydroxy-4-phosphonomethyl-hex-3-ensäure oder eines Salzes, z.B. des Natriumsalzes, davon, kann beispielsweise folgendermassen hergestellt werden:

| Zusammensetzung (für 1000 Ampullen) | |
|---|---|
| Wirkstoff | 5,0 g |
| Natriumchlorid | 22,5 g |
| Phosphatpuffer pH=7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 24: In analoger Weise wie in den Beispielen 1 bis 19 beschrieben kann man ferner herstellen:
2-Amino-8-hydroxy-4-phosphonomethyl-oct-3-ensäureäthylester, Smp. 230-233° (Zers.);
2-Amino-6-hydroxy-5-hydroxymethyl-4-phosphonomethyl-hex-3-ensäureäthylester, Smp.
2-Amino-6-hydroxy-5-hydroxymethyl-4-phosphonomethyl-hex-3-ensäureäthylester, Smp. 177-180°, und
2-Amino-10-hydroxy-4-phosphonomethyl-dec-3-ensäureäthylester, Smp. 243-244° (Zers.).

Beispiel 25: In analoger Weise wie in den vorstehenden Beispielen 20 bis 23 beschrieben kann man auch pharmazeutische Präparate, enthaltend eine andere Verbindung der Formel I gemäss einem der Herstellungsbeispiele 1 bis 19 und 24 herstellen.

## Patentansprüche (Patentansprüche für folgende(n) Vertragsstaat(en): AT, BE, CH, DE, DK, FR, GB, IT, LU, LI, NL, SE)

1. Substituierte 2-Aminoalk-3-ensäurederivate der Formel I 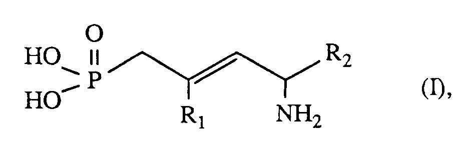 worin R₁ einen durch gegebenenfalls acyliertes oder aliphatisch oder araliphatisch veräthertes Hydroxy, durch Halogen, durch gegebenenfalls acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls aliphatisch N-substituierten oder N-acylierten azacycloaliphatischen Kohlenwasserstoffrest bedeutet und R₂ freies oder verestertes Carboxy darstellt, und ihre Salze.

2. Verbindungen gemäss Anspruch 1 der Formel I, worin R₁ einen durch gegebenenfalls aliphatisch oder araliphatisch veräthertes Hydroxy, gegebenenfalls aliphatisch substituiertes Amino oder Halogen substituierten aliphatischen Kohlenwasserstoffrest bedeutet und R₂ freies oder verestertes Carboxy darstellt, und ihre Salze.

3. Verbindungen gemäss Anspruch 1 der Formel I, worin R₁ Mono- oder Dihydroxyniederalkyl, Niederalkanoyloxyniederalkyl, Benzoyloxyniederalkyl, Niederalkoxyniederalkyl, Phenylniederalkoxyniederalkyl, Halogenniederalkyl, Aminoniederalkyl, Niederalkylaminoniederalkyl, Niederalkanoylaminoniederalkyl, Diniederalkylaminoniederalkyl, N-Niederalkyl-N-niederaikanoyl-aminoniederalkyl, 5- bis 7-gliedriges Azacycloalkylniederalkyl, dessen Azacyloalkylteil über das N- oder ein C-Atom gebunden und im zweitgenannten Fall gegebenenfalls N-niederalkyliert, N-niederalkanoyliert oder durch eine im Phenylteil gegebenenfalls substituierte Benzoylgruppe N-substituiert sein kann, 5- bis 7-gliedriges Diazacycloalkylniederalkyl, dessen Diazacycloalkylteil über ein N-Atom gebunden und gegebenenfalls N'-niederalkyliert, N'-niederalkanoyliert oder durch eine im Phenylteil gegebenenfalls substituierte Benzoylgruppe N'-substituiert ist, 5- bis 7-gliedriges, über das N-Atom gebundenes Azoxacycloalkylniederalkyl, 5- bis 7-gliedriges, über ein C-Atom gebundenes Oxacycloalkylniederalkyl, 5- bis 7-gliedriges, über ein C-Atom gebundenes, gegebenenfalls N-niederalkyliertes, N-niederalkanoyliertes oder durch eine im Phenylteil gegebenenfalls substituierte Benzoylgruppe N-substituiertes Azacycloalkyl oder 5- bis 7-gliedriges, über ein C-Atom gebundenes Oxacycloalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihre Salze.

4. Verbindungen gemäss Anspruch 2 der Formel I, worin R₁ Hydroxyniederalkyl, Niederalkoxyniederalkyl, Phenylniederalkoxyniederalkyl, Aminoniederalkyl, Niederalkylaminoniederalkyl, Diniederalkylaminoniederalkyl, N,N-Niederalkylenamino- bzw. N,N-(Aza- oder Oxaniederalkylen)aminoniederalkyl oder Halogenniederalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihre Salze.

5. Verbindungen gemäss Anspruch 1 der Formel I, worin R₁ Hydroxy-C₁-C₇-alkyl, Dihydroxy-C₂-C₇-alkyl, C₂-C₇-Alkanoyloxy-C₁-C₇-alkyl, im Phenylteil gegebenenfalls durch C₁-C₄-Alkyl, C₁-C₄-Alkoxy, Halogen der Atomnummer bis und mit 35, Cyano und/oder Trifluormethyl mono- oder disubstituiertes Benzoyloxy-C₁-C₇-alkyl, C₁-C₄-Alkoxy-C₁-C₇-alkyl, eine im Phenylteil gegebenenfalls durch C₁-C₄-Alkyl, C₁-C₄-Alkoxy, Halogen der Atomnummer bis und mit 35, Cyano und/oder Trifluormethyl mono- oder disubstituierte Phenyl-C₁-C₄-alkoxy-C₁-C₇-alkylgruppe, Halogen-C₁-C₇-alkyl, wobei Halogen Chlor oder Fluor bedeutet, Amino-C₁-C₇-alkyl, C₁-C₄-Alkylamino-C₁-C₇-alkyl, C₂-C₇-Alkanoylamino-C₁-C₇-alkyl, N-C₂-C₇-Alkanoyl-N-C₁-C₄-alkyl-amino-C₁-C₇-alkyl, Di-C₁-C₇-Alkylamino-C₁-C₇-alkyl, 5- bis 7-gliedriges Azacycloalk-1-yl-C₁-C₇-alkyl, Azacycloalk-3-yl-C₁-C₇-alkyl bzw. Azacycloalk-4-yl-C₁-C₇-alkyl, 1-C₂-C₇-Alkanoylazacycloalk-3-yl-C₁-C₇-alkyl bzw. 4-yl-C₁-C₇-alkyl, N-C₁-C₄-Alkylazacycloalkyl-3-yl-C₁-C₇-alkyl bzw. 4-yl-C₁-C₇-alkyl, N-C₁-C₄-Benzoylazacycloalkyl-3-yl-C₁-C₇-alkyl bzw. 4-yl-C₁-C₇-alkyl, 5- bis 7-gliedriges Diazacycloalk-1-yl-C₁-C₇-alkyl, N'-C₁-C₄-Alkyldiazacycloalk-1-yl-C₁-C₇-alkyl, N'-C₂-C₇-Alkanoylazacycloalk-1-yl-C₁-C₇-alkyl, 5- bis 7-gliedriges Azoxacycloalk1-yl-C₁-C₇-alkyl, 5- bis 7-gliedriges Oxacycloalk-3-yl-C₁-C₇-alkyl oder -4-yl-C₁-C₇-alkyl, 5- bis 7-gliedriges Azacycloalk-3-yl bzw. 4-yl oder 1-C₂-C₇-Alkanoylazacycloalk-3-yl bzw. 4-yl, N-C₁-C₄-Alkylazacycloalk-3-yl bzw. -4-yl oder im Phenylteil gegebenenfalls substituiertes N-Benzoylazacycloalk-3-yl oder -4-yl oder 5- bis 7-gliedriges Oxacycloalk-3-yl bzw. 4-yl darstellt und R₂ Carboxy, C₁-C₄-Alkoxycarbonyl, 5- bis 7-gliedriges Cycloalkoxycarbonyl oder gegebenenfalls durch C₁-C₄-Alkyl, C₁-C₄-Alkoxy, Halogen der Atomnummer bis und mit 35, Cyano und/oder Trifluormethyl mono- oder disubstituiertes Phenyl-C₁-C₄-alkoxycarbonyl, ist, und ihre Salze.

6. Verbindungen gemäss Anspruch 2 der Formel I, worin R₁ Hydroxy-C₁-C₇-alkyl, C₁-C₄-Alkoxy-C₁-C₇-alkyl, eine im Phenylteil gegebenenfalls durch C₁-C₄-Alkyl, C₁-C₄-Alkoxy, Halogen der Atomnummer bis und mit 35, Cyano und/oder Trifluormethyl mono-, di- oder trisubstituierte Phenyl-C₁-C₄-alkoxy-C₁-C₇-alkylgruppe, Amino-C₁-C₇-alkyl, C₁-C₄-Alkylamino-C₁-C₇-alkyl, C₂-C₇-Alkanoylamino-C₁-C₇-alkyl, Di-C₁-C₇-Alkylamino-C₁-C₇-alkyl, 5- bis 7-gliedriges N,N-(Aza- oder Oxaalkylen)amino-C₁-C₇-alkyl oder Halogen-C₁-C₇-alkyl darstellt, wobei Halogen Chlor oder Fluor bedeutet und R₂ Carboxy, C₁-C₄-Alkoxycarbonyl, 5- bis 7-gliedriges Cycloalkoxycarbonyl, gegebenenfalls durch C₁-C₄-Alkyl, C₁-C₄-Alkoxy, Halogen der Atomnummer bis und mit 35, Cyano und/oder Trifluormethyl mono- oder disubstituiertes PhenylC₁-C₄-alkoxycarbonyl, ist, und ihre Salze.

7. Verbindungen gemäss Anspruch 1 der Formel I, worin R₁ Hydroxy-C₁-C₇-alkyl, C₁-C₄-Alkoxy-C₁-C₇-alkyl, Benzoyloxy-C₁-C₄-alkyl, Amino-C₄-C₇-alkyl, N-C₂-C₇-Alkanoyl-N-C₁-C₄-alkylamino-C₂-C₇-alkyl, 5- bis 7-gliedriges Azacycloalk-3-yl bzw. 4-yl oder 1-C₂-C₇-Alkanoyl-azacycloalk-3-yl bzw. 4-yl, Halogen-C₁-C₄-alkyl, wobei Halogen Chlor oder Fluor bedeutet, Amino-C₄-C₇-alkyl, N-C₁-C₄-Alkanoyl-N-C₁-C₄-alkyl-amino-C₁-C₇-alkyl, Piperidin-4-yl oder 1-C₂-C₇-Alkanoylpiperidin-4-yl darstellt und R₂ Carboxy oder C₁-C₄-Alkoxycarbonyl bedeutet, und ihre Salze.

8. Verbindungen gemäss Anspruch 2 der Formel I, worin R₁ C₁-C₄-Alkoxy-C₂-C₄-alkyl, Hydroxy-C₂-C₄-alkyl, Amino-C₄-C₇-alkyl oder Halogen-C₂-C₄-alkyl bedeutet und R₂ Carboxy, C₁-C₄-Alkoxycarbonyl oder eine gegebenenfalls durch C₁-C₄-Alkyl, C₁-C₄-Alkoxy, Halogen der Atomnummer bis und mit 35, Cyano und/oder Trifluormethyl mono- oder disubstituierte Phenyl-C₁-C₄-alkoxycarbonyl gruppe darstellt, und ihre Salze.

9. 2-Amino-6-hydroxy-4-phosphonomethyl-hex-3-ensäureäthylester oder ein Salz davon.

10. 2-Amino-6-hydroxy-4-phosphonomethyl-hex-3-ensäure oder ein Salz davon.

11. 2,10-Diamino-4-phosphonomethyl-dec-3-ensäure oder ein Salz davon.

12. 2,8-Diamino-4-phosphonomethyl-oct-3-ensäure oder ein Salz davon.

13. 2-Amino-6-methoxy-4-phosphonomethyl-hex-3-ensäureäthylester oder ein Salz davon.

14. 2-Amino-6-fluor-4-phosphonomethyl-hex-3-ensäureäthylester oder ein Salz davon.

15. 2-Amino-6-methoxy-4-phosphonomethyl-hex-3-ensäure oder ein Salz davon.

16. 2-Amino-6-benzoyloxy-4-phosphonomethyl-hex-3-ensäureäthylester oder ein Salz davon.

17. 2-Amino-7-hydroxy-4-phosphonomethyl-hept-3-ensäureäthylester oder ein Salz davon.

18. 2-Amino-7-hydroxy-4-phosphonomethyl-hept-3-ensäure oder ein Salz davon.

19. 2-Amino-6-benzyloxy-4-phosphonomethyl-hex-3-ensäure oder ein Salz davon.

20. 6-(N-Acetyl-N-methyl-amino)-2-amino-4-phosphonomethyl-hex-3-ensäureäthylester oder ein Salz davon.

21. (1-Acetylpiperidin-4-yl)-2-amino-5-phosphono-pent-3-ensäureäthylester oder ein Salz davon.

22. 2,10-Diamino-4-phosphonomethyl-dec-3-ensäureäthylester oder ein Salz davon.

23. 2-Amino-6-fluor-4-phosphonomethyl-hex-3-ensäure oder ein Salz davon.

24. 2-Amino-5-hydroxy-4-phosphonomethyl-pent-3-ensäureäthylester oder ein Salz davon.

25. 2-Amino-5-hydroxy-4-phosphonomethyl-pent-3-ensäure oder ein Salz davon.

26. 2-Amino-5-benzyloxy-4-phosphonomethyl-pent-3-ensäureäthylester oder ein Salz davon.

27. 2-Amino-5-benzyloxy-4-phosphonomethyl-pent-3-ensäure oder ein Salz davon.

28. 2-Amino-8-hydroxy-4-phosphonomethyl-oct-3-ensäureäthylester oder ein Salz davon.

29. 2-Amino-8-hydroxy-4-phosphonomethyl-oct-3-ensäure oder ein Salz davon.

30. 2-Amino-6-(N-methylamino)-4-phosphonomethyl-hex-3-ensäureäthylester oder ein Salz davon.

31. 2-Amino-6-(N-methylamino)-4-phosphonomethyl-hex-3-ensäure oder ein Salz davon.

32. 2-Amino-6-hydroxy-4-phosphonomethyl-hex-3-ensäure oder ein Salz davon.

33. 2-Amino-6-hydroxy-4-phosphonomethyl-hex-3-ensäureäthylester oder ein Salz davon.

34. 2-Amino-4-(piperidin-4-yl)-5-phosphono-pent-3-ensäureäthylester oder ein Salz davon.

35. 2-Amino-4-(piperidin-4-yl)-5-phosphono-pent-3-ensäure oder ein Salz davon.

36. 5-Äthoxy-2-amino-4-phosphonomethyl-pent-3-ensäure oder ein Salz davon.

37. 5-Äthoxy-2-amino-4-phosphonomethyl-pent-3-ensäureäthylester oder ein Salz davon.

38. 2-Amino-8-hydroxy-4-phosphonomethyl-oct-3-ensäureäthylester oder ein Salz davon.

39. 2-Amino-6-hydroxy-5-hydroxymethyl-4-phosphonomethyl-hex-3-ensäureäthylester oder ein Salz davon.

40. 2-Amino-10-hydroxy-4-phosphonomethyl-dec-3-ensäureäthylester oder ein Salz davon.

41. Eine Verbindung gemäss einem der Ansprüche 1, 3, 5, 7 und 15 bis 40 zur Anwendung in einem Verfahren zur therapeutischen Behandlung des menschlichen oder tierischen Körpers.

42. Eine Verbindung gemäss einem der Ansprüche 2, 4, 6 und 8 bis 14 zur Anwendung in einem Verfahren zur therapeutischen Behandlung des menschlichen oder tierischen Körpers.

43. Pharmazeutische Präparate enthaltend eine Verbindung gemäss einem der Ansprüche, 3, 5, 7 und 15 bis 41 in freier Form oder pharmazeutisch verwendbarer Salzform.

44. Pharmazeutische Präparate enthaltend eine Verbindung gemäss einem der Ansprüche 2, 4, 6, 8 bis 14 und 42 in freier Form oder pharmazeutisch verwendbarer Salzform.

45. Verfahren zur Herstellung ungesättigter Aminocarbonsäurederivate der Formel I 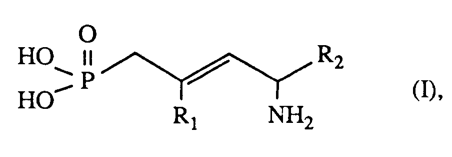 worin R₁ einen durch gegebenenfalls acyliertes oder aliphatisch oder araliphatisch veräthertes Hydroxy, durch Halogen, durch gegebenenfalls acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls aliphatisch N-substituierten oder N-acylierten azacycloaliphatischen Kohlenwasserstoffrest bedeutet und R₂ freies oder verestertes Carboxy darstellt, und ihrer Salze, dadurch gekennzeichnet, dass man
in einer Verbindung der Formel II 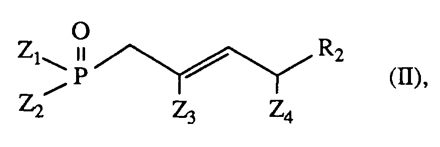 worin Z₁, Z₂ für gegebenenfalls geschütztes Hydroxy stehen, Z₃ einen einen durch gegebenenfalls geschütztes oder acyliertes oder aliphatisch oder araliphatisch veräthertes Hydroxy, durch Halogen, durch gegebenenfalls geschütztes oder acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls geschützten oder aliphatisch N-substituierten oder N-acylierten azacycloaliphatischen Kohlenwasserstoffrest darstellt und Z₄ geschütztes Amino bedeutet, geschütztes Amino Z₄ und, sofern vorhanden, als Bestandteil von Z₃ in Amino und, sofern vorhanden, geschütztes Hydroxy Z₁, Z₂ und/oder als Bestandteil von Z₃ in Hydroxy überführt und, sofern vorhanden einen geschützten azacycloaliphatischen Kohlenwasserstoffrest Z₃ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

46. Verwendung einer Verbindung gemäss einem der Ansprüche 1 bis 42 als Arzneimittelwirkstoff oder zur Herstellung eines pharmazeutischen Präparates.

## Patentansprüche (Patentansprüche für folgende(n) Vertragsstaat(en): ES, GR)

1. Verfahren zur Herstellung ungesättigter Aminocarbonsäurederivate der Formel I 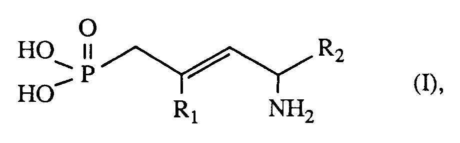 worin R₁ einen durch gegebenenfalls acyliertes oder aliphatisch oder araliphatisch veräthertes Hydroxy, durch Halogen, durch gegebenenfalls acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls aliphatisch N-substituierten oder N-acylierten azacycloaliphatischen Kohlenwasserstoffrest bedeutet und R₂ freies oder verestertes Carboxy darstellt, und ihrer Salze, dadurch gekennzeichnet, dass man
in einer Verbindung der Formel II 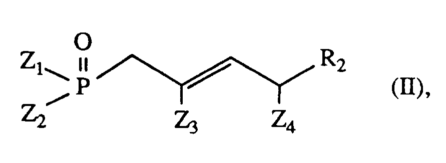 worin Z₁, Z₂ für gegebenenfalls geschütztes Hydroxy stehen, Z₃ einen einen durch gegebenenfalls geschütztes oder acyliertes oder aliphatisch oder araliphatisch veräthertes Hydroxy, durch Halogen, durch gegebenenfalls geschütztes oder acyliertes und/oder aliphatisch substituiertes Amino oder durch einen aza-, diaza-, azoxa- oder oxacycloaliphatischen Rest substituierten aliphatischen oder über ein C-Atom gebundenen oxacycloaliphatischen oder gegebenenfalls geschützten oder aliphatisch N-substituierten oder N-acylierten azacycloaliphatischen Kohlenwasserstoffrest darstellt und Z₄ geschütztes Amino bedeutet, geschütztes Amino Z₄ und, sofern vorhanden, als Bestandteil von Z₃ in Amino und, sofern vorhanden, geschütztes Hydroxy Z₁, Z₂ und/oder als Bestandteil von Z₃ in Hydroxy überführt und, sofern vorhanden einen geschützten azacycloaliphatischen Kohlenwasserstoffrest Z₃ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder ein verfahrensgemäss erhältliches Salz in die entsprechende freie Verbindung überführt.

2. Verfahren gemäss Anspruch 1 zur Herstellung von Verbindungen der Formel I, worin R₁ einen durch gegebenenfalls aliphatisch oder araliphatisch veräthertes Hydroxy, gegebenenfalls aliphatisch substituiertes Amino oder Halogen substituierten aliphatischen Kohlenwasserstoffrest bedeutet und R₂ freies oder verestertes Carboxy darstellt, und ihrer Salze.

3. Verfahren gemäss Anspruch 1 zur Herstellung von Verbindungen der Formel I, worin R₁ Mono- oder Dihydroxyniederalkyl, Niederalkanoyloxyniederalkyl, Benzoyloxyniederalkyl, Niederalkoxyniederalkyl, Phenylniederalkoxyniederalkyl, Halogenniederalkyl, Aminoniederalkyl, Niederalkylaminoniederalkyl, Niederalkanoylaminoniederalkyl, Diniederalkylaminoniederalkyl, N-Niederalkyl-N-niederalkanoyl-aminoniederalkyl, 5- bis 7-gliedriges Azacycloalkylniederalkyl, dessen Azacyloalkylteil über das N- oder ein C-Atom gebunden und im zweitgenannten Fall gegebenenfalls N-niederalkyliert, N-niederalkanoyliert oder durch eine im Phenylteil gegebenenfalls substituierte Benzoylgruppe N-substituiert sein kann, 5- bis 7-gliedriges Diazacycloalkylniederalkyl, dessen Diazacycloalkylteil über ein N-Atom gebunden und gegebenenfalls N'-niederalkyliert, N'-niederalkanoyliert oder durch eine im Phenylteil gegebenenfalls substituierte Benzoylgruppe N'-substituiert ist, 5- bis 7-gliedriges, über das N-Atom gebundenes Azoxacycloalkylniederalkyl, 5- bis 7-gliedriges, über ein C-Atom gebundenes Oxacycloalkylniederalkyl, 5- bis 7-gliedriges, über ein C-Atom gebundenes, gegebenenfalls N-niederalkyliertes, N-niederalkanoyliertes oder durch eine im Phenylteil gegebenenfalls substituierte Benzoylgruppe N-substituiertes Azacycloalkyl oder 5- bis 7-gliedriges, über ein C-Atom gebundenes Oxacycloalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihrer Salze.

4. Verfahren gemäss Anspruch 1 zur Herstellung von Verbindungen der Formel I, worin R₁ Hydroxyniederalkyl, Niederalkoxyniederalkyl, Phenylniederalkoxyniederalkyl, Aminoniederalkyl, Niederalkylaminoniederalkyl, Diniederalkylaminoniederalkyl, N,N-Niederalkylenamino bzw. N,N-(Aza- oder Oxaniederalkylen)aminoniederalkyl oder Halogenniederalkyl bedeutet und R₂ Carboxy, Niederalkoxycarbonyl, 4- bis und mit 7-gliedriges Cycloalkoxycarbonyl oder Phenylniederalkoxycarbonyl darstellt, wobei in den genannten Gruppen R₁ und/oder R₂ gegebenenfalls vorhandene Phenylreste unsubstituiert oder durch Niederalkyl, Niederalkoxy, Halogen, Cyano und/oder Trifluormethyl mono-, di- oder trisubstituiert sein können, und ihrer Salze.

5. Verfahren gemäss Anspruch 1 zur Herstellung von Verbindungen der Formel I, worin R₁ Hydroxy-C₁-C₇-alkyl, Dihydroxy-C₂-C₇-alkyl, C₂-C₇-Alkanoyloxy-C₁-C₇-alkyl, im Phenylteil gegebenenfalls durch C₁-C₄-Alkyl, C₁-C₄-Alkoxy, Halogen der Atomnummer bis und mit 35, Cyano und/oder Trifluormethyl mono- oder disubstituiertes Benzoyloxy-C₁-C₇-alkyl, C₁-C₄-Alkoxy-C₁-C₇-alkyl, eine im Phenylteil gegebenenfalls durch C₁-C₄-Alkyl, C₁-C₄-Alkoxy, Halogen der Atomnummer bis und mit 35, Cyano und/oder Trifluormethyl mono- oder disubstituierte Phenyl-C₁-C₄-alkoxy-C₁-C₇-alkylgruppe, Halogen-C₁-C₇-alkyl, wobei Halogen Chlor oder Fluor bedeutet, Amino-C₁-C₇-alkyl, C₁-C₄-Alkylamino-C₁-C₇-alkyl, C₂-C₇-Alkanoylamino-C₁-C₇-alkyl, N-C₂-C₇-Alkanoyl-N-C₁-C₄-alkyl-amino-C₁-C₇-alkyl, Di-C₁-C₇-Alkylamino-C₁-C₇-alkyl, 5- bis 7-gliedriges Azacycloalk-1-yl-C₁-C₇-alkyl, Azacycloalk-3-yl-C₁-C₇-alkyl bzw. Azacycloalk-4-yl-C₁-C₇-alkyl, 1-C₂-C₇-Alkanoylazacycloalk-3-yl-C₁-C₇-alkyl bzw. 4-yl-C₁-C₇-alkyl, N-C₁-C₄-Alkylazacycloalkyl-3-yl-C₁-C₇-alkyl bzw. 4-yl-C₁-C₇-alkyl, N-C₁-C₄-Benzoylazacycloalkyl-3-yl-C₁-C₇-alkyl bzw. 4-yl-C₁-C₇-alkyl, 5- bis 7-gliedriges Diazacycloalk-1-yl-C₁-C₇-alkyl, N'-C₁-C₄-Alkyldiazacycloalk-1-yl-C₁-C₇-alkyl, N'-C₂-C₇-Alkanoylazacycloalk-1-yl-C₁-C₇-alkyl, 5- bis 7-gliedriges Azoxacycloalk-1-yl-C₁-C₇-alkyl, 5- bis 7-gliedriges Oxacycloalk-3-yl-C₁-C₇-alkyl oder -4-yl-C₁-C₇-alkyl, 5- bis 7-gliedriges Azacycloalk-3-yl bzw. 4-yl oder 1-C₂-C₇-Alkanoyl-azacycloalk-3-yl bzw. 4-yl, N-C₁-C₄-Alkylazacycloalk-3-yl bzw. -4-yl oder im Phenylteil gegebenenfalls substituiertes N-Benzoylazacycloalk-3-yl oder -4-yl oder 5- bis 7-gliedriges Oxacycloalk-3-yl bzw. 4-yl darstellt und R₂ Carboxy, C₁-C₄-Alkoxycarbonyl, 5- bis 7-gliedriges Cycloalkoxycarbonyl oder gegebenenfalls durch C₁-C₄-Alkyl, C₁-C₄-Alkoxy, Halogen der Atomnummer bis und mit 35, Cyano und/oder Trifluormethyl mono- oder disubstituiertes Phenyl-C₁-C₄-alkoxycarbonyl ist, und ihrer Salze.

6. Verfahren gemäss Anspruch 1 zur Herstellung von Verbindungen der Formel I, worin R₁ Hydroxy-C₁-C₇-alkyl, C₁-C₄-Alkoxy-C₁-C₇-alkyl, eine im Phenylteil gegebenenfalls durch C₁-C₄-Alkyl, C₁-C₄-Alkoxy, Halogen der Atomnummer bis und mit 35, Cyano und/oder Trifluormethyl mono-, di- oder trisubstituierte Phenyl-C₁-C₄-alkoxy-C₁-C₇-alkylgruppe, Amino-C₁-C₇-alkyl, C₁-C₄-Alkylamino-C₁-C₇-alkyl, C₂-C₇-Alkanoylamino-C₁-C₇-alkyl, Di-C₁-C₇-Alkylamino-C₁-C₇-alkyl, 5- bis 7-gliedriges N,N-(Aza- oder Oxaalkylen)amino-C₁-C₇-alkyl oder Halogen-C₁-C₇-alkyl darstellt, wobei Halogen Chlor oder Fluor bedeutet und R₂ Carboxy, C₁-C₄-Alkoxycarbonyl, 5- bis 7-gliedriges Cycloalkoxycarbonyl, gegebenenfalls durch C₁-C₄-Alkyl, C₁-C₄-Alkoxy, Halogen der Atomnummer bis und mit 35, Cyano und/oder Trifluormethyl mono- oder disubstituiertes Phenyl-C₁-C₄-alkoxycarbonyl ist, und ihrer Salze.

7. Verfahren gemäss Anspruch 1 zur Herstellung von Verbindungen der Formel I, worin R₁ Hydroxy-C₁-C₇-alkyl, C₁-C₄-Alkoxy-C₁-C₇-alkyl, Benzoyloxy-C₁-C₄-alkyl, Amino-C₄-C₇-alkyl, N-C₂-C₇-Alkanoyl-N-C₁-C₄-alkyl-amino-C₂-C₇-alkyl, 5-bis 7-gliedriges Azacycloalk-3-yl bzw. 4-yl oder 1-C₂-C₇-Alkanoyl-azacycloalk-3-yl bzw. 4-yl, Halogen-C₁-C₄-alkyl, wobei Halogen Chlor oder Fluor bedeutet, Amino-C₄-C₇-alkyl, N-C₁-C₄-Alkanoyl-N-C₁-C₄-alkyl-amino-C₁-C₇-alkyl, Piperidin-4-yl oder 1-C₂-C₇-Alkanoylpiperidin-4-yl darstellt und R₂ Carboxy oder C₁-C₄-Alkoxycarbonyl bedeutet, und ihrer Salze.

8. Verfahren gemäss Anspruch 1 zur Herstellung von Verbindungen der Formel I, worin R₁ C₁-C₄-Alkoxy-C₂-C₄-alkyl, Hydroxy-C₂-C₄-alkyl, Amino-C₄-C₇-alkyl oder Halogen-C₂-C₄-alkyl bedeutet und R₂ Carboxy, C₁-C₄-Alkoxycarbonyl oder eine gegebenenfalls durch C₁-C₄-Alkyl, C₁-C₄-Alkoxy, Halogen der Atomnummer bis und mit 35, Cyano und/oder Trifluormethyl mono- oder disubstituierte Phenyl-C₁-C₄-alkoxycarbonylgruppe darstellt, und ihrer Salze.

9. Verfahren gemäss Anspruch 1 zur Herstellung von 2-Amino-6-hydroxy-4-phosphonomethyl-hex-3-ensäureäthylester oder eines Salzes davon.

10. Verfahren gemäss Anspruch 1 zur Herstellung von 2-Amino-6-hydroxy-4-phosphonomethyl-hex-3-ensäure oder eines Salzes davon.

11. Verfahren gemäss Anspruch 1 zur Herstellung von 2,10-Diamino-4-phosphonomethyl-dec-3-ensäure oder eines Salzes davon.

12. Verfahren gemäss Anspruch 1 zur Herstellung von 2,8-Diamino-4-phosphonomethyl-oct-3-ensäure oder eines Salzes davon.

13. Verfahren gemäss Anspruch 1 zur Herstellung von 2-Amino-6-methoxy-4-phosphonomethyl-hex-3-ensäureäthylester oder eines Salzes davon.

14. Verfahren gemäss Anspruch 1 zur Herstellung von 2-Amino-6-fluor-4-phosphonomethyl-hex-3-ensäureäthylester oder eines Salzes davon.

15. Verfahren gemäss Anspruch 1 zur Herstellung von 2-Amino-6-methoxy-4-phosphonomethyl-hex-3-ensäure oder eines Salzes davon.

16. Verfahren gemäss Anspruch 1 zur Herstellung von 2-Amino-6-benzoyloxy-4-phosphonomethyl-hex-3-ensäureäthylester oder eines Salzes davon.

17. Verfahren gemäss Anspruch 1 zur Herstellung von 2-Amino-7-hydroxy-4-phosphonomethyl-hept-3-ensäureäthylester oder eines Salzes davon.

18. Verfahren gemäss Anspruch 1 zur Herstellung von 2-Amino-7-hydroxy-4-phosphonomethyl-hept-3-ensäure oder eines Salzes davon.

19. Verfahren gemäss Anspruch 1 zur Herstellung von 2-Amino-6-benzyloxy-4-phosphonomethyl-hex-3-ensäure oder eines Salzes davon.

20. Verfahren gemäss Anspruch 1 zur Herstellung von 6-(N-Acetyl-N-methyl-amino)-2-amino-4-phosphonomethyl-hex-3-ensäureäthylester oder eines Salzes davon.

21. Verfahren gemäss Anspruch 1 zur Herstellung von (1-Acetylpiperidin-4-yl)-2-amino-5-phosphonopent-3-ensäureäthylester oder eines Salzes davon.

22. Verfahren gemäss Anspruch 1 zur Herstellung von 2,10-Diamino-4-phosphonomethyl-dec-3-ensäureäthylester oder eines Salzes davon.

23. Verfahren gemäss Anspruch 1 zur Herstellung von 2-Amino-6-fluor-4-phosphonomethyl-hex-3-ensäure oder eines Salzes davon.

24. Verfahren gemäss Anspruch 1 zur Herstellung von 2-Amino-5-hydroxy-4-phosphonomethyl-pent-3-ensäureäthylester oder eines Salzes davon.

25. Verfahren gemäss Anspruch 1 zur Herstellung von 2-Amino-5-hydroxy-4-phosphonomethyl-pent-3-ensäure oder eines Salzes davon.

26. Verfahren gemäss Anspruch 1 zur Herstellung von 2-Amino-5-benzyloxy-4-phosphonomethyl-pent-3-ensäureäthylester oder eines Salzes davon.

27. Verfahren gemäss Anspruch 1 zur Herstellung von 2-Amino-5-benzyloxy-4-phosphonomethyl-pent-3-ensäure oder eines Salzes davon.

28. Verfahren gemäss Anspruch 1 zur Herstellung von 2-Amino-8-hydroxy-4-phosphonomethyl-oct-3-ensäureäthylester oder eines Salzes davon.

29. Verfahren gemäss Anspruch 1 zur Herstellung von 2-Amino-8-hydroxy-4-phosphonomethyl-oct-3-ensäure oder eines Salzes davon.

30. Verfahren gemäss Anspruch 1 zur Herstellung von 2-Amino-6-(N-methylamino)-4-phosphonomethyl-hex-3-ensäureäthylester oder eines Salzes davon.

31. Verfahren gemäss Anspruch 1 zur Herstellung von 2-Amino-6-(N-methylamino)-4-phosphonomethyl-hex-3-ensäure oder eines Salzes davon.

32. Verfahren gemäss Anspruch 1 zur Herstellung von 2-Amino-6-hydroxy-4-phosphonomethyl-hex-3-ensäure oder eines Salzes davon.

33. Verfahren gemäss Anspruch 1 zur Herstellung von 2-Amino-6-hydroxy-4-phosphonomethyl-hex-3-ensäureäthylester oder eines Salzes davon.

34. Verfahren gemäss Anspruch 1 zur Herstellung von 2-Amino-4-(piperidin-4-yl)-5-phosphono-pent-3-ensäureäthylester oder eines Salzes davon.

35. Verfahren gemäss Anspruch 1 zur Herstellung von 2-Amino-4-(piperidin-4-yl)-5-phosphono-pent-3-ensäure oder eines Salzes davon.

36. Verfahren gemäss Anspruch 1 zur Herstellung von 5-Äthoxy-2-amino-4-phosphonomethyl-pent-3-ensäure oder eines Salzes davon.

37. Verfahren gemäss Anspruch 1 zur Herstellung von 5-Äthoxy-2-amino-4-phosphonomethyl-pent-3-ensäureäthylester oder eines Salzes davon.

38. Verfahren gemäss Anspruch 1 zur Herstellung von 2-Amino-8-hydroxy-4-phosphonomethyl-oct-3-ensäureäthylester oder eines Salzes davon.

39. Verfahren gemäss Anspruch 1 zur Herstellung von 2-Amino-6-hydroxy-5-hydroxymethyl-4-phosphonomethyl-hex-3-ensäureäthylester oder eines Salzes davon.

40. Verfahren gemäss Anspruch 1 zur Herstellung von 2-Amino-10-hydroxy-4-phosphonomethyl-dec-3-ensäureäthylester oder eines Salzes davon.

41. Verfahren zur Herstellung pharmazeutischer Präparate, dadurch gekennzeichnet, dass man eine gemäss einem der Ansprüche 1 bis 40 erhältliche Verbindung in freier Form oder in pharmazeutisch verwendbarer Salzform mit üblichen pharmazeutischen Hilfsstoffen vermischt.

## Claims (Claims for the following Contracting State(s): AT, BE, CH, DE, DK, FR, GB, IT, LU, LI, NL, SE)

1. A substituted 2-aminoalk-3-enoic acid derivative of formula I 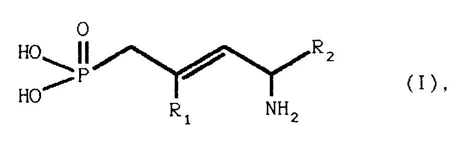 wherein R₁ is an aliphatic hydrocarbon radical that is substituted by optionally acylated or aliphatically or araliphatically etherified hydroxy, by halogen, by optionally acylated and/or aliphatically substituted amino or by an aza-, diaza-, azoxa- or oxa-cycloaliphatic radical, or is an oxacycloaliphatic hydrocarbon radical bonded via a carbon atom, or is an optionally aliphatically N-substituted or N-acylated azacycloaliphatic hydrocarbon radical bonded via a carbon atom, and R₂ is free or esterified carboxy, or a salt thereof.

2. A compound according to claim 1 of formula I, wherein R₁ is an aliphatic hydrocarbon radical that is substituted by optionally aliphatically or araliphatically etherified hydroxy, optionally aliphatically substituted amino or by halogen, and R₂ is free or esterified carboxy, or a salt thereof.

3. A compound according to claim 1 of formula I, wherein R₁ is mono- or di-hydroxy-lower alkyl, lower alkanoyloxy-lower alkyl, benzoyloxy-lower alkyl, lower alkoxy-lower alkyl, phenyl-lower alkoxy-lower alkyl, halo-lower alkyl, amino-lower alkyl, lower alkylamino-lower alkyl, lower alkanoyl-amino-lower alkyl, di-lower alkylamino-lower alkyl, N-lower alkyl-N-lower alkanoylamino-lower alkyl, 5- to 7-membered azacycloalkyl-lower alkyl whose azacycloalkyl moiety is bonded via the N atom or a carbon atom and, in the latter case, may be N-lower alkylated, N-lower alkanoylated or N-substituted by a benzoyl group that is unsubstituted or substituted in the phenyl moiety, 5- to 7-membered diazacycloalkyl-lower alkyl whose diazacycloalkyl moiety is bonded via an N atom and is optionally N'-lower alkylated, N'-lower alkanoylated or N'-substituted by a benzoyl group that is unsubstituted or substituted in the phenyl moiety, 5- to 7-membered azoxacycloalkyl-lower alkyl bonded via the N atom, 5- to 7-membered oxacycloalkyl-lower alkyl bonded via a carbon atom, 5- to 7-membered azacycloalkyl that is bonded via a carbon atom and is optionally N-lower alkylated, N-lower alkanoylated or N-substituted by a benzoyl group that is unsubstituted or substituted in the phenyl moiety, or 5-to 7-membered oxacycloalkyl bonded via a carbon atom, and R₂ is carboxy, lower alkoxycarbonyl, 4- up to and including 7-membered cycloalkoxycarbonyl or phenyl-lower alkoxycarbonyl, any phenyl radicals in the mentioned groups R₁ and/or R₂ being unsubstituted or mono-, di- or tri-substituted by lower alkyl, lower alkoxy, halogen, cyano and/or by trifluoromethyl, or a salt thereof.

4. A compound according to claim 2 of formula I, wherein R₁ is hydroxy-lower alkyl, lower alkoxy-lower alkyl, phenyl-lower alkoxy-lower alkyl, amino-lower alkyl, lower alkylamino-lower alkyl, di-lower alkylamino-lower alkyl, N,N-lower alkyleneamino- or N,N-(aza- or oxa-lower alkylene)amino-lower alkyl or halo-lower alkyl, and R₂ is carboxy, lower alkoxycarbonyl, 4- up to and including 7-membered cycloalkoxycarbonyl or phenyl-lower alkoxycarbonyl, any phenyl radicals in the mentioned groups R₁ and/or R₂ being unsubstituted or mono-, di- or tri-substituted by lower alkyl, lower alkoxy, halogen, cyano and/or by trifluoromethyl, or a salt thereof.

5. A compound according to claim 1 of formula I, wherein R₁ is hydroxy-C₁-C₇alkyl, dihydroxy-C₂-C₇alkyl, C₂-C₇alkanoyloxy-C₁-C₇alkyl, benzoyloxy-C₁-C₇alkyl that is unsubstituted or mono- or di-substituted in the phenyl moiety by C₁-C₄alkyl, C₁-C₄alkoxy, halogen having an atomic number of up to and including 35, cyano and/or by trifluoromethyl, C₁-C₄alkoxy-C₁-C₇alkyl, a phenyl-C₁-C₄alkoxy-C₁-C₇alkyl group that is unsubstituted or mono- or di-substituted in the phenyl moiety by C₁-C₄alkyl, C₁-C₄alkoxy, halogen having an atomic number of up to and including 35, cyano and/or by trifluoromethyl, halo-C₁-C₇alkyl, in which halogen is chlorine or fluorine, amino-C₁-C₇alkyl, C₁-C₄-alkylamino-C₁-C₇alkyl, C₂-C₇alkanoylamino-C₁-C₇alkyl, N-C₂-C₇alkanoyl-N-C₁-C₄alkylamino-C₁-C₇alkyl, di-C₁-C₇alkylamino-C₁-C₇alkyl, 5- to 7-membered azacycloalk-1-yl-C₁-C₇alkyl, azacycloalk-3-yl-C₁-C₇alkyl or azacycloalk-4-yl-C₁-C₇alkyl, 1-C₂-C₇alkanoylazacycloalk-3-yl-C₁-C₇alkyl or -4-yl-C₁-C₇alkyl, N-C₁-C₄alkylazacycloalk-3-yl-C₁-C₇alkyl or -4-yl-C₁-C₇alkyl, N-benzoylazacycloalk-3-yl-C₁-C₇alkyl or -4-yl-C₁-C₇alkyl, 5- to 7-membered diazacycloalk-1-yl-C₁-C₇alkyl, N'-C₁-C₄alkyldiazacycloalk-1-yl-C₁-C₇alkyl, N'-C₂-C₇alkanoylazacycloalk-1-yl-C₁-C₇alkyl, 5- to 7-membered azoxacycloalk-1-yl-C₁-C₇alkyl, 5- to 7-membered oxacycloalk-3-yl-C₁-C₇alkyl or -4-yl-C₁-C₇alkyl, 5- to 7-membered azacycloalk-3-yl or -4-yl or 1-C₂-C₇alkanoylazacycloalk-3-yl or -4-yl, N-C₁-C₄alkylazacycloalk-3-yl or -4-yl, or N-benzoylazacycloalk-3-yl or -4-yl that is unsubstituted or substituted in the phenyl moiety, or 5- to 7-membered oxacycloalk-3-yl or -4-yl, and R₂ is carboxy, C₁-C₄alkoxycarbonyl, 5- to 7-membered cycloalkoxycarbonyl, or phenyl-C₁-C₄alkoxycarbonyl that is unsubstituted or mono- or di-substituted by C₁-C₄alkyl, C₁-C₄alkoxy, halogen having an atomic number of up to and including 35, cyano and/or by trifluoromethyl, or a salt thereof.

6. A compound according to claim 2 of formula I, wherein R₁ is hydroxy-C₁-C₇alkyl, C₁-C₄alkoxy-C₁-C₇alkyl, a phenyl-C₁-C₄alkoxy-C₁-C₇alkyl group that is unsubstituted or mono-, di- or tri-substituted in the phenyl moiety by C₁-C₄alkyl, C₁-C₄alkoxy, halogen having an atomic number of up to and including 35, cyano and/or by trifluoromethyl, amino-C₁-C₇alkyl, C₁-C₄alkylamino-C₁-C₇alkyl, C₂-C₇alkanoylamino-C₁-C₇alkyl, di-C₁-C₇alkylamino-C₁-C₇alkyl, 5- to 7-membered N,N-(aza- or oxa-alkylene)amino-C₁-C₇alkyl, or halo-C₁-C₇alkyl, in which halogen is chlorine or fluorine, and R₂ is carboxy, C₁-C₄alkoxycarbonyl, 5- to 7-membered cycloalkoxycarbonyl, or phenyl-C₁-C₄alkoxycarbonyl that is unsubstituted or mono- or di-substituted by C₁-C₄alkyl, C₁-C₄alkoxy, halogen having an atomic number of up to and including 35, cyano and/or by trifluoromethyl, or a salt thereof.

7. A compound according to claim 1 of formula I, wherein R₁ is hydroxy-C₁-C₇alkyl, C₁-C₄alkoxy-C₁-C₇alkyl, benzoyloxy-C₁-C₄alkyl, amino-C₄-C₇alkyl, N-C₂-C₇alkanoyl-N-C₁-C₄-alkylamino-C₂-C₇alkyl, 5- to 7-membered azacycloalk-3-yl or -4-yl or 1-C₂-C₇alkanoylazacycloalk-3-yl or -4-yl, halo-C₁-C₄alkyl, in which halogen is chlorine or fluorine, amino-C₄-C₇alkyl, N-C₁-C₄alkanoyl-N-C₁-C₄alkylamino-C₁-C₇alkyl, piperidin-4-yl or 1-C₂-C₇alkanoylpiperidin-4-yl, and R₂ is carboxy or C₁-C₄alkoxycarbonyl, or a salt thereof.

8. A compound according to claim 2 of formula I, wherein R₁ is C₁-C₄alkoxy-C₂-C₄alkyl, hydroxy-C₂-C₄alkyl, amino-C₄-C₇alkyl or halo-C₂-C₄alkyl, and R₂ is carboxy, C₁-C₄alkoxycarbonyl or a phenyl-C₁-C₄alkoxycarbonyl group that is unsubstituted or mono- or di-substituted by C₁-C₄alkyl, C₁-C₄alkoxy, halogen having an atomic number of up to and including 35, cyano and/or by trifluoromethyl, or a salt thereof.

9. 2-Amino-6-hydroxy-4-phosphonomethyl-hex-3-enoic acid ethyl ester or a salt thereof.

10. 2-Amino-6-hydroxy-4-phosphonomethyl-hex-3-enoic acid or a salt thereof.

11. 2,10-Diamino-4-phosphonomethyl-dec-3-enoic acid or a salt thereof.

12. 2,8-Diamino-4-phosphonomethyl-oct-3-enoic acid or a salt thereof.

13. 2-Amino-6-methoxy-4-phosphonomethyl-hex-3-enoic acid ethyl ester or a salt thereof.

14. 2-Amino-6-fluoro-4-phosphonomethyl-hex-3-enoic acid ethyl ester or a salt thereof.

15. 2-Amino-6-methoxy-4-phosphonomethyl-hex-3-enoic acid or a salt thereof.

16. 2-Amino-6-benzoyloxy-4-phosphonomethyl-hex-3-enoic acid ethyl ester or a salt thereof.

17. 2-Amino-7-hydroxy-4-phosphonomethyl-hept-3-enoic acid ethyl ester or a salt thereof.

18. 2-Amino-7-hydroxy-4-phosphonomethyl-hept-3-enoic acid or a salt thereof.

19. 2-Amino-6-benzyloxy-4-phosphonomethyl-hex-3-enoic acid or a salt thereof.

20. 6-(N-Acetyl-N-methylamino)-2-amino-4-phosphonomethyl-hex-3-enoic acid ethyl ester or a salt thereof.

21. (1-Acetylpiperidin-4-yl)-2-amino-5-phosphono-pent-3-enoic acid ethyl ester or a salt thereof.

22. 2,10-Diamino-4-phosphonomethyl-dec-3-enoic acid ethyl ester or a salt thereof.

23. 2-Amino-6-fluoro-4-phosphonomethyl-hex-3-enoic acid or a salt thereof.

24. 2-Amino-5-hydroxy-4-phosphonomethyl-pent-3-enoic acid ethyl ester or a salt thereof.

25. 2-Amino-5-hydroxy-4-phosphonomethyl-pent-3-enoic acid or a salt thereof.

26. 2-Amino-5-benzyloxy-4-phosphonomethyl-pent-3-enoic acid ethyl ester or a salt thereof.

27. 2-Amino-5-benzyloxy-4-phosphonomethyl-pent-3-enoic acid or a salt thereof.

28. 2-Amino-8-hydroxy-4-phosphonomethyl-oct-3-enoic acid ethyl ester or a salt thereof.

29. 2-Amino-8-hydroxy-4-phosphonomethyl-oct-3-enoic acid or a salt thereof.

30. 2-Amino-6-(N-methylamino)-4-phosphonomethyl-hex-3-enoic acid ethyl ester or a salt thereof.

31. 2-Amino-6-(N-methylamino)-4-phosphonomethyl-hex-3-enoic acid or a salt thereof.

32. 2-Amino-6-hydroxy-4-phosphonomethyl-hex-3-enoic acid or a salt thereof.

33. 2-Amino-6-hydroxy-4-phosphonomethyl-hex-3-enoic acid ethyl ester or a salt thereof.

34. 2-Amino-4-(piperidin-4-yl)-5-phosphono-pent-3-enoic acid ethyl ester or a salt thereof.

35. 2-Amino-4-(piperidin-4-yl)-5-phosphono-pent-3-enoic acid or a salt thereof.

36. 5-Ethoxy-2-amino-4-phosphonomethyl-pent-3-enoic acid or a salt thereof.

37. 5-Ethoxy-2-amino-4-phosphonomethyl-pent-3-enoic acid ethyl ester or a salt thereof.

38. 2-Amino-8-hydroxy-4-phosphonomethyl-oct-3-enoic acid ethyl ester or a salt thereof.

39. 2-Amino-6-hydroxy-5-hydroxymethyl-4-phosphonomethyl-hex-3-enoic acid ethyl ester or a salt thereof.

40. 2-Amino-10-hydroxy-4-phosphonomethyl-dec-3-enoic acid ethyl ester or a salt thereof.

41. A compound according to any one of claims 1, 3, 5, 7 and 15 to 40 for use in a method for the therapeutic treatment of the human or animal body.

42. A compound according to any one of claims 2, 4, 6 and 8 to 14 for use in a method for the therapeutic treatment of the human or animal body.

43. A pharmaceutical composition comprising a compound according to any one of claims 3, 5, 7 and 15 to 41 in free form or in the form of a pharmaceutically acceptable salt.

44. A pharmaceutical composition comprising a compound according to any one of claims 2, 4, 6, 8 to 14 and 42 in free form or in the form of a pharmaceutically acceptable salt.

45. A process for the preparation of an unsaturated aminocarboxylic acid derivative of formula I 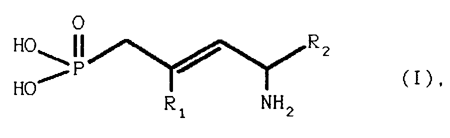 wherein R₁ is an aliphatic hydrocarbon radical that is substituted by optionally acylated or aliphatically or araliphatically etherified hydroxy, by halogen, by optionally acylated and/or aliphatically substituted amino or by an aza-, diaza-, azoxa- or oxa-cycloaliphatic radical, or is an oxacycloaliphatic hydrocarbon radical bonded via a carbon atom, or is an optionally aliphatically N-substituted or N-acylated azacycloaliphatic hydrocarbon radical bonded via a carbon atom, and R₂ is free or esterified carboxy, or of a salt thereof, which process comprises
in a compound of formula II 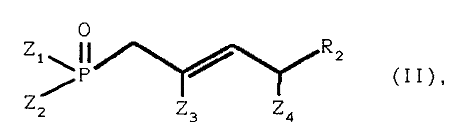 wherein Z₁, Z₂ are optionally protected hydroxy, Z₃ is an aliphatic hydrocarbon radical that is substituted by optionally protected or acylated or aliphatically or araliphatically etherified hydroxy, by halogen, by optionally protected or acylated and/or aliphatically substituted amino or by an aza-, diaza-, azoxa- or oxa-cycloaliphatic radical, or is an oxacycloaliphatic hydrocarbon radical bonded via a carbon atom, or is an optionally protected or aliphatically N-substituted or N-acylated azacycloaliphatic hydrocarbon radical, and Z₄ is protected amino, converting protected amino Z₄ and, if present, protected amino as a constituent of Z₃ into amino and, if present, converting protected hydroxy Z₁, Z₂ and/or protected hydroxy as a constituent of Z₃ into hydroxy and, if present, freeing a protected azacycloaliphatic hydrocarbon radical Z₃ and, if desired, converting a resulting compound into a different compound of formula I, separating an isomeric mixture obtainable in accordance with the process into its components and separating the preferred isomer, and/or converting a free compound obtainable in accordance with the process into a salt or converting a salt obtainable in accordance with the process into the corresponding free compound.

46. The use of a compound according to any one of claims 1 to 42 as an active ingredient in a medicament or for the preparation of a pharmaceutical composition.

## Claims (Claims for the following Contracting State(s): ES, GR)

1. A process for the preparation of an unsaturated amino-carboxylic acid derivative of formula I 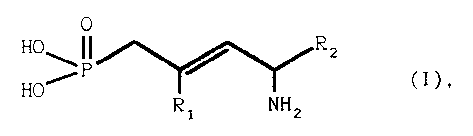 wherein R₁ is an aliphatic hydrocarbon radical that is substituted by optionally acylated or aliphatically or araliphatically etherified hydroxy, by halogen, by optionally acylated and/or aliphatically substituted amino or by an aza-, diaza-, azoxa- or oxa-cycloaliphatic radical, or is an oxacycloaliphatic hydrocarbon radical bonded via a carbon atom, or is an optionally aliphatically N-substituted or N-acylated azacycloaliphatic hydrocarbon radical bonded via a carbon atom , and R₂ is free or esterified carboxy, or of a salt thereof, which process comprises
in a compound of formula II 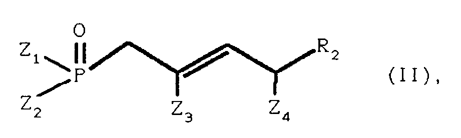 wherein Z₁, Z₂ are optionally protected hydroxy, Z₃ is an aliphatic hydrocarbon radical that is substituted by optionally protected or acylated or aliphatically or araliphatically etherified hydroxy, by halogen, by optionally protected or acylated and/or aliphatically substituted amino or by an aza-, diaza-, azoxa- or oxa-cycloaliphatic radical, or is an oxacycloaliphatic hydrocarbon radical bonded via a carbon atom, or is an optionally protected or aliphatically N-substituted or N-acylated azacycloaliphatic hydrocarbon radical, and Z₄ is protected amino, converting protected amino Z₄ and, if present, protected amino as a constituent of Z₃ into amino and, if present, converting protected hydroxy Z₁, Z₂ and/or protected hydroxy as a constituent of Z₃ into hydroxy and, if present, freeing a protected azacycloaliphatic hydrocarbon radical Z₃ and, if desired, converting a resulting compound into a different compound of formula I, separating an isomeric mixture obtainable in accordance with the process into its components and separating the preferred isomer, and/or converting a free compound obtainable in accordance with the process into a salt or converting a salt obtainable in accordance with the process into the corresponding free compound.

2. A process according to claim 1 for the preparation of a compound of formula I wherein R₁ is an aliphatic hydrocarbon radical that is substituted by optionally aliphatically or araliphatically etherified hydroxy, optionally aliphatically substituted amino or by halogen, and R₂ is free or esterified carboxy, or of a salt thereof.

3. A process according to claim 1 for the preparation of a compound of formula I wherein R₁ is mono- or di-hydroxy-lower alkyl, lower alkanoyloxy-lower alkyl, benzoyloxy-lower alkyl, lower alkoxy-lower alkyl, phenyl-lower alkoxy-lower alkyl, halo-lower alkyl, amino-lower alkyl, lower alkylamino-lower alkyl, lower alkanoylamino-lower alkyl, di-lower alkylamino-lower alkyl, N-lower alkyl-N-lower alkanoylamino-lower alkyl, 5- to 7-membered azacycloalkyl-lower alkyl whose azacycloalkyl moiety is bonded via the N atom or a carbon atom and, in the latter case, may be N-lower alkylated, N-lower alkanoylated or N-substituted by a benzoyl group that is unsubstituted or substituted in the phenyl moiety, 5- to 7-membered diazacycloalkyl-lower alkyl whose diazacycloalkyl moiety is bonded via an N atom and is optionally N'-lower alkylated, N'-lower alkanoylated or N'-substituted by a benzoyl group that is unsubstituted or substituted in the phenyl moiety, 5- to 7-membered azoxacycloalkyl-lower alkyl bonded via the N atom, 5- to 7-membered oxacycloalkyl-lower alkyl bonded via a carbon atom, 5- to 7-membered azacycloalkyl that is bonded via a carbon atom and is optionally N-lower alkylated, N-lower alkanoylated or N-substituted by a benzoyl group that is unsubstituted or substituted in the phenyl moiety, or 5- to 7-membered oxacycloalkyl bonded via a carbon atom, and R₂ is carboxy, lower alkoxycarbonyl, 4- up to and including 7-membered cycloalkoxycarbonyl or phenyl-lower alkoxycarbonyl, any phenyl radicals in the mentioned groups R₁ and/or R₂ being unsubstituted or mono-, di- or tri-substituted by lower alkyl, lower alkoxy, halogen, cyano and/or by trifluoromethyl, or of a salt thereof.

4. A process according to claim 1 for the preparation of a compound of formula I wherein R₁ is hydroxy-lower alkyl, lower alkoxy-lower alkyl, phenyl-lower alkoxy-lower alkyl, amino-lower alkyl, lower alkylamino-lower alkyl, di-lower alkylamino-lower alkyl, N,N-lower alkyleneamino- or N,N-(aza-or oxa-lower alkylene)amino-lower alkyl or halo-lower alkyl, and R₂ is carboxy, lower alkoxycarbonyl, 4- up to and including 7-membered cycloalkoxycarbonyl or phenyl-lower alkoxycarbonyl, any phenyl radicals in the mentioned groups R₁ and/or R₂ being unsubstituted or mono-, di- or tri-substituted by lower alkyl, lower alkoxy, halogen, cyano and/or by trifluoromethyl, or of a salt thereof.

5. A process according to claim 1 for the preparation of a compound of formula I wherein R₁ is hydroxy-C₁-C₇alkyl, dihydroxy-C₂-C₇alkyl, C₂-C₇alkanoyloxy-C₁-C₇alkyl, benzoyloxy-C₁-C₇alkyl that is unsubstituted or mono- or di-substituted in the phenyl moiety by C₁-C₄alkyl, C₁-C₄alkoxy, halogen having an atomic number of up to and including 35, cyano and/or by trifluoromethyl, C₁-C₄alkoxy-C₁-C₇alkyl, a phenyl-C₁-C₄alkoxy-C₁-C₇alkyl group that is unsubstituted or mono- or di-substituted in the phenyl moiety by C₁-C₄alkyl, C₁-C₄alkoxy, halogen having an atomic number of up to and including 35, cyano and/or by trifluoromethyl, halo-C₁-C₇alkyl, in which halogen is chlorine or fluorine, amino-C₁-C₇alkyl, C₁-C₄alkylamino-C₁-C₇alkyl, C₂-C₇alkanoylamino-C₁-C₇alkyl, N-C₂-C₇alkanoyl-N-C₁-C₄alkylamino-C₁-C₇alkyl, di-C₁-C₇alkylamino-C₁-C₇alkyl, 5- to 7-membered aza-cycloalk-1-yl-C₁-C₇alkyl, azacycloalk-3-yl-C₁-C₇alkyl or aza-cycloalk-4-yl-C₁-C₇alkyl, 1-C₂-C₇alkanoylazacycloalk-3-yl-C₁-C₇alkyl or -4-yl-C₁-C₇alkyl, N-C₁-C₄alkylazacycloalk-3-yl-C₁-C₇alkyl or -4-yl-C₁-C₇alkyl, N-benzoylazacycloalk-3-yl-C₁-C₇alkyl or -4-yl-C₁-C₇alkyl, 5- to 7-membered diazacycloalk-1-yl-C₁-C₇alkyl, N'-C₁-C₄alkyldiazacycloalk-1-yl-C₁-C₇alkyl, N'-C₂-C₇alkanoylazacycloalk-1-yl-C₁-C₇alkyl, 5- to 7-membered azoxacycloalk-1-yl-C₁-C₇alkyl, 5- to 7-membered oxacycloalk-3-yl-C₁-C₇alkyl or -4-yl-C₁-C₇alkyl, 5- to 7-membered azacycloalk-3-yl or -4-yl or 1-C₂-C₇alkanoylazacycloalk-3-yl or -4-yl, N-C₁-C₄alkylazacycloalk-3-yl or -4-yl, or N-benzoylazacycloalk-3-yl or -4-yl that is unsubstituted or substituted in the phenyl moiety, or 5- to 7-membered oxacycloalk-3-yl or -4-yl, and R₂ is carboxy, C₁-C₄alkoxycarbonyl, 5- to 7-membered cycloalkoxycarbonyl, or phenyl-C₁-C₄alkoxycarbonyl that is unsubstituted or mono- or di-substituted by C₁-C₄alkyl, C₁-C₄alkoxy, halogen having an atomic number of up to and including 35, cyano and/or by trifluoromethyl, or of a salt thereof.

6. A process according to claim 1 for the preparation of a compound of formula I wherein R₁ is hydroxy-C₁-C₇alkyl, C₁-C₄alkoxy-C₁-C₇alkyl, a phenyl-C₁-C₄alkoxy-C₁-C₇alkyl group that is unsubstituted or mono-, di- or tri-substituted in the phenyl moiety by C₁-C₄alkyl, C₁-C₄alkoxy, halogen having an atomic number of up to and including 35, cyano and/or by trifluoromethyl, amino-C₁-C₇alkyl, C₁-C₄alkylamino-C₁-C₇alkyl, C₂-C₇alkanoylamino-C₁-C₇alkyl, di-C₁-C₇alkylamino-C₁-C₇alkyl, 5- to 7-membered N,N-(aza- or oxa-alkylene)amino-C₁-C₇alkyl, or halo-C₁-C₇alkyl, in which halogen is chlorine or fluorine, and R₂ is carboxy, C₁-C₄alkoxycarbonyl, 5- to 7-membered cycloalkoxycarbonyl, or phenyl-C₁-C₄-alkoxycarbonyl that is unsubstituted or mono- or di-substitinted by C₁-C₄alkyl, C₁-C₄alkoxy, halogen having an atomic number of up to and including 35, cyano and/or by trifluoromethyl, or of a salt thereof.

7. A process according to claim 1 for the preparation of a compound of formula I wherein R₁ is hydroxy-C₁-C₇alkyl, C₁-C₄alkoxy-C₁-C₇alkyl, benzoyloxy-C₁-C₄alkyl, amino-C₄-C₇alkyl, N-C₂-C₇alkanoyl-N-C₁-C₄alkylamino-C₂-C₇alkyl, 5- to 7-membered azacycloalk-3-yl or -4-yl or 1-C₂-C₇alkanoylazacycloalk-3-yl or -4-yl, halo-C₁-C₄alkyl, in which halogen is chlorine or fluorine, amino-C₄-C₇alkyl, N-C₁-C₄alkanoyl-N-C₁-C₄alkylamino-C₁-C₇alkyl, piperidin-4-yl or 1-C₂-C₇alkanoylpiperidin-4-yl, and R₂ is carboxy or C₁-C₄alkoxycarbonyl, or of a salt thereof.

8. A process according to claim 1 for the preparation of a compound of formula I wherein R₁ is C₁-C₄alkoxy-C₂-C₄alkyl, hydroxy-C₂-C₄alkyl, amino-C₄-C₇alkyl or halo-C₂-C₄alkyl, and R₂ is carboxy, C₁-C₄alkoxycarbonyl or a phenyl-C₁-C₄alkoxycarbonyl group that is unsubstituted or mono- or disubstituted by C₁-C₄alkyl, C₁-C₄alkoxy, halogen having an atomic number of up to and including 35, cyano and/or by trifluoromethyl, or of a salt thereof.

9. A process according to claim 1 for the preparation of 2-amino-6-hydroxy-4-phosphonomethyl-hex-3-enoic acid ethyl ester or of a salt thereof.

10. A process according to claim 1 for the preparation of 2-amino-6-hydroxy-4-phosphonomethyl-hex-3-enoic acid or of a salt thereof.

11. A process according to claim 1 for the preparation of 2,10-diamino-4-phosphonomethyl-dec-3-enoic acid or of a salt thereof.

12. A process according to claim 1 for the preparation of 2,8-diamino-4-phosphonomethyl-oct-3-enoic acid or of a salt thereof.

13. A process according to claim 1 for the preparation of 2-amino-6-methoxy-4-phosphonomethyl-hex-3-enoic acid ethyl ester or of a salt thereof.

14. A process according to claim 1 for the preparation of 2-amino-6-fluoro-4-phosphonomethyl-hex-3-enoic acid ethyl ester or of a salt thereof.

15. A process according to claim 1 for the preparation of 2-amino-6-methoxy-4-phosphonomethyl-hex-3-enoic acid or of a salt thereof.

16. A process according to claim 1 for the preparation of 2-amino-6-benzoyloxy-4-phosphonomethyl-hex-3-enoic acid ethyl ester or of a salt thereof.

17. A process according to claim 1 for the preparation of 2-amino-7-hydroxy-4-phosphonomethyl-hept-3-enoic acid ethyl ester or of a salt thereof.

18. A process according to claim 1 for the preparation of 2-amino-7-hydroxy-4-phosphonomethyl-hept-3-enoic acid or of a salt thereof.

19. A process according to claim 1 for the preparation of 2-amino-6-benzyloxy-4-phosphonomethyl-hex-3-enoic acid or of a salt thereof.

20. A process according to claim 1 for the preparation of 6-(N-acetyl-N-methylamino)-2-amino-4-phosphonomethyl-hex-3- enoic acid ethyl ester or of a salt thereof.

21. A process according to claim 1 for the preparation of (1-acetylpiperidin-4-yl)-2-amino-5-phosphono-pent-3-enoic acid ethyl ester or of a salt thereof.

22. A process according to claim 1 for the preparation of 2,10-diamino-4-phosphonomethyl-dec-3-enoic acid ethyl ester or of a salt thereof.

23. A process according to claim 1 for the preparation of 2-amino-6-fluoro-4-phosphonomethyl-hex-3-enoic acid or of a salt thereof.

24. A process according to claim 1 for the preparation of 2-amino-5-hydroxy-4-phosphonomethyl-pent-3-enoic acid ethyl ester or of a salt thereof.

25. A process according to claim 1 for the preparation of 2-amino-5-hydroxy-4-phosphonomethyl-pent-3-enoic acid or of a salt thereof.

26. A process according to claim 1 for the preparation of 2-amino-5-benzyloxy-4-phosphonomethyl-pent-3-enoic acid ethyl ester or of a salt thereof.

27. A process according to claim 1 for the preparation of 2-amino-5-benzyloxy-4-phosphonomethyl-pent-3-enoic acid or of a salt thereof.

28. A process according to claim 1 for the preparation of 2-amino-8-hydroxy-4-phosphonomethyl-oct-3-enoic acid ethyl ester or of a salt thereof.

29. A process according to claim 1 for the preparation of 2-amino-8-hydroxy-4-phosphonomethyl-oct-3-enoic acid or of a salt thereof.

30. A process according to claim 1 for the preparation of 2-amino-6-(N-methylamino)-4-phosphonomethyl-hex-3-enoic acid ethyl ester or of a salt thereof.

31. A process according to claim 1 for the preparation of 2-amino-6-(N-methylamino)-4-phosphonomethyl-hex-3-enoic acid or of a salt thereof.

32. A process according to claim 1 for the preparation of 2-amino-6-hydroxy-4-phosphonomethyl-hex-3-enoic acid or of a salt thereof.

33. A process according to claim 1 for the preparation of 2-amino-6-hydroxy-4-phosphonomethyl-hex-3-enoic acid ethyl ester or of a salt thereof.

34. A process according to claim 1 for the preparation of 2-amino-4-(piperidin-4-yl)-5-phosphono-pent-3-enoic acid ethyl ester or of a salt thereof.

35. A process according to claim 1 for the preparation of 2-amino-4-(piperidin-4-yl)-5-phosphono-pent-3-enoic acid or of a salt thereof.

36. A process according to claim 1 for the preparation of 5-ethoxy-2-amino-4-phosphonomethyl-pent-3-enoic acid or of a salt thereof.

37. A process according to claim 1 for the preparation of 5-ethoxy-2-amino-4-phosphonomethyl-pent-3-enoic acid ethyl ester or of a salt thereof.

38. A process according to claim 1 for the preparation of 2-amino-8-hydroxy-4-phosphonomethyl-oct-3-enoic acid ethyl ester or of a salt thereof.

39. A process according to claim 1 for the preparation of 2-amino-6-hydroxy-5-hydroxymethyl-4-phosphonomethyl-hex-3-enoic acid ethyl ester or of a salt thereof.

40. A process according to claim 1 for the preparation of 2-amlno-10-hydroxy-4-phosphonomethyl-dec-3-enoic acid ethyl ester or of a salt thereof.

41. A process for the preparation of a pharmaceutical composition, which process comprises mixing a compound obtainable according to any one of claims 1 to 40, in free form or in the form of a pharmaceutically acceptable salt, with customary pharmaceutical excipients.

## Revendications (Revendications pour l'(les) Etat(s) contractant(s) suivant(s): AT, BE, CH, DE, DK, FR, GB, IT, LI, LU, NL, SE)

1. Dérivés substitués d'acides 2-amino-alca-3-énoïques de formule I 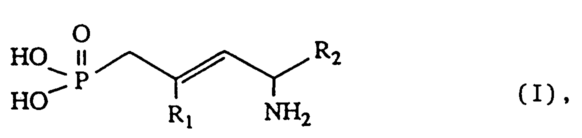 dans laquelle R₁ représente un radical hydrocarboné aliphatique substitué par un groupe hydroxy éventuellement acylé ou à l'état d'éther aliphatique ou araliphatique, par un halogène, par un groupe amino éventuellement acylé ou portant un substituant aliphatique ou par un radical aza-, diaza-, azoxa-, ou oxa-cycloaliphatique, ou un radical hydrocarboné oxacycloaliphatique relié par l'intermédiaire d'un atome de carbone, ou un radical hydrocarboné azacycloaliphatique portant éventuellement un substituant aliphatique à l'azote ou éventuellement acylé à l'azote, et R₂ représente un groupe carboxy libre ou estérifié, et leurs sels.

2. Composés selon revendication 1, répondant à la formule I dans laquelle R₁ représente un radical hydrocarboné aliphatique substitué par un groupe hydroxy éventuellement à l'état d'éther aliphatique ou araliphatique, un groupe amino portant éventuellement un substituant aliphatique ou un halogène, et R₂ représente un groupe carboxy libre ou estérifié, et leurs sels.

3. Composés selon revendication 1, répondant à la formule I dans laquelle R₁ représente un groupe monoou di-hydroxyalkyle inférieur, (alcanoyle inférieur)-oxyalkyle inférieur, benzoyloxyalkyle inférieur, (alcoxy inférieur)-alkyle inférieur, phényl-(alcoxy inférieur)-alkyle inférieur, halogénoalkyle inférieur, aminoalkyle inférieur, (alkyle inférieur)-aminoalkyle inférieur, (alcanoyle inférieur)-aminoalkyle inférieur, di-(alkyle inférieur)-aminoalkyle inférieur, N-(alkyle inférieur)-N-(alcanoyle inférieur)-aminoalkyle inférieur, azacycloalkyl-alkyle inférieur de 5 à 7 chaînons dont la partie azacycloalkyle est reliée par l'intermédiaire de l'atome d'azote ou d'un atome de carbone, et dans ce dernier cas est éventuellement substituée à l'azote par un groupe alkyle inférieur, alcanoyle inférieur ou benzoyle lui-même éventuellement substitué dans la partie phényle, un groupe diazacycloalkyl-alkyle inférieur de 5 à 7 chaînons dont la partie diazacycloalkyle est reliée par l'intermédiaire d'un atome d'azote et est éventuellement substituée sur l'azote N' par un groupe alkyle inférieur, alcanoyle inférieur ou benzoyle luimême éventuellement substitué dans la partie phényle, un groupe azoxacycloalkyl-alkyle inférieur de 5 à 7 chaînons relié par l'intermédiaire de l'atome d'azote, un groupe oxacycloalkyl-alkyle inférieur de 5 à 7 chaînons relié par l'intermédiaire d'un atome de carbone, un groupe azacycloalkyle de 5 à 7 chaînons relié par l'intermédiaire d'un atome de carbone et éventuellement substitué à l'azote par un groupe alkyle inférieur, alcanoyle inférieur ou benzoyle lui-même éventuellement substitué dans la partie phényle ou un groupe oxacycloalkyle de 5 à 7 chaînons relié par l'intermédiaire d'un atome de carbone, et R₂ représente un groupe carboxy, (alcoxy inférieur)-carbonyle, cycloalcoxycarbonyle de 4 à 7 chaînons ou phényl-(alcoxy inférieur)-carbonyle, les radicaux phényle éventuellement présents dans les groupes R₁ et/ou R₂ en question pouvant eux-mêmes être non substitués ou mono-, di- ou trisubstitués par des groupes alkyle inférieurs, alcoxy inférieurs, des halogènes, des groupes cyano et/ou trifluorométhyle, et leurs sels.

4. Composés selon revendication 2, répondant à la formule I dans laquelle R₁ représente un groupe hydroxyalkyle inférieur, (alcoxy inférieur)-alkyle inférieur, phényl-(alcoxy inférieur)-alkyle inférieur, aminoalkyle inférieur, (alkyle inférieur)-aminoalkyle inférieur, di-(alkyle inférieur)-aminoalkyle inférieur, N,N-(alkylène inférieur)-amino- ou N,N-(aza- ou oxaalkylène inférieur)-aminoalkyle inférieur ou halogénoalkyle inférieur, et R₂ représente un groupe carboxy, (alcoxy inférieur)-carbonyle, cycloalcoxycarbonyle de 4 à 7 chaînons ou phényl-(alcoxy inférieur)carbonyle, les radicaux phényle présents dans les groupes R₁ et/ou R₂ en question pouvant être non substitués ou mono-, di- ou tri-substitués par des groupes alkyle inférieurs, alcoxy inférieurs, des halogènes, des groupes cyano et/ou trifluorométhyle, et leurs sels.

5. Composés selon revendication 1, répondant à la formule I, dans laquelle R₁ représente un groupe hydroxyalkyle en C1-C7, dihydroxyalkyle en C2-C7, (alcanoyle en C2-C7)-oxyalkyle en C1-C7, benzoyloxyalkyle en C1-C7 éventuellement mono- ou di-substitué dans la partie phényle par des groupes alkyle en C1-C4, alcoxy en C1-C4, des halogènes de numéro atomique allant jusqu'à 35 inclus, des groupes cyano et/ou trifluorométhyle, un groupe (alcoxy en C1-C4)-alkyle en C1-C7, un groupe phényl-(alcoxy en C1-C4)-alkyle en C1-C7 éventuellement mono- ou di-substitué dans la partie phényle par des groupes alkyle en C1-C4, alcoxy en C1-C4, des halogènes de numéro atomique allant jusqu'à 35 inclus, des groupes cyano et/ou trifluorométhyle, un groupe halogénoalkyle en C1-C7 dans lequel l'halogène consiste en chlore ou fluor, un groupe aminoalkyle en C1-C7, (alkyle en C1-C4)-aminoalkyle en C1-C7, (alcanoyle en C2-C7)-aminoalkyle en C1-C7, N-(alcanoyle en C2-C7)-N-(alkyle en C1-C4)-aminoalkyle en C1-C7, di-(alkyle en C1-C7)-aminoalkyle en C1-C7, azacycloalca-1-yl-alkyle en C1-C7, azacycloalca-3-yl-alkyle en C1-C7 ou azacycloalca-4-yl-alkyle en C1-C7 de 5 à 7 chaînons, un groupe (alcanoyle en C2-C7)-azacycloalca-3-yl-alkyle en C1-C7 ou -4-yl-alkyle en C1-C7, N-(alkyle en C1-C4)-azacycloalkyle-3-yl-alkyle en C1-C7 ou -4-yl-alkyle en C1-C7, N-(benzoyle en C1-C4)-azacycloalkyl-3-yl-alkyle en C1-C7 ou -4-yl-alkyle en C1-C7, diazacycloalca-1-yl-alkyle en C1-C7 de 5 à 7 chaînons, N'-(alkyle en C1-C4)-diazacycloalca-1-yl-alkyle en C1-C7, N'-(alcanoyle en C2-C7)-azacycloalca-1-yl-alkyle en C1-C7, azoxacycloalca-1-yl-alkyle en C1-C7 de 5 à 7 chaînons, oxacycloalca-3-yl-alkyle en C1-C7 ou -4-yl-alkyle en C1-C7 de 5 à 7 chaînons, azacycloalca-3-yle ou -4-yle de 5 à 7 chaînons ou 1-(alcanoyle en C2-C7)-azacycloalca-3-yle ou - 4-yle, N-(alkyle en C1-C4)-azacycloalca-3-yle ou -4-yle ou N-benzoylazacycloalca-3-yle ou -4-yle éventuellement substitué dans la partie phényle ou oxacycloalca-3-yle ou -4-yle de 5 à 7 chaînons, et R₂ représente un groupe carboxy, (alcoxy en C1-C4)-carbonyle, cycloalcoxycarbonyle de 5 à 7 chaînons ou phényl-(alcoxy en C1-C4)-carbonyle éventuellement mono- ou di-substitué par des groupes alkyle en C1-C4, alcoxy en C1-C4, des halogènes de numéro atomique allant jusqu'à 35 inclus, des groupes cyano et/ou trifluorométhyle, et leurs sels.

6. Composés selon revendication 2, répondant à la formule I dans laquelle R₁ représente un groupe hydroxyalkyle en C1-C7, (alcoxy en C1-C4)-alkyle en C1-C7, un groupe phényl-(alcoxy en C1-C4)-alkyle en C1-C7 éventuellement mono-, di- ou tri-substitué dans la partie phényle par des groupes alkyle en C1-C4, alcoxy en C1-C4, des halogènes de numéro atomique allant jusqu'à 35 inclus, des groupes cyano et/ou trifluorométhyle, un groupe aminoalkyle en C1-C7, (alkyle en C1-C4)-aminoalkyle en C1-C7, (alcanoyle en C2-C7)-aminoalkyle en C1-C7, di-(alkyle en C1-C7)-aminoalkyle en C1-C7, N,N-(aza- ou oxaalkylène)-aminoalkyle en C1-C7 de 5 à 7 chaînons ou halogénoalkyle en C1-C7, l'halogène consistant en chlore ou fluor, et R₂ représente un groupe carboxy, (alcoxy en C1-C4)-carbonyle, cycloalcoxycarbonyle de 5 à 7 chaînons, phényl-(alcoxy en C1-C4)-carbonyle éventuellement mono- ou di-substitué par des groupes alkyle en C1-C4, alcoxy en C1-C4, des halogènes de numéro atomique allant jusqu'à 35 inclus, des groupes cyano et/ou trifluorométhyle, et leurs sels.

7. Composés selon revendication 1, répondant à la formule I dans laquelle R₁ représente un groupe hydroxyalkyle en C1-C7, (alcoxy en C1-C4)-alkyle en C1-C7, benzoyloxyalkyle en C1-C4, aminoalkyle en C4-C7, N-(alcanoyle en C2-C7)-N-(alkyle en C1-C4)-aminoalkyle en C2-C7, azacycloalca-3-yle ou -4-yle de 4 à 7 chaînons ou 1-(alcanoyle en C2-C7)-azacycloalca-3-yle ou -4-yle, halogénoalkyle en C1-C4, l'halogène consistant en chlore ou fluor, aminoalkyle en C4-C7, N-(alcanoyle en C1-C4)-N-(alkyle en C1-C4)-aminoalkyle en C1-C7, pipéridino-4-yle ou 1-(alcanoyle en C2-C7)-pipéridino-4-yle et R₂ représente un groupe carboxy ou (alcoxy en C1-C4)-carbonyle, et leurs sels.

8. Composés selon revendication 2, répondant à la formule 1 dans laquelle R₁ représente un groupe (alcoxy en C1-C4)-alkyle en C2-C4, hydroxyalkyle en C2-C4, aminoalkyle en C4-C7 ou halogénoalkyle en C2-C4, et R₂ représente un groupe carboxy, (alcoxy en C1-C4)-carbonyle ou un groupe phényl-(alcoxy en C1-C4)-carbonyle éventuellement mono- ou di-substitué par des groupes alkyle en C1-C4, alcoxy en C1-C4, des halogènes de numéro atomique allant jusqu'à 35 inclus, des groupes cyano et/ou trifluorométhyle, et leurs sels.

9. Le 2-amino-6-hydroxy-4-phosphonométhyl-hexa-3-énoate d'éthyle ou l'un de ses sels.

10. L'acide 2-amino-6-hydroxy-4-phosphonométhyl-hexa-3-énoïque ou l'un de ses sels.

11. L'acide 2,10-diamino-4-phoshonométhyl-déca-3-énoïque ou l'un de ses sels.

12. L'acide 2,8-diamino-4-phosphonométhyl-octa-3-énoïque ou l'un de ses sels.

13. Le 2-amino-6-méthoxy-4-phosphonométhylhexa-3-énoate d'éthyle ou l'un de ses sels.

14. Le 2-amino-6-fluoro-4-phosphonométhyl-hexa-3-énoate d'éthyle ou l'un de ses sels.

15. L'acide 2-amino-6-méthoxy-4-phosphonométhyl-hexa-3-énoïque ou l'un de ses sels.

16. Le 2-amino-6-benzyloxy-4-phosphonométhyl-hexa-3-énoate d'éthyle ou l'un de ses sels.

17. Le 2-amino-7-hydroxy-4-phosphonométhylhepta-3-énoate d'éthyle ou l'un de ses sels.

18. L'acide 2-amino-7-hydroxy-4-phosphonométhyl-hepta-3-énoïque ou l'un de ses sels.

19. L'acide 2-amino-6-benzyloxy-4-phosphonométhyl-hexa-3-énoïque ou l'un de ses sels.

20. Le 6-(N-acétyl-N-méthyl-amino)-2-amino-4-phosphonométhyl-hexa-3-énoate d'éthyle ou l'un de ses sels.

21. Le (1-acétylpipéridino-4-yl)-2-amino-5-phosphono-penta-3-énoate d'éthyle ou l'un de ses sels.

22. Le 2,10-diamino-4-phosphonométhyl-déca-3-énoate d'éthyle ou l'un de ses sels.

23. L'acide 2-amino-6-fluoro-4-phosphonométhyl-hexa-3-énoïque ou l'un de ses sels.

24. Le 2-amino-5-hydroxy-4-phosphonométhyl-penta-3-énoate d'éthyle ou l'un de ses sels.

25. L'acide 2-amino-5-hydroxy-4-phosphonométhyl-penta-3-énoïque ou l'un de ses sels.

26. Le 2-amino-5-benzyloxy-4-phosphonométhyl-penta-3-énoate d'éthyle ou l'un de ses sels.

27. L'acide 2-amino-5-benzyloxy-4-phosphonométhyl-penta-3-énoïque ou l'un de ses sels.

28. Le 2-amino-8-hydroxy-4-phosphonométhyl-octa-3-énoate d'éthyle ou l'un de ses sels.

29. L'acide 2-amino-8-hydroxy-4-phosphonométhyl-octa-3-énoïque ou l'un de ses sels.

30. Le 2-amino-6-(N-méthylamino)-4-phosphonométhyl-hexa-3-énoate d'éthyle ou l'un de ses sels.

31. L'acide 2-amino-6-(N-méthylamino)-4-phosphonométhyl-hexa-3-énoïque ou l'un de ses sels.

32. L'acide 2-amino-6-hydroxy-4-phosphonométhyl-hexa-3-énoïque ou l'un de ses sels.

33. Le 2-amino-6-hydroxy-4-phosphonométhyl-hexa-3-énoate d'éthyle ou l'un de ses sels.

34. Le 2-amino-4-(pipéridino-4-yl)-5-phosphono-penta-3-énoate d'éthyle ou l'un de ses sels.

35. L'acide 2-amino-4-(pipéridino-4-yl)-5-phosphono-penta-3-énoïque ou l'un de ses sels.

36. L'acide 5-éthoxy-2-amino-4-phosphonométhyl-penta-3-énoïque ou l'un de ses sels.

37. Le 5-éthoxy-2-amino-4-phosphonométhyl-penta-3-énoate d'éthyle ou l'un de ses sels.

38. Le 2-amino-8-hydroxy-4-phosphonométhyl-octa-3-énoate d'éthyle ou l'un de ses sels.

39. Le 2-amino-6-hydroxy-5-hydroxyméthyl-4-phosphonométhyl-hexa-3-énoate d'éthyle ou l'un de ses sels.

40. Le 2-amino-10-hydroxy-4-phosphonométhyl-déca-3-énoate d'éthyle ou l'un de ses sels.

41. Un composé selon l'une des revendications 1, 3, 5, 7 et 15 à 40 pour l'utilisation dans un procédé pour le traitement thérapeutique de l'organisme humain ou animal.

42. Un composé selon l'une des revendications 2, 4, 6 et 8 à 14, pour l'utilisation dans un procédé pour le traitement thérapeutique de l'organisme humain ou animal.

43. Compositions pharmaceutiques contenant un composé selon l'une des revendications 3, 5, 7 et 15 à 41, à l'état libre ou à l'état de sel acceptable pour l'usage pharmaceutique.

44. Compositions pharmaceutiques contenant un composé selon l'une des revendications 2, 4, 6, 8 à 14 et 42 à l'état libre ou à l'état de sel acceptable pour l'usage pharmaceutique.

45. Procédé de préparation de dérivés insaturés d'acides aminocarboxyliques de formule I 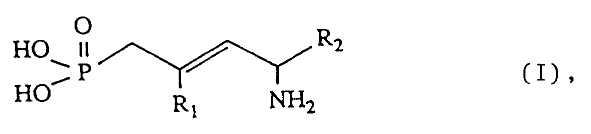 dans laquelle R₁ représente un radical hydrocarboné aliphatique substitué par un groupe hydroxy éventuellement acylé ou à l'état d'éther aliphatique ou araliphatique, par un halogène, par un groupe amino éventuellement acylé et/ou portant un substituant aliphatique ou par un radical aza-, diaza-, azoxa- ou oxa-cycloaliphatique, ou un radical hydrocarboné oxa-cycloaliphatique relié par l'intermédiaire d'un atome de carbone, ou un radical hydrocarboné aza-cycloaliphatique portant éventuellement un substituant aliphatique à l'azote ou éventuellement acylé à l'azote, et R₂ représente un groupe carboxy libre ou estérifié, et de leurs sels, caractérisé en ce que dans un composé de formule II 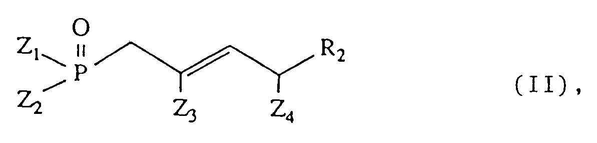 dans laquelle Z₁, Z₂ représentent des groupes hydroxy éventuellement protégés, Z₃ un radical hydrocarboné aliphatique substitué par un groupe hydroxy éventuellement protégé ou acylé ou à l'état d'éther aliphatique ou araliphatique, par un halogène, par un groupe amino éventuellement protégé ou acylé et/ou portant un substituant aliphatique, ou par un radical aza-, diaza-, azoxa- ou oxa-cycloaliphatique, ou un radical hydrocarboné oxa-cycloaliphatique relié par l'intermédiaire d'un atome de carbone ou un radical hydrocarboné azacycloaliphatique éventuellement protégé ou portant un substituant aliphatique à l'azote ou acylé à l'azote, et Z₄ représente un groupe amino protégé, on convertit le groupe amino protégé Z₄ et, le cas échéant, un groupe amino protégé constituant de Z₃, en groupes amino et, le cas échéant, les groupes hydroxy protégés, Z₁, Z₂ et/ou constituants de Z₃, en groupes hydroxy et, le cas échéant, on libère un radical hydrocarboné azacycloaliphatique protégé Z₃ et, si on le désire, on convertit un composé ainsi obtenu en un autre composé de formule I, on sépare un mélange d'isomères ainsi obtenu en les composants et on isole l'isomère préféré dans chaque cas et/ou on convertit un composé ainsi obtenu à l'état libre en un sel ou un sel ainsi obtenu en le composé libre correspondant.

46. Utilisation d'un composé selon l'une des revendications 1 à 42 en tant que substance active médicamenteuse ou pour la préparation d'une composition pharmaceutique.

## Revendications (Revendications pour l'(les) Etat(s) contractant(s) suivant(s): ES, GR)

1. Procédé de préparation de dérivés insaturés d'acides aminocarboxyliques de formule I 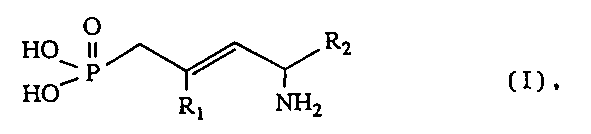 dans laquelle R₁ représente un radical hydrocarboné aliphatique substitué par un groupe hydroxy éventuellement acylé ou à l'état d'éther aliphatique ou araliphatique, par un halogène, par un groupe amino éventuellement acylé ou portant un substituant aliphatique ou par un radical aza-, diaza-, azoxa- ou oxa-cycloaliphatique, ou un radical hydrocarboné oxacycloaliphatique relié par l'intermédiaire d'un atome de carbone, ou un radical hydrocarboné azacycloaliphatique portant éventuellement un substituant aliphatique à l'azote ou éventuellement acylé à l'azote, et R₂ représente un groupe carboxy libre ou estérifié, et leurs sels, caractérisé en ce que dans un composé de formule II 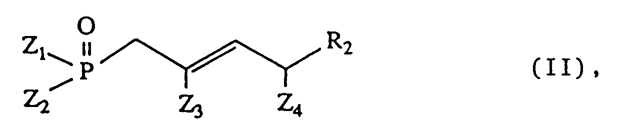 dans laquelle Z₁, Z₂ représentent des groupes hydroxy éventuellement protégés, Z₃ un radical hydrocarboné aliphatique substitué par un groupe hydroxy éventuellement protégé ou acylé ou à l'état d'éther aliphatique ou araliphatique, par un halogène, par un groupe amino éventuellement protégé ou acylé et/ou portant un substituant aliphatique, ou par un radical aza-, diaza-, azoxa- ou oxa-cycloaliphatique, ou un radical hydrocarboné oxa-cycloaliphatique relié par l'intermédiaire d'un atome de carbone ou un radical hydrocarboné azacycloaliphatique éventuellement protégé ou portant un substituant aliphatique à l'azote ou acylé à l'azote, et Z₄ représente un groupe amino protégé, on convertit le groupe amino protégé Z₄ et, le cas échéant, un groupe amino protégé constituant de Z₃, en groupes amino et, le cas échéant, les groupes hydroxy protégés, Z₁, Z₂ et/ou constituants de Z₃, en groupes hydroxy et, le cas échéant, on libère un radical hydrocarboné azacycloaliphatique protégé Z₃ et, si on le désire, on convertit un composé ainsi obtenu en un autre composé de formule I, on sépare un mélange d'isomères ainsi obtenu en les composants et on isole l'isomère préféré dans chaque cas et/ou on convertit un composé ainsi obtenu à l'état libre en un sel ou un sel ainsi obtenu en le composé libre correspondant.

2. Procédé selon revendication 1 pour la préparation des composés de formule I dans laquelle R₁ représente un radical hydrocarboné aliphatique substitué par un groupe hydroxy éventuellement à l'état d'éther aliphatique ou araliphatique, un groupe amino portant éventuellement un substituant aliphatique ou un halogène, et R₂ représente un groupe carboxy libre ou estérifié, et de leurs sels.

3. Procédé selon revendication 1 pour la préparation des composés de formule I dans laquelle R₁ représente un groupe mono- ou di-hydroxyalkyle inférieur, (alcanoyle inférieur)-oxyalkyle inférieur, benzoyloxyalkyle inférieur, (alcoxy inférieur)-alkyle inférieur, phényl-(alcoxy inférieur)-alkyle inférieur, halogénoalkyle inférieur, aminoalkyle inférieur, (alkyle inférieur)-aminoalkyle inférieur, (alcanoyle inférieur)aminoalkyle inférieur, di-(alkyle inférieur)-aminoalkyle inférieur, N-(alkyle inférieur)-N-(alcanoyle inférieur)aminoalkyle inférieur, azacycloalkyl-alkyle inférieur de 5 à 7 chaînons dont la partie azacycloalkyle est reliée par l'intermédiaire de l'atome d'azote ou d'un atome de carbone, et dans ce dernier cas est éventuellement substituée à l'azote par un groupe alkyle inférieur, alcanoyle inférieur ou benzoyle lui-même éventuellement substitué dans la partie phényle, un groupe diazacycloalkyl-alkyle inférieur de 5 à 7 chaînons dont la partie diazacycloalkyle est reliée par l'intermédiaire d'un atome d'azote et est éventuellement substituée sur l'azote N' par un groupe alkyle inférieur, alcanoyle inférieur ou benzoyle lui-même éventuellement substitué dans la partie phényle, un groupe azoxacycloalkyl-alkyle inférieur de 5 à 7 chaînons relié par l'intermédiaire de l'atome d'azote, un groupe oxacycloalkyl-alkyle inférieur de 5 à 7 chaînons relié par l'intermédiaire d'un atome de carbone, un groupe azacycloalkyle de 5 à 7 chaînons relié par l'intermédiaire d'un atome de carbone et éventuellement substitué à l'azote par un groupe alkyle inférieur, alcanoyle inférieur ou benzoyle lui-même éventuellement substitué dans la partie phényle ou un groupe oxacycloalkyle de 5 à 7 chaînons relié par l'intermédiaire d'un atome de carbone, et R₂ représente un groupe carboxy, (alcoxy inférieur)-carbonyle, cycloalcoxycarbonyle de 4 à 7 chaînons ou phényl-(alcoxy inférieur)-carbonyle, les radicaux phényle éventuellement présents dans les groupes R₁ et/ou R₂ en question pouvant eux-mêmes être non substitués ou mono-, di- ou trisubstitués par des groupes alkyle inférieurs, alcoxy inférieurs, des halogènes, des groupes cyano et/ou trifluorométhyle, et leurs sels.

4. Procédé selon revendication 1 pour la préparation des composés de formule I dans laquelle R₁ représente un groupe hydroxyalkyle inférieur, (alcoxy inférieur)-alkyle inférieur, phényl-(alcoxy inférieur)alkyle inférieur, aminoalkyle inférieur, (alkyle inférieur)-aminoalkyle inférieur, di-(alkyle inférieur)-aminoalkyle inférieur, N,N-(alkylène inférieur)-amino- ou N,N-(aza- ou oxaalkylène inférieur)-aminoalkyle inférieur ou halogénoalkyle inférieur, et R₂ représente un groupe carboxy, (alcoxy inférieur)-carbonyle, cycloalcoxycarbonyle de 4 à 7 chaînons ou phényl-(alcoxy inférieur)carbonyle, les radicaux phényle présents dans les groupes R₁ et/ou R₂ en question pouvant être non substitués ou mono-, di- ou tri-substitués par des groupes alkyle inférieurs, alcoxy inférieurs, des halogènes, des groupes cyano et/ou trifluorométhyle, et leurs sels.

5. Procédé selon revendication 1 pour la préparation des composés de formule I dans laquelle R₁ représente un groupe hydroxyalkyle en C1-C7, dihydroxyalkyle en C2-C7, (alcanoyle en C2-C7)-oxyalkyle en C1-C7, benzoyloxyalkyle en C1-C7 éventuellement monoou di-substitué dans la partie phényle par des groupes alkyle en C1-C4, alcoxy en C1-C4, des halogènes de numéro atomique allant jusqu'à 35 inclus, des groupes cyano et/ou trifluorométhyle, un groupe (alcoxy en C1-C4)-alkyle en C1-C7, un groupe phényl-(alcoxy en C1-C4)-alkyle en C1-C7 éventuellement mono- ou di-substitué dans la partie phényle par des groupes alkyle en C1-C4, alcoxy en C1-C4, des halogènes de numéro atomique allant jusqu'à 35 inclus, des groupes cyano et/ou trifluorométhyle, un groupe halogénoalkyle en C1-C7 dans lequel l'halogène consiste en chlore ou fluor, un groupe aminoalkyle en C1-C7, (alkyle en C1-C4)-aminoalkyle en C1-C7, (alcanoyle en C2-C7)-aminoalkyle en C1-C7, N-(alcanoyle en C2-C7)-N-(alkyle en C1-C4)-aminoalkyle en C1-C7, di-(alkyle en C1-C7)-aminoalkyle en C1-C7, azacycloalca-1-yl-alkyle en C1-C7, azacycloalca-3-yl-alkyle en C1-C7 ou azacycloalca-4-yl-alkyle en C1-C7 de 5 à 7 chaînons, un groupe (alcanoyle en C2-C7)-azacycloalca-3-yl-alkyle en C1-C7 ou -4-yl-alkyle en C1-C7, N-(alkyle en C1-C4)-azacycloalkyle-3-yl-alkyle en C1-C7 ou -4-yl-alkyle en C1-C7, N-(benzoyle en C1-C4)-azacycloalkyl-3-yl-alkyle en C1-C7 ou -4-yl-alkyle en C1-C7, diazacycloalca-1-yl-alkyle en C1-C7 de 5 à 7 chaînons, N'-(alkyle en C1-C4)-diazacycloalca-1-yl-alkyle en C1-C7, N'-(alcanoyle en C2-C7)-azacycloalca-1-yl-alkyle en C1-C7, azoxacycloalca-1-yl-alkyle en C1-C7 de 5 à 7 chaînons, oxacycloalca-3-yl-alkyle en C1-C7 ou -4-yl-alkyle en C1-C7 de 5 à 7 chaînons, azacycloalca-3-yle ou -4-yle de 5 à 7 chaînons ou 1-(alcanoyle en C2-C7)-azacycloalca-3-yle ou - 4-yle, N-(alkyle en C1-C4)-azacycloalca-3-yle ou -4-yle ou N-benzoylazacycloalca-3-yle ou -4-yle éventuellement substitué dans la partie phényle ou oxacycloalca-3-yle ou -4-yle de 5 à 7 chaînons, et R₂ représente un groupe carboxy, (alcoxy en C1-C4)-carbonyle, cycloalcoxycarbonyle de 5 à 7 chaînons ou phényl-(alcoxy en C1-C4)-carbonyle éventuellement mono- ou di-substitué par des groupes alkyle en C1-C4, alcoxy en C1-C4, des halogènes de numéro atomique allant jusqu'à 35 inclus, des groupes cyano et/ou trifluorométhyle, et leurs sels.

6. Procédé selon revendication 1, pour la préparation des composés de formule I dans laquelle R₁ représente un groupe hydroxyalkyle en C1-C7, (alcoxy en C1-C4)-alkyle en C1-C7, un groupe phényl-(alcoxy en C1-C4)-alkyle en C1-C7 éventuellement mono-, di- ou trisubstitué dans la partie phényle par des groupes alkyle en C1-C4, alcoxy en C1-C4, des halogènes de numéro atomique allant jusqu'à 35 inclus, des groupes cyano et/ou trifluorométhyle, un groupe aminoalkyle en C1-C7, (alkyle en C1-C4)-aminoalkyle en C1-C7, (alcanoyle en C2-C7)-aminoalkyle en C1-C7, di-(alkyle en C1-C7)-aminoalkyle en C1-C7, N,N-(aza- ou oxa-alkylène)-aminoalkyle en C1-C7 de 5 à 7 chaînons ou halogénoalkyle en C1-C7, l'halogène consistant en chlore ou fluor, et R₂ représente un groupe carboxy, (alcoxy en C1-C4)-carbonyle, cycloalcoxycarbonyle de 5 à 7 chaînons, phényl-(alcoxy en C1-C4)-carbonyle éventuellement mono- ou di-substitué par des groupes alkyle en C1-C4, alcoxy en C1-C4, des halogènes de numéro atomique allant jusqu'à 35 inclus, des groupes cyano et/ou trifluorométhyle, et leurs sels.

7. Procédé selon revendication 1 pour la préparation des composés de formule I dans laquelle R₁ représente un groupe hydroxyalkyle en C1-C7, (alcoxy en C1-C4)-alkyle en C1-C7, benzoyloxyalkyle en C1-C4, aminoalkyle en C4-C7, N-(alcanoyle en C2-C7)-N-(alkyle en C1-C4)-aminoalkyle en C2-C7, azacycloalca-3-yle ou -4-yle de 4 à 7 chaînons ou 1-(alcanoyle en C2-C7)-azacycloalca-3-yle ou -4-yle, halogénoalkyle en C1-C4, l'halogène consistant en chlore ou fluor, aminoalkyle en C4-C7, N-(alcanoyle en C1-C4)-N-(alkyle en C1-C4)-aminoalkyle en C1-C7, pipéridino-4-yle ou 1-(alcanoyle en C2-C7)-pipéridino-4-yle, et R₂ représente un groupe carboxy ou (alcoxy en C1-C4)-carbonyle, et leurs sels.

8. Procédé selon revendication 1 pour la préparation des composés de formule I dans laquelle R₁ représente un groupe (alcoxy en C1-C4)-alkyle en C2-C4, hydroxyalkyle en C2-C4, aminoalkyle en C4-C7 ou halogénoalkyle en C2-C4, et R₂ représente un groupe carboxy, (alcoxy en C1-C4)-carbonyle ou un groupe phényl(alcoxy en C1-C4)-carbonyle éventuellement mono- ou disubstitué par des groupes alkyle en C1-C4, alcoxy en C1-C4, des halogènes de numéro atomique allant jusqu'à 35 inclus, des groupes cyano et/ou trifluorométhyle, et leurs sels.

9. Procédé selon revendication 1 pour la préparation du 2-amino-6-hydroxy-4-phosphonométhyl-hexa-3-énoate d'éthyle ou de l'un de ses sels.

10. Procédé selon revendication 1 pour la préparation de l'acide 2-amino-6-hydroxy-4-phosphonométhyl-hexa-3-énoïque ou de l'un de ses sels.

11. Procédé selon revendication 1 pour la préparation de l'acide 2,10-diamino-4-phosphonométhyl-déca-3-énoïque ou de l'un de ses sels.

12. Procédé selon revendication 1 pour la préparation de l'acide 2,8-diamino-4-phosphonométhyl-octa-3-énoïque ou de l'un de ses sels.

13. Procédé selon revendication 1 pour la préparation du 2-amino-6-méthoxy-4-phosphonométhyl-hexa-3-énoate d'éthyle ou de l'un de ses sels.

14. Procédé selon revendication 1 pour la préparation du 2-amino-6-fluoro-4-phosphonométhyl-hexa-3-énoate d'éthyle ou de l'un de ses sels.

15. Procédé selon revendication 1 pour la préparation de l'acide 2-amino-6-méthoxy-4-phosphonométhyl-hexa-3-énoïque ou de l'un de ses sels.

16. Procédé selon revendication 1 pour la préparation du 2-amino-6-benzyloxy-4-phosphonométhyl-hexa-3-énoate d'éthyle ou de l'un de ses sels.

17. Procédé selon revendication 1 pour la préparation du 2-amino-7-hydroxy-4-phosphonométhyl-hepta-3-énoate d'éthyle ou de l'un de ses sels.

18. Procédé selon revendication 1 pour la préparation de l'acide 2-amino-7-hydroxy-4-phosphonométhyl-hepta-3-énoïque ou de l'un de ses sels.

19. Procédé selon revendication 1 pour la préparation de l'acide 2-amino-6-benzyloxy-4-phosphonométhyl-hexa-3-énoïque ou de l'un de ses sels.

20. Procédé selon revendication 1 pour la préparation du 6-(N-acétyl-N-méthyl-amino)-2-amino-4-phosphonométhyl-hexa-3-énoate d'éthyle ou de l'un de ses sels.

21. Procédé selon revendication 1 pour la préparation du (1-acétylpipéridino-4-yl)-2-amino-5-phosphono-penta-3-énoate d'éthyle ou de l'un de ses sels.

22. Procédé selon revendication 1 pour la préparation du 2,10-diamino-4-phosphonométhyl-déca-3-énoate d'éthyle ou de l'un de ses sels.

23. Procédé selon revendication 1 pour la préparation de l'acide 2-amino-6-fluoro-4-phosphonométhyl-hexa-3-énoïque ou de l'un de ses sels.

24. Procédé selon revendication 1 pour la préparation du 2-amino-5-hydroxy-4-phosphonométhyl-penta-3-énoate d'éthyle ou de l'un de ses sels.

25. Procédé selon revendication 1 pour la préparation de l'acide 2-amino-5-hydroxy-4-phosphonométhyl-penta-3-énoïque ou de l'un de ses sels.

26. Procédé selon revendication 1 pour la préparation du 2-amino-5-benzyloxy-4-phosphonométhyl-penta-3-énoate d'éthyle ou de l'un de ses sels.

27. Procédé selon revendication 1 pour la préparation de l'acide 2-amino-5-benzyloxy-4-phosphonométhyl-penta-3-énoïque ou de l'un de ses sels.

28. Procédé selon revendication 1 pour la préparation du 2-amino-8-hydroxy-4-phosphonométhyl-octa-3-énoate d'éthyle ou de l'un de ses sels.

29. Procédé selon revendication 1 pour la préparation de l'acide 2-amino-8-hydroxy-4-phosphonométhyl-octa-3-énoïque ou de l'un de ses sels.

30. Procédé selon la revendication 1 pour la préparation du 2-amino-6-(N-méthylamino)-4-phosphonométhyl-hexa-3-énoate d'éthyle ou de l'un de ses sels.

31. Procédé selon revendication 1 pour la préparation de l'acide 2-amino-6-(N-méthylamino)-4-phosphonométhyl-hexa-3-énoïque ou de l'un de ses sels.

32. Procédé selon revendication 1 pour la préparation de l'acide 2-amino-6-hydroxy-4-phosphonométhyl-hexa-3-énoïque ou de l'un de ses sels.

33. Procédé selon revendication 1 pour la préparation du 2-amino-6-hydroxy-4-phosphonométhyl-hexa-3-énoate d'éthyle ou de l'un de ses sels.

34. Procédé selon revendication 1 pour la préparation du 2-amino-4-(pipéridino-4-yl)-5-phosphono-penta-3-énoate d'éthyle ou de l'un de ses sels.

35. Procédé selon revendication 1 pour la préparation de l'acide 2-amino-4-(pipéridino-4-yl)-5-phosphono-penta-3-énoïque ou de l'un de ses sels.

36. Procédé selon revendication 1 pour la préparation de l'acide 5-éthoxy-2-amino-4-phosphonométhyl-penta-3-énoïque ou de l'un de ses sels.

37. Procédé selon revendication 1 pour la préparation du 5-éthoxy-2-amino-4-phosphonométhyl-penta-3-énoate d'éthyle ou de l'un de ses sels.

38. Procédé selon revendication 1 pour la préparation du 2-amino-8-hydroxy-4-phosphonométhyl-octa-3-énoate d'éthyle ou de l'un de ses sels.

39. Procédé selon revendication 1 pour la préparation du 2-amino-6-hydroxy-5-hydroxyméthyl-4-phosphonométhyl-hexa-3-énoate d'éthyle ou de l'un de ses sels.

40. Procédé selon revendication 1 pour la préparation du 2-amino-10-hydroxy-4-phosphonométhyl-déca-3-énoate d'éthyle ou de l'un de ses sels.

41. Procédé de préparation de compositions pharmaceutiques, caractérisé en ce que l'on mélange un composé obtenu selon l'une des revendications 1 à 40, à l'état libre ou à l'état de sel acceptable pour l'usage pharmaceutique, avec des produits auxiliaires pharmaceutiques usuels.
